(19) Europäisches Patentamt
European Patent Office
Office européen des brevets

(11) EP 3 342 427 A1

(12) **EUROPEAN PATENT APPLICATION**

(43) Date of publication:
**04.07.2018 Bulletin 2018/27**

(21) Application number: **17198484.2**

(22) Date of filing: **16.09.2009**

(51) Int Cl.:
***A61K 47/60*** *(2017.01)* ***A61P 25/04*** *(2006.01)*
***A61P 25/36*** *(2006.01)*

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **16.09.2008 US 192247 P**
**11.09.2009 US 558395**

(62) Document number(s) of the earlier application(s) in accordance with Art. 76 EPC:
**09789323.4 / 2 340 044**

(71) Applicant: **Nektar Therapeutics**
**San Francisco, CA 94158 (US)**

(72) Inventors:
- **JUDE-FISHBURN, Simone C.**
**Redwood City, CA 94062 (US)**
- **RIELY, Timothy A.**
**Huntsville, AL 35824 (US)**
- **ZACARIAS, Alberto**
**Oakland, CA 94619 (US)**
- **GURSAHANI, Hema**
**Foster City, CA 94404 (US)**

(74) Representative: **Boult Wade Tennant**
**Verulam Gardens**
**70 Gray's Inn Road**
**London WC1X 8BT (GB)**

Remarks:
•This application was filed on 26-10-2017 as a divisional application to the application mentioned under INID code 62.
•Claims filed after the date of filing of the application/after the date of receipt of the divisional application (Rule 68(4) EPC)

(54) **PEGYLATED OPIOIDS WITH LOW POTENTIAL FOR ABUSE**

(57) The invention provides opioid agonists, in particular morphine and codeine, covalently bound to a water-soluble oligomer, namely a polyethylene glycol (PEG) oligomer having reduced potential for substance abuse and uses thereof. The compounds of the invention possess altered pharmacokinetic profiles relative to the opioid agonists alone, but are not subject to the risk of physical tampering that allows for the recovery and abuse of the opioid agonist associated with certain alternative delivery formulations.

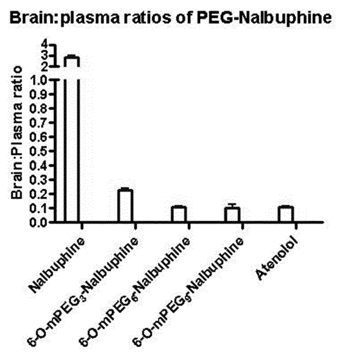


| Molecule | Brain:plasma ratios |
|---|---|
| Nalbuphine | 2.86 |
| 6-O-mPEG$_3$-Nalbuphine | 0.23 |
| 6-O-mPEG$_6$-Nalbuphine | 0.11 |
| 6-O-mPEG$_9$-Nalbuphine | 0.10 |
| Atenolol | 0.11 |

FIG. 1

EP 3 342 427 A1

**Brain:plasma ratios of PEG-Nalbuphine**

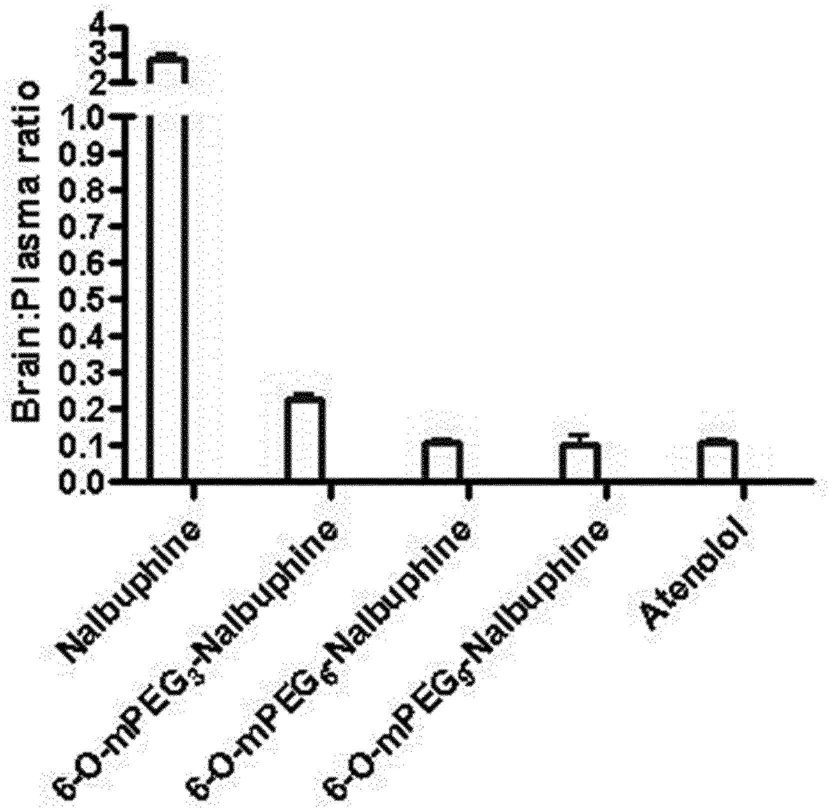


| Molecule | Brain:plasma ratios |
|---|---|
| Nalbuphine | 2.86 |
| 6-O-mPEG$_3$-Nalbuphine | 0.23 |
| 6-O-mPEG$_6$-Nalbuphine | 0.11 |
| 6-O-mPEG$_9$-Nalbuphine | 0.10 |
| Atenolol | 0.11 |

FIG. 1

## Description

### CROSS REFERENCE TO RELATED APPLICATIONS

This application clauses priority to U.S. Provisional Patent Application No. 61/192,247 filed September 16, 2008 and to U.S. Patent Application No. 12/558,395 filed September 11, 2009, the disclosures of which are incorporated herein by reference in their entireties.

### FIELD

Among other things, the present invention relates to opioid agonists that are covalently bound to a water-soluble oligomer (i.e., opioid agonist oligomer conjugates), the conjugates having reduced potential for substance abuse, among other features and advantages, and related uses thereof.

### BACKGROUND

Opioid agonists, such as morphine, have long been used to treat patients suffering from pain. Opioid agonists exert their analgesic and other pharmacological effects through interactions with opioid receptors, of which there are three main classes: mu ($\mu$) receptors, kappa ($\kappa$) receptors, and delta ($\delta$) receptors. Many of the clinically used opioid agonists are relatively selective for mu receptors, although opioid agonists typically have agonist activity at other opioid receptors (particularly at increased concentrations).

Opioids exert their effects, at least in part, by selectively inhibiting the release of neurotransmitters, such as acetylcholine, norepinephrine, dopamine, serotonin, and substance P.

Pharmacologically, opioid agonists represent an important class of agents employed in the management of pain. Opioid agonists currently used in analgesia, however, contain considerable addictive properties that complicate and limit their use in therapeutic practice. The medical, social and financial complications arising from opioid abuse impose severe constraints on the ability of physicians to prescribe opioids for use in chronic pain.

Typical opioids pass rapidly through the blood-brain-barrier (BBB) and rapidly reach peak concentrations that relate to the "highs" experienced by opioid abusers. Evidence indicates that reduced addictive properties can be achieved through an altered pharmacokinetic profile, which would deliver opioids at a constant low concentration to the brain, avoiding the concentration peaks of traditional modes of delivery that underlie the addictive potential of opioid agonists. Balster and Schuster, J Exp Anal Behav 20:119-129 (1973); Panlilio and Schindler, Psychopharmacology 150:61-66 (2000); Winger et al., J Pharmacol Exp Ther 301:690-697 (2002); Ko et al., J Pharmacol Exp Ther 301:698-704; Abreu et al., Psychopharmacologia 154:76-84 (2001). Development efforts in this regard have focused primarily on alternative delivery strategies, such as orally administered delayed release tablets and transdermal patches. These aim to supply a constant low concentration of drug to the circulation, but are complicated by the fact that they can be physically disrupted by crushing or cutting up, enabling the drug to be accessed and then injected directly into the circulation to give the desired pharmacokinetic profile for addictive behavior.

Thus there exists a need in the art for opioid agonists with low addiction properties and concomitant low abuse potential over currently available opioids used in analgesia. In particular, there exists a need for modifications to opioid agonists that alter the molecule itself and slow the penetration of the blood-brain-barrier such that direct injection of the drug would not provide the immediate central nervous system penetration that underlies the addictive "rush." The present invention seeks to address this and other needs by providing opioid agonists covalently bound to a water-soluble oligomer that retain their analgesic properties but have a reduced potential for substance abuse.

### SUMMARY

Accordingly, in one aspect, provided herein is a compound of the formula OP-X-POLY, wherein OP is an opioid compound, X is a linker, and POLY is a small water-soluble oligomer.

In a related embodiment, provided is a composition comprising a compound of the formula OP-X-POLY (where OP, X and POLY are as defined above) and a pharmaceutically acceptable excipient or carrier.

In another aspect, provided is a method of treating a patient in need of opioid therapy comprising administering an effective amount of a compound of the formula OP-X-POLY.

In yet another aspect, the provided is a method of reducing the abuse potential of an opioid compound comprising conjugating the compound to a small water-soluble oligomer.

In a further aspect, provided is a method of reducing the addictive properties of an opioid agonist comprising conjugating the opioid agonist to a small water-soluble oligomer.

In another aspect, provided is a method of reducing, but not substantially eliminating, the rate of crossing the

blood brain barrier of an opioid compound comprising conjugating the compound to a small water-soluble oligomer.

In yet another aspect, provided is a prodrug comprising a mu, kappa, or delta opioid agonist reversibly attached via a covalent bond to a releasable water soluble oligomeric moiety, wherein a given molar amount of the prodrug administered to a patient exhibits a rate of accumulation and a $C_{max}$ of the mu, kappa, or delta opioid agonist in the central nervous system in the mammal that is less than the rate of accumulation and the $C_{max}$ of an equal molar amount of the mu, kappa, or delta opioid agonist had the mu, kappa, or delta opioid agonist not been administered as part of a prodrug.

These and other objects, aspects, embodiments and features of the invention will become more fully apparent when read in conjunction with the following detailed description.

## BRIEF DESCRIPTION OF THE FIGURES

**FIG. 1** is a graph showing brain:plasma ratios of various $PEG_{oligo}$-nalbuphine conjugates, as described in greater detail in Example 8. The plot demonstrates that PEG conjugation results in a decrease in the brain:plasma ratios of nalbuphine.

**FIG. 2** is a graph showing percent writhing per total number of mice, n, in the study group, versus dose of $mPEG_n$-O-morphine conjugate administered in an analgesic assay for evaluating the extent of reduction or prevention of visceral pain in mice as described in detail in Example 13. Morphine was used as a control; unconjugated parent molecule, morphine sulfate, was also administered to provide an additional point of reference. Conjugates belonging to the following conjugate series: $mPEG_{2-7,9}$-O-morphine were evaluated.

**FIG. 3** is a graph showing percent writhing per total number of mice, n, in the study group, versus dose of $mPEG_n$-O-hydroxycodone conjugate administered in an analgesic assay for evaluating the extent of reduction or prevention of visceral pain in mice as described in detail in Example 13. Morphine was used as a control; unconjugated parent molecule, oxycodone, was also administered to provide an additional point of reference. Conjugates belonging to the following conjugate series: $mPEG_{1-4, 6, 7,,9}$-O-hydroxycodone were evaluated.

**FIG. 4** is a graph showing percent writhing per total number of mice, n, in the study group, versus dose of $mPEG_n$-O-codeine conjugate administered in an analgesic assay for evaluating the extent of reduction or prevention of visceral pain in mice as described in detail in Example 13. Morphine was used as a control; unconjugated parent molecule, codeine, was also administered to provide an additional point of reference. Conjugates belonging to the following conjugate series: $mPEG_{3-7,,9}$-O-codeine were evaluated.

**FIGS. 5 - 7** are plots indicating the results of a hot plate latency analgesic assay in mice as described in detail in Example 14. Specifically, the figures correspond to graphs showing latency (time to lick hindpaw), in seconds versus dose of compound. **FIG. 5** provides results for $mPEG_{1-5}$-I-hydroxycodone conjugates as well as for unconjugated parent molecule; **FIG. 6** provides results for $mPEG_{1-5}$-O-morphine conjugates as well for unconjugated parent molecule; and **FIG. 7** provides results for $mPEG_{2-5}$, 9-O-codeine conjugates as well as for the parent molecule. The presence of an asterisk by a data point indicates $p < 0.05$ versus saline by ANOVA/Dunnett's.

**FIG. 8** shows the mean (+SD) plasma concentration-time profiles for the compounds, oxycodone ($mPEG_0$-oxycodone), $mPEG_1$-O-hydroxycodone, $mPEG_2$-O-hydroxycodone, $mPEG_3$-O-hydroxycodone, $mPEG_4$-O-hydroxycodone, $mPEG_5$-O-hydroxycodone, $mPEG_6$-O-hydroxycodone, $mPEG_7$-O-hydroxycodone, and $mPEG_9$-O-hydroxycodone, following 1.0 mg/kg intravenous administration to rats as described in Example 16.

**FIG. 9** shows the mean (+SD) plasma concentration-time profiles for the compounds, oxycodone ($mPEG_0$-oxycodone), $mPEG_1$-O-hydroxycodone, $mPEG_2$-O-hydroxycodone, $mPEG_3$-O-hydroxycodone, $mPEG_4$-O-hydroxycodone, $mPEG_5$-O-hydroxycodone, $mPEG_6$-O-hydroxycodone, $mPEG_7$-O-hydroxycodone, and $mPEG_9$-O-hydroxycodone, following 5.0 mg/kg oral administration to rats as described in Example 16.

**FIG. 10** shows the mean (+SD) plasma concentration-time profiles for the compounds, morphine ($mPEG_0$-morphine), and $mPEG_{1-7,9}$-O-morphine conjugates, following 1.0 mg/kg intravenous administration to rats as described in detail in Example 17.

**FIG. 11** shows the mean (+SD) plasma concentration-time profiles for the compounds, morphine ($mPEG_0$-morphine),

and $mPEG_{1-7,9}$-O-morphine conjugates, following 5.0 mg/kg oral administration to rats as described in Example 17.

**FIG. 12** shows the mean (+SD) plasma concentration-time profiles for the compounds, codeine ($mPEG_0$-codeine), and $mPEG_{1-7,9}$-O-codeine conjugates, following 1.0 mg/kg intravenous administration to rats as described in detail in Example 18.

**FIG. 13** shows the mean (+SD) plasma concentration-time profiles for the compounds, codeine ($mPEG_0$-codeine), and $mPEG_{1-7,9}$-O-codeine conjugates, following 5.0 mg/kg oral administration to rats as described in Example 18.

**FIGS. 14A, 14B** and **14C** illustrate the brain:plasma ratios of various oligomeric $mPEG_n$-O-morphine, $mPEG_n$-O-codeine and $mPEG_n$-O-hydroxycodone conjugates, respectively, following IV administration to rats as described in Example 21. The brain:plasma ratio of atenolol is provided in each figure as a basis for comparison.

**FIGS. 15A-H** illustrate brain and plasma concentrations of morphine and various $mPEG_n$-O-morphine conjugates over time following IV administration to rats as described in Example 22. FIG. 15A (morphine, n=0); FIG. 15B (n=1); FIG. 15C (n=2); FIG. 15D (n=3); FIG. 15E (n=4); FIG. 15F (n=5); FIG. 15G (n=6); FIG. 15H (n=7).

**FIGS. 16A-H** illustrate brain and plasma concentrations of codeine and various $mPEG_n$-O-codeine conjugates over time following IV administration to rats as described in Example 22. FIG. 16A (codeine, n=0); FIG. 16B (n=1); FIG. 16C (n=2); FIG. 16D (n=3); FIG. 16E (n=4); FIG. 16F (n=5); FIG. 16G (n=6); FIG. 16H (n=7).

**FIGS. 17A-H** illustrate brain and plasma concentrations of oxycodone and various $mPEG_n$-O-hydroxycodone conjugates over time following IV administration to rats as described in Example 22. FIG. 17A (oxycodone, n=0); FIG. 17B (n=1); FIG. 17C (n=2); FIG. 17D (n=3); FIG. 17E (n=4); FIG. 17F (n=5); FIG. 17G (n=6); FIG. 17H (n=7).

**FIGS. 18A-C** illustrate the rate of brain penetration (Kin values) of certain exemplary $PEG_{olig}$-opioid conjugates in comparison to control compounds, antipyrine and unconjugated opioid, as described in detail in Example 3. Specifically, FIG. 18A illustrates the results for $mPEG_n$-O-morphine conjugates (where n= 1, 2, 3, and 7) in comparison to the control compounds, morphine and antipyrine. FIG. 18B illustrates the results for $mPEG_n$-O-codeine conjugates (where n= 2, 3, and 7) in comparison to the control compounds, codeine and antipyrine. FIG. 18C illustrates the results for $mPEG_n$-O-hydroxycodone conjugates (where n= 1, 2, 3, and 7) in comparison to the control compounds, oxycodone and antipyrine.

**FIG. 19** provides a graph illustrating rate of brain penetration, Kin, versus PEG oligomer size for mPEGn-O-morphine, mPEGn-O-codeine, and mPEGn-O-hydroxycodone conjugates as described in detail in Example 3.

## DETAILED DESCRIPTON OF THE INVENTION

### Definitions

As used in this specification, the singular forms "a," "an," and "the" include plural referents unless the context clearly dictates otherwise.

In describing and clauseing the present invention, the following terminology will be used in accordance with the definitions described below.

The terms "opioid compound" and "opioid agonist" are broadly used herein to refer to an organic, inorganic, or organometallic compound typically having a molecular weight of less than about 1000 Daltons (and typically less than 500 Daltons) and having some degree of activity as a mu, delta and/or kappa agonist. Opioid agonists encompass oligopeptides and other biomolecules having a molecular weight of less than about 1500.

The terms "spacer moiety," "linkage" and "linker" are used herein to refer to an atom or a collection of atoms optionally used to link interconnecting moieties such as a terminus of a polymer segment and an opioid compound or an electrophile or nucleophile of an opioid compound. The spacer moiety may be hydrolytically stable or may include a physiologically hydrolyzable or enzymatically degradable linkage. Unless the context clearly dictates otherwise, a spacer moiety optionally exists between any two elements of a compound (*e.g.*, the provided conjugates comprising an opioid compound and a water-soluble oligomer that can be attached directly or indirectly through a spacer moiety).

"Water soluble oligomer" indicates a non-peptidic oligomer that is at least 35% (by weight) soluble, preferably greater than 70% (by weight), and more preferably greater than 95% (by weight) soluble, in water at room temperature. Typically, an unfiltered aqueous preparation of a "water-soluble" oligomer transmits at least 75%, more preferably at least 95%, of the amount of light transmitted by the same solution after filtering. It is most preferred, however, that the

water-soluble oligomer is at least 95% (by weight) soluble in water or completely soluble in water. With respect to being "non-peptidic," an oligomer is non-peptidic when it has less than 35% (by weight) of amino acid residues.

The terms "monomer," "monomeric subunit" and "monomeric unit" are used interchangeably herein and refer to one of the basic structural units of a polymer or oligomer. In the case of a homo-oligomer, a single repeating structural unit forms the oligomer. In the case of a co-oligomer, two or more structural units are repeated -- either in a pattern or randomly -- to form the oligomer. Preferred oligomers used in connection with present the invention are homo-oligomers. The water-soluble oligomer typically comprises one or more monomers serially attached to form a chain of monomers. The oligomer can be formed from a single monomer type (*i.e.,* is homo-oligomeric) or two or three monomer types (*i.e.,* is co-oligomeric).

An "oligomer" is a molecule possessing from about 2 to about 50 monomers, preferably from about 2 to about 30 monomers. The architecture of an oligomer can vary. Specific oligomers for use in the invention include those having a variety of geometries such as linear, branched, or forked, to be described in greater detail below.

"PEG" or "polyethylene glycol," as used herein, is meant to encompass any water-soluble poly(ethylene oxide). Unless otherwise indicated, a "PEG oligomer" (also called an oligoethylene glycol) is one in which substantially all (and more preferably all) monomeric subunits are ethylene oxide subunits. The oligomer may, however, contain distinct end capping moieties or functional groups, *e.g.,* for conjugation. Typically, PEG oligomers for use in the present invention will comprise one of the two following structures: "$-(CH_2CH_2O)_n-$" or "$-(CH_2CH_2O)_{n-1}CH_2CH_2-$," depending upon whether the terminal oxygen(s) has been displaced, *e.g.*, during a synthetic transformation. For PEG oligomers, "n" varies from about 2 to 50, preferably from about 2 to about 30, and the terminal groups and architecture of the overall PEG can vary. When PEG further comprises a functional group, A, for linking to, *e.g.,* an opioid compound, the functional group when covalently attached to a PEG oligomer does not result in formation of (i) an oxygen-oxygen bond (-O-O-, a peroxide linkage), or (ii) a nitrogen-oxygen bond (N-O, O-N).

An "end capping group" is generally a non-reactive carbon-containing group attached to a terminal oxygen of a PEG oligomer. Exemplary end capping groups comprise a $C_{1-5}$ alkyl group, such as methyl, ethyl and benzyl), as well as aryl, heteroaryl, cyclo, heterocyclo, and the like. For the purposes of the present invention, the preferred capping groups have relatively low molecular weights such as methyl or ethyl. The end-capping group can also comprise a detectable label. Such labels include, without limitation, fluorescers, chemiluminescers, moieties used in enzyme labeling, colorimetric labels (*e.g.,* dyes), metal ions, and radioactive moieties.

"Branched", in reference to the geometry or overall structure of an oligomer, refers to an oligomer having two or more polymers representing distinct "arms" that extend from a branch point.

"Forked" in reference to the geometry or overall structure of an oligomer, refers to an oligomer having two or more functional groups (typically through one or more atoms) extending from a branch point.

A "branch point" refers to a bifurcation point comprising one or more atoms at which an oligomer branches or forks from a linear structure into one or more additional arms.

The term "reactive" or "activated" refers to a functional group that reacts readily or at a practical rate under conventional conditions of organic synthesis. This is in contrast to those groups that either do not react or require strong catalysts or impractical reaction conditions in order to react (*i.e.,* a "nonreactive" or "inert" group).

"Not readily reactive," with reference to a functional group present on a molecule in a reaction mixture, indicates that the group remains largely intact under conditions that are effective to produce a desired reaction in the reaction mixture.

A "protecting group" is a moiety that prevents or blocks reaction of a particular chemically reactive functional group in a molecule under certain reaction conditions. The protecting group will vary depending upon the type of chemically reactive group being protected as well as the reaction conditions to be employed and the presence of additional reactive or protecting groups in the molecule. Functional groups which may be protected include, by way of example, carboxylic acid groups, amino groups, hydroxyl groups, thiol groups, carbonyl groups and the like. Representative protecting groups for carboxylic acids include esters (such as a *p*-methoxybenzyl ester), amides and hydrazides; for amino groups, carbamates (such as *tert*-butoxycarbonyl) and amides; for hydroxyl groups, ethers and esters; for thiol groups, thioethers and thioesters; for carbonyl groups, acetals and ketals; and the like. Such protecting groups are well-known to those skilled in the art and are described, for example, in T.W. Greene and G.M. Wuts, Protecting Groups in Organic Synthesis, Third Edition, Wiley, New York, 1999, and references cited therein.

A functional group in "protected form" refers to a functional group bearing a protecting group. As used herein, the term "functional group" or any synonym thereof encompasses protected forms thereof.

A "physiologically cleavable" bond is a hydrolyzable bond or an enzymatically degradable linkage. A "hydrolyzable" or "degradable" bond is a relatively labile bond that reacts with water (*i.e.,* is hydrolyzed) under ordinary physiological conditions. The tendency of a bond to hydrolyze in water under ordinary physiological conditions will depend not only on the general type of linkage connecting two central atoms but also on the substituents attached to these central atoms. Such bonds are generally recognizable by those of ordinary skill in the art. Appropriate hydrolytically unstable or weak linkages include but are not limited to carboxylate ester, phosphate ester, anhydrides, acetals, ketals,

acyloxyalkyl ether, imines, orthoesters, peptides, oligonucleotides, thioesters, and carbonates.

An "enzymatically degradable linkage" means a linkage that is subject to degradation by one or more enzymes under ordinary physiological conditions.

"Releasably attached," *e.g.,* in reference to an opioid compound releasably attached to a water-soluble oligomer, refers to an opioid compound that is covalently attached via a linker that includes a physiologically cleavable or degradable (including enzymatically) linkage as disclosed herein, wherein upon degradation (*e.g.*, by hydrolysis), the opioid compound is released. The opioid compound thus released will typically correspond to the unmodified opioid compound, or may be slightly altered, *e.g.*, possessing a short organic tag of about 8 atoms, *e.g.*, typically resulting from cleavage of a part of the water-soluble oligomer linker not immediately adjacent to the opioid compound. Preferably, the unmodified opioid compound is released.

A "stable" linkage or bond refers to a chemical moiety or bond, typically a covalent bond, that is substantially stable in water, that is to say, does not undergo hydrolysis under ordinary physiological conditions to any appreciable extent over an extended period of time. Examples of hydrolytically stable linkages include but are not limited to the following: carbon-carbon bonds (*e.g.,* in aliphatic chains), ethers, amides, urethanes, amines, and the like. Generally, a stable linkage is one that exhibits a rate of hydrolysis of less than about 1-2% per day under ordinary physiological conditions. Hydrolysis rates of representative chemical bonds can be found in most standard chemistry textbooks.

In the context of describing the consistency of oligomers in a given composition, "substantially" or "essentially" means nearly totally or completely, for instance, 95% or greater, more preferably 97% or greater, still more preferably 98% or greater, even more preferably 99% or greater, yet still more preferably 99.9% or greater, with 99.99% or greater being most preferred of some given quantity.

"Monodisperse" refers to an oligomer composition wherein substantially all of the oligomers in the composition have a well-defined, single molecular weight and defined number of monomers, as determined by chromatography or mass spectrometry. Monodisperse oligomer compositions are in one sense pure, that is, substantially comprising molecules having a single and definable number of monomers rather than several different numbers of monomers (*i.e.,* an oligomer composition having three or more different oligomer sizes). A monodisperse oligomer composition possesses a MW/Mn value of 1.0005 or less, and more preferably, a MW/Mn value of 1.0000. By extension, a composition comprised of monodisperse conjugates means that substantially all oligomers of all conjugates in the composition have a single and definable number (as a whole number) of monomers rather than a distribution and would possess a MW/Mn value of 1.0005, and more preferably, a MW/Mn value of 1.0000 if the oligomer were not attached to the residue of the opioid agonist. A composition comprised of monodisperse conjugates can include, however, one or more nonconjugate substances such as solvents, reagents, excipients, and so forth.

"Bimodal," in reference to an oligomer composition, refers to an oligomer composition wherein substantially all oligomers in the composition have one of two definable and different numbers (as whole numbers) of monomers rather than a distribution, and whose distribution of molecular weights, when plotted as a number fraction versus molecular weight, appears as two separate identifiable peaks. Preferably, for a bimodal oligomer composition as described herein, each peak is generally symmetric about its mean, although the size of the two peaks may differ. Ideally, the polydispersity index of each peak in the bimodal distribution, Mw/Mn, is 1.01 or less, more preferably 1.001 or less, and even more preferably 1.0005 or less, and most preferably a MW/Mn value of 1.0000. By extension, a composition comprised of bimodal conjugates means that substantially all oligomers of all conjugates in the composition have one of two definable and different numbers (as whole numbers) of monomers rather than a large distribution and would possess a MW/Mn value of 1.01 or less, more preferably 1.001 or less and even more preferably 1.0005 or less, and most preferably a MW/Mn value of 1.0000 if the oligomer were not attached to the residue of the opioid agonist. A composition comprised of bimodal conjugates can include, however, one or more nonconjugate substances such as solvents, reagents, excipients, and so forth.

A "biological membrane" is any membrane, typically made from specialized cells or tissues, that serves as a barrier to at least some foreign entities or otherwise undesirable materials. As used herein a "biological membrane" includes those membranes that are associated with physiological protective barriers including, for example: the blood-brain barrier (BBB); the blood-cerebrospinal fluid barrier; the blood-placental barrier; the blood-milk barrier; the blood-testes barrier; and mucosal barriers including the vaginal mucosa, urethral mucosa, anal mucosa, buccal mucosa, sublingual mucosa, rectal mucosa, and so forth. In certain contexts the term "biological membrane" does not include those membranes associated with the middle gastro-intestinal tract (*e.g.*, stomach and small intestines) For example, in some instances it may be desirable for a compound of the invention to have a limited ability to cross the blood-brain barrier, yet be desirable that the same compound cross the middle gastro-intestinal tract.

A "biological membrane crossing rate," as used herein, provides a measure of a compound's ability to cross a biological membrane (such as the membrane associated with the blood-brain barrier). A variety of methods can be used to assess transport of a molecule across any given biological membrane. Methods to assess the biological membrane crossing rate associated with any given biological barrier (*e.g.,* the blood-cerebrospinal fluid barrier, the blood-placental barrier, the blood-milk barrier, the intestinal barrier, and so forth), are known in the art, described herein and/or in the

relevant literature, and/or can be determined by one of ordinary skill in the art.

"Alkyl" refers to a hydrocarbon chain, typically ranging from about 1 to 20 atoms in length. Such hydrocarbon chains are preferably but not necessarily saturated and may be branched or straight chain, although typically straight chain is preferred. Exemplary alkyl groups include methyl, ethyl, propyl, butyl, pentyl, 2-methylbutyl, 2-ethylpropyl, 3-methylpentyl, and the like. As used herein, "alkyl" includes cycloalkyl when three or more carbon atoms are referenced. An "alkenyl" group is an alkyl of 2 to 20 carbon atoms with at least one carbon-carbon double bond.

The terms "substituted alkyl" or "substituted $C_{q-r}$ alkyl" where q and r are integers identifying the range of carbon atoms contained in the alkyl group, denotes the above alkyl groups that are substituted by one, two or three halo (*e.g.,* F, Cl, Br, I), trifluoromethyl, hydroxy, $C_{1-7}$ alkyl (*e.g.*, methyl, ethyl, n-propyl, isopropyl, butyl, t-butyl, and so forth), $C_{1-7}$ alkoxy, $C_{1-7}$ acyloxy, $C_{3-7}$ heterocyclic, amino, phenoxy, nitro, carboxy, carboxy, acyl, cyano. The substituted alkyl groups may be substituted once, twice or three times with the same or with different substituents.

"Lower alkyl" refers to an alkyl group containing from 1 to 6 carbon atoms, and may be straight chain or branched, as exemplified by methyl, ethyl, n-butyl, i-butyl, t-butyl. "Lower alkenyl" refers to a lower alkyl group of 2 to 6 carbon atoms having at least one carbon-carbon double bond.

"Non-interfering substituents" are those groups that, when present in a molecule, are typically non-reactive with other functional groups contained within the molecule.

"Alkoxy" refers to an -O-R group, wherein R is alkyl or substituted alkyl, preferably $C_1$-$C_{20}$ alkyl (*e.g.*, methoxy, ethoxy, propyloxy, benzyl, etc.), preferably $C_1$-$C_7$.

"Pharmaceutically acceptable excipient" or "pharmaceutically acceptable carrier" refers to component that can be included in the compositions of the invention in order to provide for a composition that has an advantage (*e.g.*, more suited for administration to a patient) over a composition lacking the component and that is recognized as not causing significant adverse toxicological effects to a patient.

The term "aryl" means an aromatic group having up to 14 carbon atoms. Aryl groups include phenyl, naphthyl, biphenyl, phenanthrenyl, naphthacenyl, and the like. "Substituted phenyl" and "substituted aryl" denote a phenyl group and aryl group, respectively, substituted with one, two, three, four or five (*e.g.* 1-2, 1-3 or 1-4 substituents) chosen from halo (F, Cl, Br, I), hydroxy, hydroxy, cyano, nitro, alkyl (*e.g.,* $C_{1-6}$ alkyl), alkoxy (*e.g.,* $C_{1-6}$ alkoxy), benzyloxy, carboxy, aryl, and so forth.

An "aromatic-containing moiety" is a collection of atoms containing at least aryl and optionally one or more atoms. Suitable aromatic-containing moieties are described herein.

For simplicity, chemical moieties are defined and referred to throughout primarily as univalent chemical moieties (*e.g.*, alkyl, aryl, etc.). Nevertheless, such terms are also used to convey corresponding multivalent moieties under the appropriate structural circumstances clear to those skilled in the art. For example, while an "alkyl" moiety generally refers to a monovalent radical (*e.g.,* $CH_3$-$CH_2$-), in certain circumstances a bivalent linking moiety can be "alkyl," in which case those skilled in the art will understand the alkyl to be a divalent radical (*e.g.*, -$CH_2$-$CH_2$-), which is equivalent to the term "alkylene." (Similarly, in circumstances in which a divalent moiety is required and is stated as being "aryl," those skilled in the art will understand that the term "aryl" refers to the corresponding divalent moiety, arylene). All atoms are understood to have their normal number of valences for bond formation (*i.e.,* 4 for carbon, 3 for N, 2 for O, and 2, 4, or 6 for S, depending on the oxidation state of the S).

"Pharmacologically effective amount," "physiologically effective amount," and "therapeutically effective amount" are used interchangeably herein to mean the amount of a water-soluble oligomer-opioid compound conjugate present in a composition that is needed to provide a threshold level of active agent and/or conjugate in the bloodstream or in the target tissue. The precise amount will depend upon numerous factors, *e.g.,* the particular active agent, the components and physical characteristics of the composition, intended patient population, patient considerations, and the like, and can readily be determined by one skilled in the art, based upon the information provided herein and available in the relevant literature.

A "difunctional" oligomer is an oligomer having two functional groups contained therein, typically at its termini. When the functional groups are the same, the oligomer is said to be homodifunctional. When the functional groups are different, the oligomer is said to be heterobifunctional.

A basic reactant or an acidic reactant described herein include neutral, charged, and any corresponding salt forms thereof.

The term "patient," refers to a living organism suffering from or prone to a condition that can be prevented or treated by administration of a conjugate as described herein, typically, but not necessarily, in the form of a water-soluble oligomer-opioid compound conjugate, and includes both humans and animals.

"Optional" or "optionally" means that the subsequently described circumstance may but need not necessarily occur, so that the description includes instances where the circumstance occurs and instances where it does not.

Unless the context clearly dictates otherwise, when the term "about" precedes a numerical value, the numerical value is understood to mean the stated numerical value and also $\pm$ 10% of the stated numerical value.

**Water Soluble Oligomer Opioid Conjugates**

As indicated above, the present disclosure is directed to (among other things) compounds of the formula:

OP-X-POLY

wherein OP is an opioid compound, X is a linker, and POLY is a small water-soluble oligomer. In preparing and characterizing the subject conjugates, the inventors have discovered that derivatization of an opioid compound with a small water-soluble oligomer reduces the speed of delivery of the opioid compound to the brain. Based on the covalent modification of the opioid agonist molecule itself, the conjugates described herein represent an improvement over the anti-abuse opioid agonist formulations of the prior art. That is to say, opioid compounds conjugated with small water-oligomers possess altered pharmacokinetic profiles, but are not subject to the risk of physical tampering that allows for the recovery and abuse of the rapid acting opioid compound associated with certain alternative delivery formulations such as transdermal patches.

Opioids

Accordingly, OP can be any opioid compound, including any compound interacting with mu ($\mu$), kappa ($\kappa$), or delta ($\delta$) opioid receptors, or any combination thereof. In one embodiment, the opioid is selective for the mu ($\mu$) opioid receptor. In another embodiment, the opioid is selective for the kappa ($\kappa$) opioid receptor. In a further embodiment, the opioid is selective for the delta ($\delta$) opioid receptor. Opioids suitable for use can be naturally occurring, semi-synthetic or synthetic molecules.

Opioid compounds that may be used include, but are not limited to, acetorphine, acetyldihydrocodeine, acetyldihydrocodeinone, acetylmorphinone, alfentanil, allylprodine, alphaprodine, anileridine, benzylmorphine, bezitramide, biphalin, buprenorphine, butorphanol, clonitazene, codeine, desomorphine, dextromoramide, dezocine, diampromide, diamorphone, dihydrocodeine, dihydromorphine, dimenoxadol, dimepheptanol, dimethylthiambutene, dioxaphetyl butyrate, dipipanone, dynorphins (including dynorphin A and dynorphin B), endorphins (including beta-endorphin and $\alpha$/$\beta$-neo-endorphin), enkephalins (including Met-enkephalin and Leu-enkephalin), eptazocine, ethoheptazine, ethylmethylthiambutene, ethylmorphine, etonitazene, etorphine, dihydroetorphine, fentanyl and derivatives, heroin, hydrocodone, hydromorphone, hydroxypethidine, isomethadone, ketobemidone, levorphanol, levophenacylmorphan, lofentanil, meperidine, meptazinol, metazocine, methadone, metopon, morphine, myrophine, narceine, nicomorphine, norlevorphanol, normethadone, nalorphine, nalbuphine, normorphine, norpipanone, opium, oxycodone, oxymorphone, papaveretum, pentazocine, phenadoxone, phenomorphan, phenazocine, phenoperidine, piminodine, piritramide, propheptazine, promedol, properidine, propoxyphene, sufentanil, tilidine, and tramadol.

In certain embodiments, the opioid agonist is selected from the group consisting of hydrocodone, morphine, hydromorphone, oxycodone, codeine, levorphanol, meperidine, methadone, oxymorphone, buprenorphine, fentanyl, dipipanone, heroin, tramadol, nalbuphine, etorphine, dihydroetorphine, butorphanol, and levorphanol.

In other embodiments, the opioid agonist is selected from the group consisting of fentanyl, hydromorphone, methadone, morphine, codeine, oxycodone, and oxymorphone.

Any other opioid compound having opioid agonist activity may also be used. Assays for determining whether a given compound (regardless of whether the compound is in conjugated form or not) can act as an agonist on an opioid receptor are described herein and are known in the art.

In some instances, opioid agonists can be obtained from commercial sources. In addition, opioid agonists can be synthesized using standard techniques of synthetic organic chemistry. Synthetic approaches for preparing opioid agonists are described in the literature and in, for example, U.S. Patent Nos.: 2,628,962, 2,654,756, 2,649,454, and 2,806,033.

Each of these (and other) opioid agonists can be covalently attached (either directly or through one or more atoms) to a water-soluble oligomer.

Opioid compounds useful in the invention generally have a molecular weight of less than about 1500 Da (Daltons), and even more typically less than about 1000 Da. Exemplary molecular weights of opioid compounds include molecular weights of: less than about 950 Da; less than about 900 Da; less than about 850 Da; less than about 800 Da; less than about 750 Da; less than about 700 Da; less than about 650 Da; less than about 600 Da; less than about 550 Da; less than about 500 Da; less than about 450 Da; less than about 400 Da; less than about 350 Da; and less than about 300 Da.

The opioid compounds used in the invention, if chiral, may be in a racemic mixture, or an optically active form, for example, a single optically active enantiomer, or any combination or ratio of enantiomers (*i.e.,* scalemic mixture). In addition, the opioid compound may possess one or more geometric isomers. With respect to geometric isomers, a composition can comprise a single geometric isomer or a mixture of two or more geometric isomers. An opioid compound

for use in the present invention can be in its customary active form, or may possess some degree of modification. For example, an opioid compound may have a targeting agent, tag, or transporter attached thereto, prior to or after covalent attachment of a water-soluble oligomer. Alternatively, the opioid compound may possess a lipophilic moiety attached thereto, such as a phospholipid (*e.g.,* distearoylphosphatidylethanolamine or "DSPE," dipalmitoylphosphatidylethanolamine or "DPPE," and so forth) or a small fatty acid. In some instances, however, it is preferred that the opioid compound does not include attachment to a lipophilic moiety.

The opioid agonist for coupling to a water-soluble oligomer possesses a free hydroxyl, carboxyl, thio, amino group, or the like (*i.e.,* "handle") suitable for covalent attachment to the oligomer. In addition, the opioid agonist can be modified by introduction of a reactive group, preferably by conversion of one of its existing functional groups to a functional group suitable for formation of a stable covalent linkage between the oligomer and the opioid compound.

Water Soluble Oligomer

Accordingly, each oligomer is composed of up to three different monomer types selected from the group consisting of: alkylene oxide, such as ethylene oxide or propylene oxide; olefinic alcohol, such as vinyl alcohol, 1-propenol or 2-propenol; vinyl pyrrolidone; hydroxyalkyl methacrylamide or hydroxyalkyl methacrylate, where alkyl is preferably methyl; $\alpha$-hydroxy acid, such as lactic acid or glycolic acid; phosphazene, oxazoline, amino acids, carbohydrates such as monosaccharides, saccharide or mannitol; and N-acryloylmorpholine. Preferred monomer types include alkylene oxide, olefinic alcohol, hydroxyalkyl methacrylamide or methacrylate, N-acryloylmorpholine, and $\alpha$-hydroxy acid. Preferably, each oligomer is, independently, a co-oligomer of two monomer types selected from this group, or, more preferably, is a homo-oligomer of one monomer type selected from this group.

The two monomer types in a co-oligomer may be of the same monomer type, for example, two alkylene oxides, such as ethylene oxide and propylene oxide. Preferably, the oligomer is a homo-oligomer of ethylene oxide. Usually, although not necessarily, the terminus (or termini) of the oligomer that is not covalently attached to an opioid compound is capped to render it unreactive. Alternatively, the terminus may include a reactive group. When the terminus is a reactive group, the reactive group is either selected such that it is unreactive under the conditions of formation of the final oligomer or during covalent attachment of the oligomer to an opioid compound, or it is protected as necessary. One common end-functional group is hydroxyl or -OH, particularly for oligoethylene oxides.

The water-soluble oligomer (*e.g.*, "POLY" in the structures provided herein) can have any of a number of different geometries. For example, it can be linear, branched, or forked. Most typically, the water-soluble oligomer is linear or is branched, for example, having one branch point. Although much of the discussion herein is focused upon poly(ethylene oxide) as an illustrative oligomer, the discussion and structures presented herein can be readily extended to encompass any of the water-soluble oligomers described above.

The molecular weight of the water-soluble oligomer, excluding the linker portion, in certain embodiments is generally relatively low. For example, the molecular weight of the water-soluble oligomer is typically below about 2200 Daltons, and more typically at around 1500 Daltons or below. In certain other embodiments, the molecular weight of the water-soluble oligomer may be below 800 Daltons.

In certain embodiments, exemplary values of the molecular weight of the water-soluble oligomer include less than or equal to about 500 Daltons, or less than or equal to about 420 Daltons, or less than or equal to about 370 Daltons, or less than or equal to about 370 Daltons, or less than or equal to about 325 Daltons, less than or equal to about 280 Daltons, less than or equal to about 235 Daltons, or less than or equal to about 200 Daltons, less than or equal to about 175 Daltons, or less than or equal to about 150 Daltons, or less than or equal to about 135 Daltons, less than or equal to about 90 Daltons, or less than or equal to about 60 Daltons, or evan less than or equal to about 45 Daltons.

In other embodiments, exemplary values of the molecular weight of the water-soluble oligomer, excluding the linker portion, include: below about 1500 Daltons; below about 1450 Daltons; below about 1400 Daltons; below about 1350 Daltons; below about 1300 Daltons; below about 1250 Daltons; below about 1200 Daltons; below about 1150 Daltons; below about 1100 Daltons; below about 1050 Daltons; below about 1000 Daltons; below about 950 Daltons; below about 900 Daltons; below about 850 Daltons; below about 800 Daltons; below about 750 Daltons; below about 700 Daltons; below about 650 Daltons; below about 600 Daltons; below about 550 Daltons; below about 500 Daltons; below about 450 Daltons; below about 400 Daltons; and below about 350 Daltons; but in each case above about 250 Daltons.

In certain other embodiments, rather than being bound to an oligomer, the opioid is covalently attached to a water-soluble polymer, i.e., a moiety having a more than 50 repeating subunits. For instance, the molecular weight of the water-soluble polymer, excluding the linker portion, may be below about 80,000 Daltons; below about 70,000 Daltons; below about 60,000 Daltons; below about 50,000 Daltons; below about 40,000 Daltons; below about 30,000 Daltons; below about 20,000 Daltons; below about 10,000 Daltons; below about 8,000 Daltons; below about 6,000 Daltons; below about 4,000 Daltons; below about 3,000 Daltons; and below about 2,000 Daltons; but in each case above about 250 Daltons.

In certain embodiments, exemplary ranges of molecular weights of the water-soluble, oligomer (excluding the linker) include: from about 45 to about 225 Daltons; from about 45 to about 175 Daltons; from about 45 to about 135 Daltons; from about 45 to about 90 Daltons; from about 90 to about 225 Daltons; from about 90 to about 175 Daltons; from about 90 to about 135 Daltons; from about 135 to about 225 Daltons; from about 135 to about 175 Daltons; and from about 175 to about 225 Daltons.

In other alternative embodiments, exemplary ranges of molecular weights of the water-soluble oligomer (excluding the linker) include: from about 250 to about 1500 Daltons; from about 250 to about 1200 Daltons; from about 250 to about 800 Daltons; from about 250 to about 500 Daltons; from about 250 to about 400 Daltons; from about 250 to about 500 Daltons; from about 250 to about 1000 Daltons; and from about 250 to about 500 Daltons.

In other embodiments related to water-soluble polymer bound opioids, exemplary ranges of molecular weights of the water-soluble polymer (excluding the linker) include: from about 2,000 to about 80,000 Daltons; from about 2,000 to about 70,000 Daltons; from about 2,000 to about 60,000 Daltons; from about 2,000 to about 50,000 Daltons; from about 2,000 to about 40,000 Daltons; from about 2,000 to about 30,000 Daltons; from about 2,000 to about 20,000 Daltons; from about 2,000 to about 10,000 Daltons; from about 2,000 to about 8,000 Daltons; from about 2,000 to about 6,000 Daltons; from about 2,000 to about 4,000 Daltons; from about 2,000 to about 3,000 Daltons; from about 10,000 to about 80,000 Daltons; from about 10,000 to about 60,000 Daltons; from about 10,000 to about 40,000 Daltons; from about 30,000 to about 80,000 Daltons; from about 30,000 to about 60,000 Daltons; from about 40,000 to about 80,000 Daltons; and from about 60,000 to about 80,000 Daltons.

The number of monomers in the water-soluble oligomer may be between about 1 and about 1825 (inclusive), including all integer values within this range.

Preferably, in some embodiments, the number of monomers in the water-soluble oligomer falls within one or more of the following inclusive ranges: between 1 and 5 (i.e., is selected from 1, 2, 3, 4, and 5); between 1 and 4 (i.e., can be 1, 2, 3, or 4); between 1 and 3 (i.e., selected from 1, 2, or 3); between 1 and 2 (i.e., can be 1 or 2); between 2 and 5 (i.e., can be selected from 2, 3, 4, and 5); between 2 and 4 (i.e., is selected from 2, 3, and 4); between 2 and 3 (i.e., is either 2 or 3); between 3 and 5 (i.e., is either 3, 4 or 5); between 3 and 4 (i.e., is 3 or 4); and between 4 and 5 (i.e., is 4 or 5). In a specific instance, the number of monomers in series in the oligomer (and the corresponding conjugate) is selected from 1, 2, 3, 4, or 5. Thus, for example, when the water-soluble oligomer includes $CH_3$-$(OCH_2CH_2)_n$-, "n" is an integer that can be 1, 2, 3, 4, or 5.

Preferably, in other embodiments\, the number of monomers in the water-soluble oligomer falls within one or more of the following inclusive ranges: between 6 and 30 (i.e., is selected from 6, 7, 8, 9, 10, 11, 12, 13, 14, 15, 16, 17, 18, 19, 20, 21, 22, 23, 24, 25, 26, 27, 28, 29, and 30); between 6 and 25 (i.e., is selected from 6, 7, 8, 9, 10, 11, 12, 13, 14, 15, 16, 17, 18, 19, 20, 21, 22, 23, 24, and 25); between 6 and 20 (i.e., is selected from 6, 7, 8, 9, 10, 11, 12, 13, 14, 15, 16, 17, 18, 19, and 20); between 6 and 15 (is selected from 6, 7, 8, 9, 10, 11, 12, 13, 14, 15); between 6 and 10 (i.e., is selected from 6, 7, 8, 9, and 10); between 10 and 25 (i.e., is selected from 10, 11, 12, 13, 14, 15, 16, 17, 18, 19, 20, 21, 22, 23, 24, and 25); and between 15 and 20 (i.e., is selected from 15, 16, 17, 18, 19, and 20). In certain instances, the number of monomers in series in the oligomer (and the corresponding conjugate) is one of 6, 7, 8, 9, 10, 11, 12, 13, 14, 15, 16, 17, 18, 19, 20, 21, 22, 23, 24, or 25. Thus, for example, when the water-soluble oligomer includes $CH_3$-$(OCH_2CH_2)_n$-, "n" is an integer that can be 6, 7, 8, 9, 10, 11, 12, 13, 14, 15, 16, 17, 18, 19, 20, 21, 22, 23, 24, or 25.

In certain other embodiments, the number of monomers in the water-soluble oligomer falls within one or more of the following inclusive ranges: between 35 and 1825; between 100 and 1800; between 200 and 1600; between 400 and 1400; between 600 and 1200; between 800 and 1000; between 35 and 1000; between 35 and 600; between 35 and 400; between 35 and 200; between 35 and 100; between 1000 and 1825; between 1200 and 1825; between 1400 and 1825; and between 1600 and 1825.

When the water-soluble oligomer has 1, 2, 3, 4, or 5 monomers, these values correspond to a methoxy end-capped oligo(ethylene oxide) having a molecular weight of about 75, 119, 163, 207, and 251 Daltons, respectively. When the oligomer has 6, 7, 8, 9, 10, 11, 12, 13, 14, or 15 monomers, these values correspond to a methoxy end-capped oligo(ethylene oxide) having a molecular weight of about 295, 339, 383, 427, 471, 515, 559, 603, 647, and 691 Daltons, respectively.

When the water-soluble oligomer is attached to the opioid agonist (in contrast to the step-wise addition of one or more monomers to effectively "grow" the oligomer onto the opioid agonist), it is preferred that the composition containing an activated form of the water-soluble oligomer be monodispersed. In those instances, however, where a bimodal composition is employed, the composition will possess a bimodal distribution centering around any two of the above numbers of monomers. Ideally, the polydispersity index of each peak in the bimodal distribution, Mw/Mn, is 1.01 or less, and even more preferably, is 1.001 or less, and even more preferably is 1.0005 or less. Most preferably, each peak possesses a MW/Mn value of 1.0000. For instance, a bimodal oligomer may have any one of the following exemplary combinations of monomer subunits: 1-2, 1-3, 1-4, 1-5, 1-6, 1-7, 1-8, 1-9, 1-10, and so forth; 2-3, 2-4, 2-5, 2-6, 2-7, 2-8, 2-9, 2-10, and so forth; 3-4, 3-5, 3-6, 3-7, 3-8, 3-9, 3-10, and so forth; 4-5, 4-6, 4-7, 4-8, 4-9, 4-10, and so forth; 5-6, 5-7, 5-8, 5-9, 5-10, and so forth; 6-7, 6-8, 6-9, 6-10, and so forth; 7-8, 7-9, 7-10, and so forth; and 8-9, 8-10, and so forth.

In some instances, the composition containing an activated form of the water-soluble oligomer will be trimodal or even tetramodal, possessing a range of monomers units as previously described. Oligomer compositions possessing a well-defined mixture of oligomers (*i.e.,* being bimodal, trimodal, tetramodal, and so forth) can be prepared by mixing purified monodisperse oligomers to obtain a desired profile of oligomers (a mixture of two oligomers differing only in the number of monomers is bimodal; a mixture of three oligomers differing only in the number of monomers is trimodal; a mixture of four oligomers differing only in the number of monomers is tetramodal), or alternatively, can be obtained from column chromatography of a polydisperse oligomer by recovering the "center cut", to obtain a mixture of oligomers in a desired and defined molecular weight range.

It is preferred that the water-soluble oligomer is obtained from a composition that is preferably unimolecular or monodisperse. That is, the oligomers in the composition possess the same discrete molecular weight value rather than a distribution of molecular weights. Some monodisperse oligomers can be purchased from commercial sources such as those available from Sigma-Aldrich, or alternatively, can be prepared directly from commercially available starting materials such as Sigma-Aldrich. Water-soluble oligomers can be prepared as described in Chen and Baker, J. Org. Chem. 6870-6873 (1999), WO 02/098949, and U.S. Patent Application Publication 2005/0136031.

<u>Spacer/Linker Moiety</u>

When present, the spacer moiety (through which the water-soluble oligomer is attached to the opioid agonist) may be a single bond, a single atom, such as an oxygen atom or a sulfur atom, two atoms, or a number of atoms. In particular, "X" may represent a covalent bond between OP and POLY, or alternatively it may represent a chemical moiety not present on OP and/or POLY alone. A spacer moiety is typically but is not necessarily linear in nature. In certain embodiments, the spacer moiety, "X" is preferably hydrolytically stable, and is preferably also enzymatically stable. In other embodiments, the spacer moiety, "X" is preferably physiologically cleavable, *i.e.* hydrolytically cleavable or enzymatically degradable. Preferably, the spacer moiety "X" is one having a chain length of less than about 12 atoms, and preferably less than about 10 atoms, and even more preferably less than about 8 atoms and even more preferably less than about 5 atoms, whereby length is meant the number of atoms in a single chain, not counting substituents. For instance, a urea linkage such as this, $R_{oligomer}$-NH-(C=O)-NH-$R'_{OP,}$ is considered to have a chain length of 3 atoms (-NH-C(O)-NH-). In selected embodiments, the spacer moiety linkage does not comprise further spacer groups.

In some instances, the spacer moiety "X" comprises an ether, amide, urethane, amine, thioether, urea, or a carbon-carbon bond. Functional groups are typically used for forming the linkages. The spacer moiety may less preferably also comprise (or be adjacent to or flanked by) spacer groups, as described further below.

More specifically, in selected embodiments, a spacer moiety, X, may be any of the following: "-" (*i.e.*, a covalent bond, that may be stable or degradable, between the residue of the opioid agonist and the water-soluble oligomer), -O-, -NH-, -S-, -C(O)-, -C(O)O-, -OC(O)-, -$CH_2$-C(O)O-, -$CH_2$-OC(O)-, -C(O)O-$CH_2$-, -OC(O)-$CH_2$-, C(O)-NH, NH-C(O)-NH, O-C(O)-NH, -C(S)-, -$CH_2$-, -$CH_2$-$CH_2$-, -$CH_2$-$CH_2$-$CH_2$-, -$CH_2$-$CH_2$-$CH_2$-$CH_2$-, -O-$CH_2$-, -$CH_2$-O-, -O-$CH_2$-$CH_2$-, -$CH_2$-O-$CH_2$-, -$CH_2$-$CH_2$-O-, -O-$CH_2$-$CH_2$-$CH_2$-, -$CH_2$-O-$CH_2$-$CH_2$-, -$CH_2$-$CH_2$-O-$CH_2$-, -$CH_2$-$CH_2$-$CH_2$-O-, -O-$CH_2$-$CH_2$-$CH_2$-$CH_2$-, -$CH_2$-O-$CH_2$-$CH_2$-$CH_2$-, -$CH_2$-$CH_2$-O-$CH_2$-$CH_2$-, -$CH_2$-$CH_2$-$CH_2$-O-$CH_2$-, -$CH_2$-$CH_2$-$CH_2$-$CH_2$-O-, -C(O)-NH-$CH_2$-, -C(O)-NH-$CH_2$-$CH_2$-, -$CH_2$-C(O)-NH-$CH_2$-, -$CH_2$-$CH_2$-C(O)-NH-, -C(O)-NH-$CH_2$-$CH_2$-$CH_2$-, -$CH_2$-C(O)-NH-$CH_2$-$CH_2$-, -$CH_2$-$CH_2$-C(O)-NH-$CH_2$-, -$CH_2$-$CH_2$-$CH_2$-C(O)-NH-, -C(O)-NH-$CH_2$-$CH_2$-$CH_2$-$CH_2$-, -$CH_2$-C(O)-NH-$CH_2$-$CH_2$-$CH_2$-, -$CH_2$-$CH_2$-C(O)-NH-$CH_2$-$CH_2$-, -$CH_2$-$CH_2$-$CH_2$-C(O)-NH-$CH_2$-, -$CH_2$-$CH_2$-$CH_2$-C(O)-NH-$CH_2$-$CH_2$-, -$CH_2$-$CH_2$-$CH_2$-$CH_2$-C(O)-NH -, -NH-C(O)-$CH_2$-, -$CH_2$-NH-C(O)-$CH_2$-, -$CH_2$-$CH_2$-NH-C(O)-$CH_2$-, -NH-C(O)-$CH_2$-$CH_2$-, -$CH_2$-NH-C(O)-$CH_2$-$CH_2$, -$CH_2$-$CH_2$-NH-C(O)-$CH_2$-$CH_2$, -C(O)-NH-$CH_2$-, -C(O)-NH-$CH_2$-$CH_2$-, -O-C(O)-NH-$CH_2$-, -O-C(O)-NH-$CH_2$-$CH_2$-, -NH-$CH_2$-, -NH-$CH_2$-$CH_2$-, -$CH_2$-NH-$CH_2$-, -$CH_2$-$CH_2$-NH-$CH_2$-, - C(O)-$CH_2$-, -C(O)-$CH_2$-$CH_2$-, -$CH_2$-C(O)-$CH_2$-, -$CH_2$-$CH_2$-C(O)-$CH_2$-, -$CH_2$-$CH_2$-C(O)-$CH_2$-$CH_2$-, -$CH_2$-$CH_2$-C(O)-, -$CH_2$-$CH_2$-$CH_2$-C(O)-NH-$CH_2$-$CH_2$-NH-, -$CH_2$-$CH_2$-$CH_2$-C(O)-NH-$CH_2$-$CH_2$-NH-C(O)-, -$CH_2$-$CH_2$-$CH_2$-C(O)-NH-$CH_2$-$CH_2$-NH-C(O)-$CH_2$-, bivalent cycloalkyl group, -N($R^6$)-, where $R^6$ is H or an organic radical selected from the group consisting of alkyl, substituted alkyl, alkenyl, substituted alkenyl, alkynyl, substituted alkynyl, aryl and substituted aryl.

For purposes of the present invention, however, a group of atoms is not considered a spacer moiety when it is immediately adjacent to an oligomer segment, and the group of atoms is the same as a monomer of the oligomer such that the group would represent a mere extension of the oligomer chain.

<u>Conjugation</u>

The linkage "X" between the water-soluble oligomer and the opioid compound is typically formed by reaction of a functional group on a terminus of the oligomer (or one or more monomers when it is desired to "grow" the oligomer onto the opioid agonist) with a corresponding functional group within the opioid agonist. For example, an amino group on an oligomer may be reacted with a carboxylic acid or an activated carboxylic acid derivative on the opioid compound,

or vice versa, to produce an amide linkage. Alternatively, reaction of an amine on an oligomer with an activated carbonate (*e.g.* succinimidyl or benzotriazyl carbonate) on the opioid compound, or vice versa, forms a carbamate linkage. Reaction of an amine on an oligomer with an isocyanate (R-N=C=O) on an opioid compound, or vice versa, forms a urea linkage (R-NH-(C=O)-NH-R'). Further, reaction of an alcohol (alkoxide) group on an oligomer with an alkyl halide, or halide group within an opioid compound, or vice versa, forms an ether linkage. In yet another coupling approach, an opioid compound having an aldehyde function is coupled to an oligomer amino group by reductive amination, resulting in formation of a secondary amine linkage between the oligomer and the opioid compound.

A particularly preferred water-soluble oligomer is an oligomer bearing an aldehyde functional group. In this regard, the oligomer will have the following structure: $CH_3O-(CH_2-CH_2-O)_n-(CH_2)_p-C(O)H$, wherein (n) is one of 1, 2, 3, 4, 5, 6, 7, 8, 9 and 10 and (p) is one of 1, 2, 3, 4, 5, 6 and 7. Preferred (n) values include 1, 2, 3, 4, 7, 8, 9, and 10 and preferred (p) values 2, 3 and 4. In addition, the carbon atom alpha to the -C(O)H moiety can optionally be substituted with alkyl.

Typically, the terminus of the water-soluble oligomer not bearing a functional group is capped to render it unreactive. When the oligomer does include a further functional group at a terminus other than that intended for formation of a conjugate, that group is either selected such that it is unreactive under the conditions of formation of the linkage "X," or it is protected during the formation of the linkage "X."

As stated above, the water-soluble oligomer includes at least one functional group prior to conjugation. The functional group typically comprises an electrophilic or nucleophilic group for covalent attachment to an opioid compound, depending upon the reactive group contained within or introduced into the opioid compound. Examples of nucleophilic groups that may be present in either the oligomer or the opioid compound include hydroxyl, amine, hydrazine ($-NHNH_2$), hydrazide ($-C(O)NHNH_2$), and thiol. Preferred nucleophiles include amine, hydrazine, hydrazide, and thiol, particularly amine. Most opioid compounds for covalent attachment to an oligomer will possess a free hydroxyl, amino, thio, aldehyde, ketone, or carboxyl group.

Examples of electrophilic functional groups that may be present in either the oligomer or the opioid compound include carboxylic acid, carboxylic ester, particularly imide esters, orthoester, carbonate, isocyanate, isothiocyanate, aldehyde, ketone, thione, alkenyl, acrylate, methacrylate, acrylamide, sulfone, maleimide, disulfide, iodo, epoxy, sulfonate, thiosulfonate, silane, alkoxysilane, and halosilane. More specific examples of these groups include succinimidyl ester or carbonate, imidazoyl ester or carbonate, benzotriazole ester or carbonate, vinyl sulfone, chloroethylsulfone, vinylpyridine, pyridyl disulfide, iodoacetamide, glyoxal, dione, mesylate, tosylate, and tresylate (2,2,2-trifluoroethanesulfonate).

Also included are sulfur analogs of several of these groups, such as thione, thione hydrate, thioketal, is 2-thiazolidine thione, etc., as well as hydrates or protected derivatives of any of the above moieties (*e.g.* aldehyde hydrate, hemiacetal, acetal, ketone hydrate, hemiketal, ketal, thioketal, thioacetal).

An "activated derivative" of a carboxylic acid refers to a carboxylic acid derivative which reacts readily with nucleophiles, generally much more readily than the underivatized carboxylic acid. Activated carboxylic acids include, for example, acid halides (such as acid chlorides), anhydrides, carbonates, and esters. Such esters include imide esters, of the general form $-(CO)O-N[(CO)-]_2$; for example, N-hydroxysuccinimidyl (NHS) esters or N-hydroxyphthalimidyl esters. Also preferred are imidazolyl esters and benzotriazole esters. Particularly preferred are activated propionic acid or butanoic acid esters, as described in co-owned U.S. Patent No. 5,672,662. These include groups of the form $-(CH_2)_{2-3}C(=O)O-Q$, where Q is preferably selected from N-succinimide, N-sulfosuccinimide, N-phthalimide, N-glutarimide, N-tetrahydrophthalimide, N-norbornene-2,3-dicarboximide, benzotriazole, 7-azabenzotriazole, and imidazole.

Other preferred electrophilic groups include succinimidyl carbonate, maleimide, benzotriazole carbonate, glycidyl ether, imidazoyl carbonate, p-nitrophenyl carbonate, acrylate, tresylate, aldehyde, and orthopyridyl disulfide.

These electrophilic groups are subject to reaction with nucleophiles, e.g. hydroxy, thio, or amino groups, to produce various bond types. Several of the electrophilic functional groups include electrophilic double bonds to which nucleophilic groups, such as thiols, can be added, to form, for example, thioether bonds. These groups include maleimides, vinyl sulfones, vinyl pyridine, acrylates, methacrylates, and acrylamides. Other groups comprise leaving groups that can be displaced by a nucleophile; these include chloroethyl sulfone, pyridyl disulfides (which include a cleavable S-S bond), iodoacetamide, mesylate, tosylate, thiosulfonate, and tresylate. Epoxides react by ring opening by a nucleophile, to form, for example, an ether or amine bond. Reactions involving complementary reactive groups such as those noted above on the oligomer and the opioid compound are utilized to prepare the conjugates of the invention.

In certain embodiments of the invention, reactions which favor formation of a hydrolytically stable linkage are preferred. For example, carboxylic acids and activated derivatives thereof, which include orthoesters, succinimidyl esters, imidazolyl esters, and benzotriazole esters, react with the above types of nucleophiles to form esters, thioesters, and amides, respectively, of which amides are the most hydrolytically stable. Carbonates, including succinimidyl, imidazolyl, and benzotriazole carbonates, react with amino groups to form carbamates. Isocyanates (R-N=C=O) react with hydroxyl or amino groups to form, respectively, carbamate (RNH-C(O)-OR') or urea (RNH-C(O)-NHR') linkages. Aldehydes, ketones, glyoxals, diones and their hydrates or alcohol adducts (*i.e.* aldehyde hydrate, hemiacetal, acetal, ketone hydrate,

hemiketal, and ketal) are preferably reacted with amines, followed by reduction of the resulting imine, if desired, to provide an amine linkage (reductive amination).

In other embodiments of the invention, reactions which favor formation of a physiologically cleavable linkage are preferred. The releasable linkages may, but do not necessarily, result in the water-soluble oligomer (and any spacer moiety) detaching from the opioid compound *in vivo* (and in some cases *in vitro*) without leaving any fragment of the water-soluble oligomer (and/or any spacer moiety or linker) attached to the opioid compound. Exemplary releasable linkages include carbonate, carboxylate ester, phosphate ester, thiolester, anhydrides, acetals, ketals, acyloxyalkyl ether, imines, certain carbamates, and orthoesters. Such linkages can be readily formed by reaction of the opioid compound and/or the polymeric reagent using coupling methods commonly employed in the art. Hydrolyzable linkages are often readily formed by reaction of a suitably activated oligomer with a non-modified functional group contained within the opioid compound.

In some instances the opioid agonist may not have a functional group suited for conjugation. In this instance, it is possible to modify the "original" opioid agonist so that it does have the desired functional group. For example, if the opioid agonist has an amide group, but an amine group is desired, it is possible to modify the amide group to an amine group by way of a Hofmann rearrangement, Curtius rearrangement (once the amide is converted to an azide) or Lossen rearrangement (once amide is concerted to hydroxamide followed by treatment with tolyene-2-sulfonyl chloride/base).

It is possible to prepare a conjugate of an opioid agonist bearing a carboxyl group wherein the carboxyl group-bearing opioid agonist is coupled to an amino-terminated oligomeric ethylene glycol, to provide a conjugate having an amide group covalently linking the opioid agonist to the oligomer. This can be performed, for example, by combining the carboxyl group-bearing opioid agonist with the amino-terminated oligomeric ethylene glycol in the presence of a coupling reagent, (such as dicyclohexylcarbodiimide or "DCC") in an anhydrous organic solvent.

Further, it is possible to prepare a conjugate of an opioid agonist bearing a hydroxyl group wherein the hydroxyl group-bearing opioid agonist is coupled to an oligomeric ethylene glycol halide to result in an ether (-O-) linked opioid compound conjugate. This can be performed, for example, by using sodium hydride to deprotonate the hydroxyl group followed by reaction with a halide-terminated oligomeric ethylene glycol.

In another example, it is possible to prepare a conjugate of an opioid agonist bearing a ketone group by first reducing the ketone group to form the corresponding hydroxyl group. Thereafter, the opioid agonist now bearing a hydroxyl group can be coupled as described herein.

In still another instance, it is possible to prepare a conjugate of an opioid agonist bearing an amine group. In one approach, the amine group-bearing opioid agonist and an aldehyde-bearing oligomer are dissolved in a suitable buffer after which a suitable reducing agent (*e.g.*, $NaCNBH_3$) is added. Following reduction, the result is an amine linkage formed between the amine group of the amine group-containing opioid agonist and the carbonyl carbon of the aldehyde-bearing oligomer.

In another approach for preparing a conjugate of an opioid agonist bearing an amine group, a carboxylic acid-bearing oligomer and the amine group-bearing opioid agonist are combined, typically in the presence of a coupling reagent (*e.g.,* DCC). The result is an amide linkage formed between the amine group of the amine group-containing opioid agonist and the carbonyl of the carboxylic acid-bearing oligomer.

## Blood Brain Barrier Crossing

In certain embodiments of the invention, X is preferably a stable linker. In accordance with the invention, it has been found that certain opioid compounds bound to small water-soluble oligomers via a stable linkage, while retaining the ability to cross the blood-brain barrier, do so at a reduced BBB crossing rate relative to the unconjugated opioid compound. Without wishing to be bound by any particular theory, it is believed that the reduced BBB membrane crossing rate is a direct function of changes in the intrinsic BBB permeability properties of the molecule relative to the unconjugated opioid compound. Again not wishing to be bound by any particular theory, it is presumed that such opioid conjugates possess low addictive properties due to a slow crossing of the BBB, avoiding the rapid peak concentrations associated with unconjugated opioid agonists and underlying addictive highs. Additionally, the compounds of the present invention may exhibit an improved side effect profile relative to the unconjugated opioid due to an altered tissue distribution of the opioid *in vivo* or decreased activity at peripheral opioid receptors.

Thus, in accordance with these embodiments of the invention, any combination of opioid compound, linker, and water-soluble oligomer may be used, provided that the conjugate is able to cross the BBB. Preferably, the conjugate crosses the BBB at a reduced rate relative to the unconjugated opioid agonist. In a preferred embodiment, the water-soluble oligomer is a PEG moiety. Typically, the PEG moiety is a small monomeric PEG consisting of 1-3 (*i.e.* 1, 2, or 3) polyethylene glycol units. In other embodiments the PEG moiety may be 4 or 5 polyethylene glycol units.

With respect to the blood-brain barrier ("BBB"), this barrier restricts the transport of drugs from the blood to the brain. This barrier consists of a continuous layer of unique endothelial cells joined by tight junctions. The cerebral capillaries, which comprise more than 95% of the total surface area of the BBB, represent the principal route for the

entry of most solutes and drugs into the central nervous system.

As will be understood by one of skill in the art, molecular size, lipophilicity, and PgP interaction are among the primary parameters affecting the intrinsic BBB permeability properties of a given molecule. That is to say, these factors, when taken in combination, control whether a given molecule passes through the BBB, and if so, at what rate.

Due to the small pore size within the BBB, molecular size plays a significant role in determining whether a given molecule will pass through the BBB. Very large molecules, for example a molecule having a molecular weight of 5,000 Daltons, will not cross the BBB, whereas small molecules are more likely to permeate the BBB. Other factors, however, also play a role in BBB crossing. Antipyrine and atenolol are both small molecule drugs; antipyrine readily crosses the BBB, whereas passage of atenolol is very limited, or effectively non-existent. Antipyrine is an industry standard for a high BBB permeation; atenolol is an industry standard for low permeation of the BBB. *See, e.g.,* Summerfield et al., J Pharmacol Exp Ther 322:205-213 (2007). Therefore, in accordance with the invention, where X is a stable linker, opioid conjugates having 1-3 polyethylene glycol units can generally be expected to cross the BBB. In certain circumstances, where the intrinsic BBB permeability properties as a whole are suitable, particular opioid conjugates having 4 or 5 polyethylene glycol units may also cross the BBB.

Lipophilicity is also a factor in BBB permeation. Lipophilicity may be expressed as logP (partition coefficient) or in some instances logD (distribution coefficient). The logP (or logD) for a given molecule can be readily assessed by one of skill in the art. The value for logP may be a negative number (more hydrophilic molecules) or a positive number (more hydrophobic molecules). As used herein when referring to logP, "more negative" means moving in the direction, on the logP scale, from positive to negative logP (*e.g.,* a logP of 2.0 is "more negative" than a logP of 4.0, a logP of -2.0 is "more negative" than a logP of -1.0). Molecules having a negative logP (hydrophilic molecules) generally do not permeate the BBB. In certain embodiments, the opioid conjugates of the invention have a logP between about 0 and about 4.0. Preferably, the opioid conjugates of the invention have a logP between about 1.0 and about 3.5. In certain embodiments, the conjugates of the invention have a logP of about 4.0, of about 3.5, of about 3.0, of about 2.5, of about 2.0, of about 1.5, of about 1.0, of about 0.5, or of about 0, or they may have a logP in the range of about 0 to about 3.5, of about 0 to about 3.0, of about 0 to about 2.0, of about 0 to about 1.0, of about 1.0 to about 4.0, of about 1.0 to about 3.0, of about 1.0 to about 2.0, of about 2.0 to about 4.0, of about 2.0 to about 3.5, of about 2.0 to about 3.0, of about 3.0 to about 4.0, or of about 3.0 to about 3.5.

Permeability across the BBB is also dependent on P-glycoprotein, or PgP, an ATP-dependent efflux transporter highly expressed at the BBB. One of skill in the art can readily determine whether a compound is a substrate for PgP using *in vitro* methods. Compounds which are substrates for PgP *in vitro* likely will not permeate the BBB *in vivo.* Conversely, poor substrates for PgP, as assessed *in vitro,* are generally likely to display *in vivo* permeability of the BBB, provided the compound meets other criteria as discussed herein and as known to one of skill in the art. *See, e.g.,* Tsuji, NeuroRx 2:54-62 (2005) and Rubin and Staddon, Annu. Rev. Neurosci. 22:11-28 (1999).

In certain embodiments, the water-soluble oligomer may be selected in accordance with the desired pharmacokinetic profile of the opioid conjugate. In other words, conjugation of the opioid compound to a water-soluble oligomer will result in a net reduction in BBB membrane crossing rate, however the reduction in rate may vary depending on the size of the oligomer used. Generally, where a minimal reduction in BBB crossing rate is desired, a smaller oligomer may be used; where a more extensive reduction in BBB crossing rate is desired, a larger oligomer may be used. In certain embodiments, a combination of two or more different opioid conjugates may be administered simultaneously, wherein each conjugate has a differently sized water-soluble oligomer portion, and wherein the rate of BBB crossing for each conjugate is different due to the different oligomer sizes. In this manner, the rate and duration of BBB crossing of the opioid compound can be specifically controlled through the simultaneous administration of multiple conjugates with varying pharmacokinetic profiles.

For compounds whose degree of blood-brain barrier crossing ability is not readily known, such ability can be determined using a suitable animal model such as an *in situ* rat brain perfusion ("RBP") model. Briefly, the RBP technique involves cannulation of the carotid artery followed by perfusion with a compound solution under controlled conditions, followed by a wash out phase to remove compound remaining in the vascular space. (Such analyses can be conducted, for example, by contract research organizations such as Absorption Systems, Exton, PA). More specifically, in the RBP model, a cannula is placed in the left carotid artery and the side branches are tied off. A physiologic buffer containing the analyte (typically but not necessarily at a 5 micromolar concentration level) is perfused at a flow rate of about 10 mL/minute in a single pass perfusion experiment. After 30 seconds, the perfusion is stopped and the brain vascular contents are washed out with compound-free buffer for an additional 30 seconds. The brain tissue is then removed and analyzed for compound concentrations via liquid chromatograph with tandem mass spectrometry detection (LC/MS/MS). Alternatively, blood-brain barrier permeability can be estimated based upon a calculation of the compound's molecular polar surface area ("PSA"), which is defined as the sum of surface contributions of polar atoms (usually oxygens, nitrogens and attached hydrogens) in a molecule. The PSA has been shown to correlate with compound transport properties such as blood-brain barrier transport. Methods for determining a compound's PSA can be found, *e.g.,* in, Ertl, P., et al., J. Med. Chem. 2000, 43, 3714-3717; and Kelder, J., et al., Pharm. Res. 1999,16, 1514-1519.

In those embodiments where X is a stable linker, the molecular weight of the opioid conjugate is preferably less than 2000 Daltons, and more preferably less than 1000 Daltons. In other embodiments, the molecular weight of the conjugate is less than 950 Daltons, less than 900 Daltons, less than 850 Daltons, less than 800 Daltons, less than 750 Daltons, less than 700 Daltons, less than 650 Daltons, less than 600 Daltons, less than 550 Daltons, less than 500 Daltons, less than 450 Daltons, or less than 400 Daltons.

In certain embodiments where X is a stable linker, the molecular weight of X-POLY (*i.e.* the water soluble oligomer in combination with the linker, where present) is preferably less than 2000 Daltons. In some embodiments, the molecular weight of the opioid conjugate is preferably less than 1000 Daltons. In other embodiments, the molecular weight of the conjugate is less than 950 Daltons, less than 900 Daltons, less than 850 Daltons, less than 800 Daltons, less than 750 Daltons, less than 700 Daltons, less than 650 Daltons, less than 600 Daltons, less than 550 Daltons, less than 500 Daltons, less than 450 Daltons, less than 400 Daltons, less than 350 Daltons, less than 300 Daltons, less than 250 Daltons, less than 200 Daltons, less than 150 Daltons, less than 100 Daltons, or less than 50 Daltons.

Where X is a stable linker, the conjugate (*i.e.* OP-X-POLY) is preferably less hydrophobic than the unconjugated opioid compound (*i.e.* OP), In other words, the logP of the conjugate is preferably more negative than the logP of the unconjugated opioid compound. In one embodiment, the logP of the conjugate is about 0.5 units more negative than that of the unconjugated opioid compound. In other embodiments, the log P of the conjugate is about 4.0 units more negative, about 3.5 units more negative, about 3.0 units more negative, about 2.5 units more negative, about 2.0 units more negative, about 1.5 units more negative, about 1.0 units more negative, about 0.9 units more negative, about 0.8 units more negative, about 0.7 units more negative, about 0.6 units more negative, about 0.4 units more negative, about 0.3 units more negative, about 0.2 units more negative or about 0.1 units more negative than the unconjugated opioid compound. In certain embodiments, the logP of the conjugate is about 0.1 units to about 4.0 units more negative, about 0.1 units to about 3.5 units more negative, about 0.1 units to about 3.0 units more negative, about 0.1 units to about 2.5 units more negative, about 0.1 units to about 2.0 units more negative, about 0.1 units to about 1.5 units more negative, about 0.1 units to about 1.0 units more negative, about 0.1 units to about 0.5 units more negative, about 0.5 units to about 4.0 units more negative, about 0.5 units to about 3.5 units more negative, about 0.5 units to about 3.0 units more negative, about 0.5 units to about 2.5 units more negative, about 0.5 units to about 2.0 units more negative, about 0.5 units to about 1.5 units more negative, about 0.5 units to about 1.0 units more negative, about 1.0 units to about 4.0 units more negative, about 1.0 units to about 3.5 units more negative, about 1.0 units to about 3.0 units more negative, about 1.0 units to about 2.5 units more negative, about 1.0 units to about 2.0 units more negative, about 1.0 units to about 1.5 units more negative, about 1.5 units to about 4.0 units more negative, about 1.5 units to about 3.5 units more negative, about 1.5 units to about 3.0 units more negative, about 1.5 units to about 2.5 units more negative, about 1.5 units to about 2.0 units more negative, about 2.0 units to about 4.0 units more negative, about 2.0 units to about 3.5 units more negative, about 2.0 units to about 3.0 units more negative, about 2.0 units to about 2.5 units more negative. about 2.5 units to about 4.0 units more negative, about 2.5 units to about 3.5 units more negative, about 2.5 units to about 3.0 units more negative, about 3.0 units to about 4.0 units more negative, about 3.0 units to about 3.5 units more negative, or about 3.5 units to about 4.0 units more negative than the unconjugated opioid compound. In some particular embodiments, the logP of the conjugate is the same as, or is more positive than, the logP of the unconjugated opioid compound.

Receptor Binding and Other Features

Where X is a stable linker, the conjugate of the invention preferably retains a suitable affinity for its target receptor(s), and by extension a suitable concentration and potency within the brain. Preferably the water-soluble oligomer is conjugated to the opioid in a manner such that the conjugated opioid binds, at least in part, to the same receptor(s) to which the unconjugated opioid compound binds. To determine whether the opioid agonist or the conjugate of an opioid agonist and a water-soluble oligomer has activity as mu, kappa, or delta opioid receptor agonist, for example, it is possible to test such a compound. For example, a radioligand binding assay in CHO cells that heterologously express the recombinant human mu, kappa, or delta opioid receptor can be used. Briefly, cells are plated in 24 well plates and washed with assay buffer. Competition binding assays are conducted on adherent whole cells incubated with increasing concentrations of opioid conjugates in the presence of an appropriate concentration of radioligand. [$^3$H]naloxone, [$^3$H]diprenorphine and [$^3$H]DPDPE are used as the competing radioligands for mu, kappa and delta receptors respectively. Following incubation, cells are washed, solubilized with NaOH and bound radioactivity is measured using a scintillation counter.

In certain embodiments, the Ki values of the conjugates of the invention fall within the range of 0.1 to 900 nM, preferably within the range of 0.1 and 300 nM, and more preferably within the range of 0.1 and 50 nM. In a preferred embodiment where X is a stable linker, there is no loss of affinity of the conjugated opioid compound (*i.e.* the OP of OP-X-POLY) relative to the affinity of OP to its target receptor(s), and in some embodiments the affinity of the conjugated opioid compound may be greater than the affinity of OP to its target receptor(s). In a preferred embodiment where X is

a stable linker, the affinity of the conjugated opioid compound (*i.e.* the OP of OP-X-POLY) is reduced minimally relative to the affinity of OP to its target receptor(s), and in some cases may even show an increase in affinity or no change in affinity. Preferably, there is less than about a 2-fold loss of affinity of the conjugated opioid compound relative to the affinity of the unconjugated opioid compound for its target receptor(s). In certain embodiments, there is preferably less than about a 5-fold loss, less than about a 10-fold loss, less than about a 20-fold loss, less than about a 30-fold loss, less than about a 40-fold loss, less than about a 50-fold loss, less than about a 60-fold loss, less than about a 70-fold loss, less than about an 80-fold loss, less than about a 90-fold loss, or less than about a 100-fold loss of affinity of the conjugated opioid compound relative to the affinity of the unconjugated opioid compound for its target receptor(s)

In certain other embodiments where X is a stable linker, preferably the reduction in affinity of the conjugated opioid compound relative to the affinity of the unconjugated opioid compound for its target receptor(s) is less than 20%. In some embodiments, the reduction in affinity of the conjugated opioid compound relative to the unconjugated opioid compound is less than 10%, less than 30%, less than 40%, less than 50%, less than 60%, less than 70%, less than 80%, less than 90%, or less than 95%.

In certain embodiments where X is a stable linker, the rate of crossing the BBB, or the permeability of the conjugate is less than the rate of crossing of OP alone. Preferably, the rate of crossing is at least about 50% less than the rate of OP alone. In certain other embodiments, there is at least about a 10% reduction, at least about a 15% reduction, at least about a 20% reduction, at least about a 25% reduction, at least about a 30% reduction, at least about a 35% reduction, at least about a 40% reduction, at least about a 45% reduction, at least about a 55% reduction, at least about a 60% reduction, at least about a 65% reduction, at least about a 70% reduction, at least about a 75% reduction, at least about an 80% reduction, at least about an 85% reduction, at least about a 90% reduction at least about a 95% reduction, or at least about a 99% reduction in the BBB crossing rate of the conjugate relative to the rate of crossing of OP alone. In other embodiments, the conjugates of the invention may exhibit a 10-99% reduction, a 10-50% reduction, a 50-99% reduction, a 50-60% reduction, a 60-70% reduction, a 70-80% reduction, an 80-90% reduction, or a 90-99% reduction in the BBB crossing rate of the conjugate relative to the rate of crossing of OP alone.

The conjugates of the invention, where X is a stable linker, may exhibit a 1 to 100 fold reduction in the BBB crossing rate relative to the rate of crossing of the OP alone. In certain embodiments, there may be at least about a 2-fold loss, at least about a 5-fold loss, at least about a 10-fold loss, at least about a 20-fold loss, at least about a 30-fold loss, at least about a 40-fold loss, at least about a 50-fold loss, at least about a 60-fold loss, at least about a 70-fold loss, at least about an 80-fold loss, at least about a 90-fold loss, or at least about a 100-fold loss in the BBB crossing rate of the conjugated opioid compound relative to the BBB crossing rate of the unconjugated opioid compound.

The rate of BBB crossing of the conjugates of the invention, where X is a stable linker, may also be viewed relative to the BBB crossing rate of antipyrine (high permeation standard) and/or atenolol (low permeation standard). It will be understood by one of skill in the art that implied in any reference to BBB crossing rates of the conjugates of the invention relative to the BBB crossing rate of antipyrine and/or atenolol is that the rates were evaluated in the same assay, under the same conditions. Thus, in certain embodiments the conjugates of the invention may exhibit at least about a 2-fold lower, at least about a 5-fold lower, at least about a 10-fold lower, at least about a 20-fold lower, at least about a 30-fold lower, at least about a 40-fold lower, at least about a 50-fold lower, at least about a 60-fold lower, at least about a 70-fold lower, at least about an 80-fold lower, at least about a 90-fold lower, or at least about a 100-fold lower rate of BBB crossing rate relative to the BBB crossing rate of antipyrine. In other embodiments, the conjugates of the invention, the conjugates of the invention may exhibit at least about a 2-fold greater, at least about a 5-fold greater, at least about a 10-fold greater, at least about a 20-fold greater, at least about a 30-fold greater, at least about a 40-fold greater, at least about a 50-fold greater, at least about a 60-fold greater, at least about a 70-fold greater, at least about an 80-fold greater, at least about a 90-fold greater, or at least about a 100-fold greater rate of BBB crossing rate relative to the BBB crossing rate of atenolol.

Where X is a stable linker, the conjugate (*i.e.* OP-X-POLY) may retain all or some of the opioid agonist bioactivity relative to the unconjugated opioid compound (*i.e.* OP). Preferably, the conjugate retains all the opioid agonist bioactivity relative to the unconjugated opioid compounds, or in some circumstances, is even more active than the unconjugated opioid compounds. Preferably, the conjugates of the invention exhibit less than about a 2-fold decrease, less than about a 5-fold decrease, less than about a 10-fold decrease, less than about a 20-fold decrease, less than about a 30-fold decrease, less than about a 40-fold decrease, less than about a 50-fold decrease, less than about a 60-fold decrease, less than about a 70-fold decrease, less than about an 80-fold decrease, less than about a 90-fold decrease, or less than about a 100-fold decrease in bioactivity relative to the unconjugated opioid compounds. In some embodiments, the conjugated opioid compound retains at least 1%, at least 2%, at least 3%, at least 4%, at least 5%, at least 6%, at least 7%, at least 8%, at least 9%, at least 10%, at least 20%, at least 30%, at least 40%, at least 50%, at least 60%, at least 70%, at least 75%, at least 80%, at least 85%, at least 90%, or at least 95% of the opioid agonist bioactivity relative to the unconjugated opioid compound.

It will be understood by one of skill in the art that the values recited herein are exemplary and non-limiting, and that certain conjugates of an opioid agonist and a water-soluble oligomer may fall outside the ranges recited herein yet

remain within the spirit and scope of the invention. Conjugates may be prepared and tested as a matter of routine experimentation for one of skill in the art. In particular, opioid agonists, bound to a water-soluble oligomer via a stable linkage, may be tested for penetration of the blood brain barrier as described above. Thus one of skill in the art can readily ascertain whether a conjugate is able to cross the BBB.

While it is believed that the full scope of the conjugates of these embodiments of the invention has been described, an optimally sized oligomer can be determined as follows.

First, an oligomer obtained from a monodisperse or bimodal water-soluble oligomer is conjugated to the opioid agonist through a stable linkage. Next, *in vitro* retention of activity is analyzed. The ability of the conjugate to cross the blood-brain barrier is then determined using an appropriate model and compared to that of the unmodified parent opioid compound. If the results are favorable, that is to say, if, for example, the rate of crossing is reduced to an appropriate degree, then the bioactivity of conjugate is further evaluated. Preferably, the compounds according to the invention maintain a significant degree of bioactivity relative to the parent opioid compound, *i.e.,* greater than about 30% of the bioactivity of the parent opioid compound, or even more preferably, greater than about 50% of the bioactivity of the parent opioid compound. Preferably, the opioid agonist is orally bioavailable.

The above steps are repeated one or more times using oligomers of the same monomer type but having a different number of subunits and the results are compared.

For each conjugate whose ability to cross the blood-brain barrier is appropriately reduced in comparison to the non-conjugated opioid agonist, its oral bioavailability is then assessed. Based upon these results, that is to say, based upon the comparison of conjugates of oligomers of varying size to a given opioid agonist at a given position or location within the opioid agonist, it is possible to determine the size of the oligomer most effective in providing a conjugate having an optimal balance between appropriate reduction in biological membrane crossing, oral bioavailability, and bioactivity. The small size of the oligomers makes such screenings feasible, and allows one to effectively tailor the properties of the resulting conjugate. By making small, incremental changes in oligomer size, and utilizing an experimental design approach, one can effectively identify a conjugate having a favorable balance of reduction in biological membrane crossing rate, bioactivity, and oral bioavailability. In some instances, attachment of an oligomer as described herein is effective to actually increase oral bioavailability of the opioid agonist.

For example, one of ordinary skill in the art, using routine experimentation, can determine a best suited molecular size and linkage for improving oral bioavailability by first preparing a series of oligomers with different weights and functional groups and then obtaining the necessary clearance profiles by administering the conjugates to a patient and taking periodic blood and/or urine sampling. Once a series of clearance profiles have been obtained for each tested conjugate, a suitable conjugate can be identified.

Animal models (rodents and dogs) can also be used to study oral drug transport. In addition, non-*in vivo* methods include rodent everted gut excised tissue and Caco-2 cell monolayer tissue-culture models. These models are useful in predicting oral drug bioavailability.

In certain other embodiments of the invention, X is preferably a physiologically cleavable linker. In accordance with the invention, it has been found that certain opioid compounds bound to small water-soluble oligomers via a cleavable linkage are unable to cross the BBB in their conjugated form, and therefore exhibit a net reduced BBB membrane crossing rate due to slow physiological cleavage of the opioid compound from the water-soluble oligomer. In particular, X may be selected in accordance with the desired pharmacokinetic profile of the unconjugated opioid compound. In other words, conjugation of the opioid compound to a water-soluble oligomer will result in a net reduction in BBB membrane crossing rate, however the reduction in rate may vary depending on the linker used. Where a minimal reduction in BBB crossing rate is desired, X may be a rapidly degraded linker; where an extensive reduction in BBB crossing rate is desired, X may be a more slowly degraded linker. In certain embodiments, a combination of two or more different opioid conjugates may be administered simultaneously, wherein each conjugate has a different linker X, and wherein the rate of degradation of each X is different. In other words, for each different conjugate, the opioid compound will be cleaved from the water-soluble oligomer at a different rate, resulting in different net BBB membrane crossing rates. A similar effect may be achieved through the use of multifunctional water-soluble oligomers having two or more sites of opioid attachment, with each opioid linked to the water-soluble oligomer through linkers having varying rates of degradation. In this manner, the rate and duration of BBB crossing of the opioid compound can be specifically controlled through the simultaneous administration of multiple conjugates with varying pharmacokinetic profiles.

Not wishing to be bound by any particular theory, it is presumed that such opioid conjugates possess low addictive properties due to the net slow crossing of the BBB (due to slow physiological cleavage following administration of the conjugate), avoiding the rapid peak concentrations associated with unconjugated opioid agonists and underlying addictive highs. Again, not wishing to be bound by any particular theory, it is believed that the opioid conjugates of the invention circulate in the plasma, and are cleaved *in vivo* at a rate dependant upon the specific cleavable linker used (and, for enzymatically degradable linkers, enzyme concentration and affinity), such that the concentration of unconjugated opioid circulating in the periphery is generally very low due to the slow rate of cleavage. Once cleavage has occurred, the unconjugated opioid may travel to the brain to cross the BBB; the slow release of the unconjugated opioid

through cleavage results in a net slow delivery of the unconjugated opioid to the brain. Additionally, the compounds of the present invention exhibit an improved side effect profile relative to the unconjugated opioid dues to an altered tissue distribution of the opioid *in vivo* and altered receptor interaction at the periphery.

Moreover, in accordance with these embodiments of the invention, any combination of opioid compound, linker, and water-soluble oligomer may be used, provided that the conjugate is not able to cross the BBB or only a small fraction of the conjugate, preferably less than 5% of that administered, is able to cross the BBB. Preferably, the conjugate is not able to cross the BBB. More preferably, the opioid portion of the molecule, due to physiological cleavage of the conjugate, crosses the BBB at a net reduced rate relative to the unconjugated opioid agonist. In a preferred embodiment, the water-soluble oligomer is a PEG moiety. In certain of these embodiments, the PEG moiety is a small monomeric PEG consisting of at least 6 polyethylene glycol units, preferably 6-35 polyethylene glycol units. In some instances, the PEG moiety may be 6-1825 polyethylene glycol units.

Where X is a physiologically cleavable linker, the conjugate (*i.e.* OP-X-POLY) may or may not be bioactive. Preferably, the conjugate is not bioactive. Such a conjugate is nevertheless effective when administered *in vivo* to a mammalian subject in need thereof, due to release of the opioid compound from the conjugate subsequent to administration. Preferably, the conjugates of the invention exhibit greater than about a 10-fold decrease, greater than about a 20-fold decrease, greater than about a 30-fold decrease, greater than about a 40-fold decrease, greater than about a 50-fold decrease, greater than about a 60-fold decrease, greater than about a 70-fold decrease, greater than about an 80-fold decrease, greater than about a 90-fold decrease, greater than about a 95-fold decrease, greater than about a 97-fold decrease, or greater than about a 100-fold decrease in bioactivity relative to the unconjugated opioid compounds. In some embodiments, the conjugated opioid compound retains less than 1%, less than 2%, less than 3%, less than 4%, less than 5%, less than 10%, less than 15%, less than 20%, less than 25%, less than 30%, less than 35%, less than 40%, less than 50%, less than 60%, less than 70%, less than 80% or less than 90% of the opioid agonist bioactivity relative to the unconjugated opioid compound.

In other embodiments where X is a physiologically cleavable linker, the affinity of OP-X-POLY for the OP target receptor is substantially reduced relative to the affinity of OP to its target receptor. Preferably, there is at least about a 2-fold loss of affinity of the conjugated opioid compound relative to the affinity of the unconjugated opioid compound for its target receptor(s). In certain embodiments, there is preferably at least about a 5-fold loss, at least about a 10-fold loss, at least about a 20-fold loss, at least about a 30-fold loss, at least about a 40-fold loss, at least about a 50-fold loss, at least about a 60-fold loss, at least about a 70-fold loss, at least about an 80-fold loss, at least about a 90-fold loss, or at least about a 100-fold loss of affinity of the conjugated opioid compound relative to the affinity of the unconjugated opioid compound for its target receptor(s).

In certain other embodiments where X is a physiologically cleavable linker, preferably the reduction in affinity of the conjugated opioid compound relative to the affinity of the unconjugated opioid compound for its target receptor(s) is at least 20%. In some embodiments, the reduction in affinity of the conjugated opioid compound relative to the unconjugated opioid compound is at least 10%, at least 30%, at least 40%, at least 50%, at least 60%, at least 70%, at least 80%, at least 90%, or at least 95%.

As previously noted, in certain embodiments where X is a physiologically cleavable linker, the conjugate is not bioactive. Such a conjugate represents a prodrug, where the compound as administered is inactive, and is made active subsequent to administration through physiological processes. Thus, in certain embodiments, the invention provides a prodrug comprising an opioid agonist reversibly attached via a covalent bond to a releasable moiety, wherein a given molar amount of the prodrug administered to a patient exhibits a rate of accumulation and a $C_{max}$ of the opioid agonist in the central nervous system in the mammal that is less than the rate of accumulation and the $C_{max}$ of an equal molar amount of the opioid agonist had the opioid agonist not been administered as part of a prodrug. The releasable moiety may be a water-soluble oligomer, preferably a polyethylene glycol oligomer. The agonist may be a mu, kappa, or delta opioid agonist.

In certain other embodiments of the invention, X is preferably a physiologically cleavable linker and POLY is a small monomeric PEG consisting of 1-5 (*i.e.* 1, 2, 3, 4, or 5) polyethylene glycol units, preferably 1-3 (*i.e.* 1, 2, or 3) polyethylene glycol units. Such compounds are small enough to cross the blood-brain barrier, but do so at a reduced membrane crossing rate relative to the unconjugated opioid compound, and as such possess low addictive properties as previously discussed. Preferably, X is selected to provide for cleavage of the linker and release of the opioid compound subsequent to crossing the BBB. Alternatively, cleavage of the linker may happen both prior to, and after, crossing the BBB; in this manner the rate and duration of BBB crossing of the opioid compound can be specifically controlled.

<u>Pharmaceutical Compositions</u>

In further embodiments, the invention provides for compositions comprising the OP-X-POLY compounds disclosed herein and a pharmaceutically acceptable excipient or carrier. Generally, the conjugate itself will be in a solid form (*e.g.,* a precipitate), which can be combined with a suitable pharmaceutical excipient that can be in either solid or

liquid form.

Exemplary excipients include, without limitation, those selected from the group consisting of carbohydrates, inorganic salts, antimicrobial agents, antioxidants, surfactants, buffers, acids, bases, and combinations thereof.

A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like.

The excipient can also include an inorganic salt or buffer such as citric acid, sodium chloride, potassium chloride, sodium sulfate, potassium nitrate, sodium phosphate monobasic, sodium phosphate dibasic, and combinations thereof.

The preparation may also include an antimicrobial agent for preventing or deterring microbial growth. Nonlimiting examples of antimicrobial agents suitable for the present invention include benzalkonium chloride, benzethonium chloride, benzyl alcohol, cetylpyridinium chloride, chlorobutanol, phenol, phenylethyl alcohol, phenylmercuric nitrate, thimersol, and combinations thereof.

An antioxidant can be present in the preparation as well. Antioxidants are used to prevent oxidation, thereby preventing the deterioration of the conjugate or other components of the preparation. Suitable antioxidants for use in the present invention include, for example, ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

A surfactant may be present as an excipient. Exemplary surfactants include: polysorbates, such as "Tween 20" and "Tween 80," and pluronics such as F68 and F88 (both of which are available from BASF, Mount Olive, New Jersey); sorbitan esters; lipids, such as phospholipids such as lecithin and other phosphatidylcholines, phosphatidylethanolamines (although preferably not in liposomal form), fatty acids and fatty esters; steroids, such as cholesterol; and chelating agents, such as EDTA, zinc and other such suitable cations.

Pharmaceutically acceptable acids or bases may be present as an excipient in the preparation. Nonlimiting examples of acids that can be used include those acids selected from the group consisting of hydrochloric acid, acetic acid, phosphoric acid, citric acid, malic acid, lactic acid, formic acid, trichloroacetic acid, nitric acid, perchloric acid, phosphoric acid, sulfuric acid, fumaric acid, and combinations thereof. Examples of suitable bases include, without limitation, bases selected from the group consisting of sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumerate, and combinations thereof.

The amount of the conjugate in the composition will vary depending on a number of factors, but will optimally be a therapeutically effective dose when the composition is stored in a unit dose container. A therapeutically effective dose can be determined experimentally by repeated administration of increasing amounts of the conjugate in order to determine which amount produces a clinically desired endpoint.

The amount of any individual excipient in the composition will vary depending on the activity of the excipient and particular needs of the composition. Typically, the optimal amount of any individual excipient is determined through routine experimentation, *i.e.,* by preparing compositions containing varying amounts of the excipient (ranging from low to high), examining the stability and other parameters, and then determining the range at which optimal performance is attained with no significant adverse effects.

Generally, however, the excipient will be present in the composition in an amount of about 1% to about 99% by weight, preferably from about 5%-98% by weight, more preferably from about 15-95% by weight of the excipient, with concentrations less than 30% by weight most preferred.

These foregoing pharmaceutical excipients along with other excipients and general teachings regarding pharmaceutical compositions are described in "Remington: The Science & Practice of Pharmacy", 19th ed., Williams & Williams, (1995), the "Physician's Desk Reference", 52nd ed., Medical Economics, Montvale, NJ (1998), and Kibbe, A.H., Handbook of Pharmaceutical Excipients, 3rd Edition, American Pharmaceutical Association, Washington, D.C., 2000.

The pharmaceutical compositions can take any number of forms and the invention is not limited in this regard. Exemplary preparations are most preferably in a form suitable for oral administration such as a tablet, caplet, capsule, gel cap, troche, dispersion, suspension, solution, elixir, syrup, lozenge, transdermal patch, spray, suppository, and powder.

Oral dosage forms are preferred for those conjugates that are orally active, and include tablets, caplets, capsules, gel caps, suspensions, solutions, elixirs, and syrups, and can also comprise a plurality of granules, beads, powders or pellets that are optionally encapsulated. Such dosage forms are prepared using conventional methods known to those in the field of pharmaceutical formulation and described in the pertinent texts.

Tablets and caplets, for example, can be manufactured using standard tablet processing procedures and

equipment. Direct compression and granulation techniques are preferred when preparing tablets or caplets containing the conjugates described herein. In addition to the conjugate, the tablets and caplets will generally contain inactive, pharmaceutically acceptable carrier materials such as binders, lubricants, disintegrants, fillers, stabilizers, surfactants, coloring agents, and the like. Binders are used to impart cohesive qualities to a tablet, and thus ensure that the tablet remains intact. Suitable binder materials include, but are not limited to, starch (including corn starch and pregelatinized starch), gelatin, sugars (including sucrose, glucose, dextrose and lactose), polyethylene glycol, waxes, and natural and synthetic gums, *e.g.*, acacia sodium alginate, polyvinylpyrrolidone, cellulosic polymers (including hydroxypropyl cellulose, hydroxypropyl methylcellulose, methyl cellulose, microcrystalline cellulose, ethyl cellulose, hydroxyethyl cellulose, and the like), and Veegum. Lubricants are used to facilitate tablet manufacture, promoting powder flow and preventing particle capping (*i.e.,* particle breakage) when pressure is relieved. Useful lubricants are magnesium stearate, calcium stearate, and stearic acid. Disintegrants are used to facilitate disintegration of the tablet, and are generally starches, clays, celluloses, algins, gums, or crosslinked polymers. Fillers include, for example, materials such as silicon dioxide, titanium dioxide, alumina, talc, kaolin, powdered cellulose, and microcrystalline cellulose, as well as soluble materials such as mannitol, urea, sucrose, lactose, dextrose, sodium chloride, and sorbitol. Stabilizers, as well known in the art, are used to inhibit or retard drug decomposition reactions that include, by way of example, oxidative reactions.

Capsules are also preferred oral dosage forms, in which case the conjugate-containing composition can be encapsulated in the form of a liquid or gel (*e.g.,* in the case of a gel cap) or solid (including particulates such as granules, beads, powders or pellets). Suitable capsules include hard and soft capsules, and are generally made of gelatin, starch, or a cellulosic material. Two-piece hard gelatin capsules are preferably sealed, such as with gelatin bands or the like.

Included are parenteral formulations in the substantially dry form (typically as a lyophilizate or precipitate, which can be in the form of a powder or cake), as well as formulations prepared for injection, which are typically liquid and requires the step of reconstituting the dry form of parenteral formulation. Examples of suitable diluents for reconstituting solid compositions prior to injection include bacteriostatic water for injection, dextrose 5% in water, phosphate-buffered saline, Ringer's solution, saline, sterile water, deionized water, and combinations thereof.

In some cases, compositions intended for parenteral administration can take the form of nonaqueous solutions, suspensions, or emulsions, each typically being sterile. Examples of nonaqueous solvents or vehicles are propylene glycol, polyethylene glycol, vegetable oils, such as olive oil and corn oil, gelatin, and injectable organic esters such as ethyl oleate.

The parenteral formulations described herein can also contain adjuvants such as preserving, wetting, emulsifying, and dispersing agents. The formulations are rendered sterile by incorporation of a sterilizing agent, filtration through a bacteria-retaining filter, irradiation, or heat.

The conjugate can also be administered through the skin using conventional transdermal patch or other transdermal delivery system, wherein the conjugate is contained within a laminated structure that serves as a drug delivery device to be affixed to the skin. In such a structure, the conjugate is contained in a layer, or "reservoir," underlying an upper backing layer. The laminated structure can contain a single reservoir, or it can contain multiple reservoirs.

The conjugate can also be formulated into a suppository for rectal administration. With respect to suppositories, the conjugate is mixed with a suppository base material which is (*e.g.,* an excipient that remains solid at room temperature but softens, melts or dissolves at body temperature) such as coca butter (theobroma oil), polyethylene glycols, glycerinated gelatin, fatty acids, and combinations thereof. Suppositories can be prepared by, for example, performing the following steps (not necessarily in the order presented): melting the suppository base material to form a melt; incorporating the conjugate (either before or after melting of the suppository base material); pouring the melt into a mold; cooling the melt (*e.g.,* placing the melt-containing mold in a room temperature environment) to thereby form suppositories; and removing the suppositories from the mold.

## Administration

The invention also provides a method for administering a conjugate as provided herein to a patient suffering from a condition that is responsive to treatment with the conjugate. The method comprises administering, generally orally, a therapeutically effective amount of the conjugate (preferably provided as part of a pharmaceutical preparation). Other modes of administration are also contemplated, such as pulmonary, nasal, buccal, rectal, sublingual, transdermal, and parenteral. As used herein, the term "parenteral" includes subcutaneous, intravenous, intra-arterial, intraperitoneal, intracardiac, intrathecal, and intramuscular injection, as well as infusion injections.

In instances where parenteral administration is utilized, it may be necessary to employ somewhat bigger oligomers than those described previously (e.g., polymers), with molecular weights ranging from about 500 to 30K Daltons (*e.g.*, having molecular weights of about 500, 1000, 2000, 2500, 3000, 5000, 7500, 10000, 15000, 20000, 25000, 30000 or even more).

The method of administering may be used to treat any condition that can be remedied or prevented by administration of the particular conjugate. Those of ordinary skill in the art appreciate which conditions a specific conjugate

can effectively treat. The actual dose to be administered will vary depend upon the age, weight, and general condition of the subject as well as the severity of the condition being treated, the judgment of the health care professional, and conjugate being administered. Therapeutically effective amounts are known to those skilled in the art and/or are described in the pertinent reference texts and literature. Generally, a therapeutically effective amount will range from about 0.001 mg to 1000 mg, preferably in doses from 0.01 mg/day to 750 mg/day, and more preferably in doses from 0.10 mg/day to 500 mg/day.

The unit dosage of any given conjugate (again, preferably provided as part of a pharmaceutical preparation) can be administered in a variety of dosing schedules depending on the judgment of the clinician, needs of the patient, and so forth. The specific dosing schedule will be known by those of ordinary skill in the art or can be determined experimentally using routine methods. Exemplary dosing schedules include, without limitation, administration five times a day, four times a day, three times a day, twice daily, once daily, three times weekly, twice weekly, once weekly, twice monthly, once monthly, and any combination thereof. Once the clinical endpoint has been achieved, dosing of the composition is halted.

One advantage of administering the conjugates of the present invention is that a reduction in speed of delivery of the opioid agonist to the brain is achieved, thus avoiding the rapid peak concentrations associated with unconjugated opioid agonists and underlying addictive highs. Moreover, based on the covalent modification of the opioid agonist molecule, the conjugates of the invention are not subject to the risk of physical tampering that allows for the recovery and abuse of the rapid acting opioid compound associated with certain alternative delivery forms intended to provide, *in vivo,* a reduced BBB crossing rate. As such, the compounds of the invention possess low addictive, anti-abuse properties. The desired pharmacokinetic properties of the conjugates may be modulated by selecting the oligomer molecular size, linkage, and position of covalent attachment to the opioid compound. One of ordinary skill in the art can determine the ideal molecular size of the oligomer based upon the teachings herein.

Uses

Thus, the invention provides a method of treating a patient in need of opioid therapy comprising administering an effective amount of a compound having a formula OP-X-POLY as disclosed herein above. Preferably, the invention further provides a method of reducing the abuse potential of an opioid compound comprising conjugating the compound to a small water-soluble oligomer. Preferably, the conjugate is of the formula OP-X-POLY as described herein.

In a further embodiment, the invention provides a method of reducing the addictive properties of an opioid agonist comprising conjugating the opioid agonist to a small water-soluble oligomer. Preferably, the conjugate is of the formula OP-X-POLY as described herein.

In another embodiment, provided is a method of reducing, but not substantially eliminating, the rate of crossing the blood brain barrier of an opioid compound comprising conjugating the compound to a small water-soluble oligomer. Preferably, the conjugate is of the formula OP-X-POLY as described herein.

It is to be understood that while the invention has been described in conjunction with certain preferred and specific embodiments, the foregoing description as well as the examples that follow are intended to illustrate and not limit the scope of the invention. Other aspects, advantages and modifications within the scope of the invention will be apparent to those skilled in the art to which the invention pertains.

## EXAMPLES

All chemical reagents referred to in the appended examples are commercially available unless otherwise indicated. The preparation of PEG-mers is described in, for example, U.S. Patent Application Publication No. 2005/0136031.

### Example 1

### Determination of logP Values

Log P and Log D provide measures of the lipophilicity of a compound, such that a higher or more positive value represents a more hydrophobic compound whereas a lower or more negative value represents a more hydrophilic compound. LogP (Octanol: isopronalol/water partition coefficient) of test compounds is measured using a potentiometric titration method with a Sirius GLpKa instrument (Sirius Analytical Instruments, Ltd, East Sussex, UK). A 50 μL aliquot of the 0.1 M test compound solution in DMSO is placed into a titration vial. Assays are conducted at 25°C. A measured volume of octanol is added to the sample automatically, after which the instrument adds a measured volume of isopropanol water. The pH of the solution is adjusted to 2 by adding 0.5 M HCl automatically. A titration with 0.5 M KOH is performed automatically until a pH value of 12 is reached. To perform the second and third titrations, an additional octanol volume is delivered automatically to the titration vial. The data sets for the three titrations are combined in RefinementPro to

create a Multiset. The Log (D), at various pH values, is automatically calculated by the software.

Log P and Log D values are used in predicting or evaluating the properties of a molecule that relate to its lipophilicity such as the ability to traverse membranes.

**Example 2**

**Transport assays for PgP**

P-glycoprotein, PgP, is an efflux transporter expressed in various cells in the body, and highly expressed at the blood-brain barrier. Molecules that are substrates for PgP show poor penetration into, or efflux from, tissues where the PgP is expressed.

The contribution of PgP to net transport is measured in MDCKII cells that over-express PgP (MDR-MDCKII). For transport studies, MDR-MDCKII and MDCKII cells are grown on permeable inserts (3-4 days) until a tight monolayer is formed, as measured by transepithelial measurements. Test compounds in Krebs buffer are added at 10μM to the apical or basolateral sides of the MDCKII cells and allowed to incubate at 37°C. The transport of compounds is measured in two directions: Apical-basolateral (A-B) and basolateral-apical (B-A) in both parent and MDR overexpressing cells. At times 0, 30, 60, 90, 120 and 180 minutes, aliquots are withdrawn from the apical and basolateral compartments. Samples are analyzed for test compounds by LC-MS/MS. The flux is calculated as the slope of the linear portion of the cumulative concentration versus time plot. The apparent permeability is calculated as Papp = Flux/$C_0$.A, where $C_0$ is the initial concentration of test compound (10 μM) used and A is the surface area of the insert. The efflux ratio is calculated as the ratio of Papp(B-A)/Papp(A-B). Involvement of a PgP mediated efflux mechanism is indicated when the ratio of the efflux in MDCKII -MDRI cells to that in parent MDCKII cells is greater than 2, *i.e.* Efflux ratio(MDCKII-MDRI)/Efflux ratio (MDCKII cells) ≥ 2.

PgP interaction data are used in predicting or evaluating the properties of a molecule that relate to its PgP status such as the ability to traverse membranes or enter compartments such as the CNS where PgP is highly expressed.

**Example 3**

***In situ* Rat Brain Perfusion**

The *in situ* perfusion experiment measures the relative permeability of compounds across a model of the blood-brain barrier. *In situ* perfusion of opioids into rat brain is performed as described in Summerfield et al., J Pharmacol Exp Ther 322: 205-213 (2007).

Adult male Sprague Dawley rats are used for the study. Rats are anaesthetized and the left common carotid artery is surgically cannulated for perfusion. Test compounds are perfused at concentrations of 5-50 μM in a Krebs Ringer perfusion buffer (pH 7.4). Atenolol and antipyrine are included as low and moderate permeability markers, respectively. At the end of a 30 second perfusion, the brains are removed, the left brain hemisphere is excised and homogenized. Test compounds are extracted and analyzed using LC-MS/MS. The brain permeability of the test compounds is calculated as follows:

$$P = Kin/S,$$

where P is the permeability in cm/s, Kin is the unidirectional transfer constant (ml/min/gram), and S is the luminal area of the brain vascular space.

The relative permeability as determined in the in situ brain perfusion experiment provides information regarding the rates at which compounds enter the central nervous system from the periphery. It is used to characterize and compare the degree to which conjugation with a water-soluble oligomer slows penetration of the BBB for a given opioid compound.

The following experiments were conducted to determine the rate of brain penetration of certain exemplary oligomeric PEG-opioids:

The brain penetration potential morphine, codeine, oxydone and their respective PEG conjugates was evaluated in male Sprague-Dawley rats using an *in-situ* brain perfusion model. Synthesis of the conjugates is described in Examples 10, 11 and 12. Rats were anesthetized and a cannula was implanted into the left carotid artery. Branch arteries were tied, and the cardiac supply was cut off prior to brain perfusion. Perfusion was performed using the single time-point method. Each animal was co-perfused with a test compound (10 μM) and control compounds (5 μM antipyrine and 50 μM atenolol). The compounds in Kreb's Ringer buffer were infused into the animals via the left external carotid artery for 30 seconds by an infusion pump. Following 30 seconds of perfusion, the pump was stopped, and the brain was immediately removed from the skull. The brain was cut longitudinally in half. Each left hemisphere was placed into a

chilled tube, frozen on dry ice, and stored frozen at -60°C to -80°C until analyzed.

For bioanalysis, each left brain hemisphere was thawed, weighed and homogenized by sonication in 20% methanol. Concentrations of test and control compounds were determined by LC-MS/MS analyses using pre-validated analytical methods.

Results for the brain penetration of the test and control compounds are presented as the unidirectional brain transfer constants Kin (mL/g/min) using the following equation for the single-point perfusion assay:

$$Kin = [Cbr/Cpf]/t,$$

where:

Cbr/Cpf is the apparent brain distribution volume (mL/g of brain tissue).
Cbr is the concentration of drug in the brain tissue (pmol of drug per g of brain tissue).
Cpf is the drug concentration in the perfusion fluid (pmol/mL of perfusate).
t is the net perfusion time (minutes).

To exclude the drug contained in the capillary space from the brain concentration values, the apparent brain distribution volume of atenolol was subtracted from the drug values in each animal. If the concentration of the test compound was a negative value after correcting for the brain distribution volume of atenolol, the Kin value is reported as zero.

Following perfusion, the vascular space marked by atenolol, a compound that does not penetrate the brain, did not exceed 20 μL/g of brain tissue. These results indicate preserved blood-brain barrier properties during perfusion. The Kin values for morphine, codeine and oxycodone are shown in FIGS. 18A-C and FIG. 19. The Kin values for parent morphine, codeine and oxycodone compounds were approximately 14%, 40% and 60% of Kin values of antipyrine, the positive control that possess high brain penetration potential. PEG conjugation resulted in a further size-dependent decrease in the rate of brain entry of codeine and oxycodone conjugates. The rates of brain entry of PEG-7 codeine and PEG-7-oxycodone were <1% of their respective parent compounds. However, the Kin values of PEG-1, PEG-2 morphine were greater than parent morphine, and equivalent to parent in the case of PEG-3-morphine. The Kin value of PEG-7-morphine was significantly lower (<4%) than that of parent morphine.

### Example 4

### Opioid Receptor Binding Assay in Whole Cells

Receptor binding affinity is used as a measure of the intrinsic bioactivity of the compound. The receptor binding affinity of the opioid conjugates (or opioid alone) is measured using a radioligand binding assay in CHO cells that heterologously express the recombinant human mu, delta or kappa opioid receptor. Cells are plated in 24 well plates at a density of 0.2-0.3 x $10^{-6}$ cells/well and washed with assay buffer containing 50 mM Tris.HCl and 5 mM $MgCl_2$ (pH 7.4). Competition binding assays are conducted on adherent whole cells incubated with increasing concentrations of opioid conjugates in the presence of an appropriate concentration of radioligand. 0.5 nM [$^3$H]naloxone, 0.5 nM [$^3$H]diprenorphine and 0.5 nM [$^3$H]DPDPE are used as the competing radioligands for mu, kappa and delta receptors respectively. Incubations are carried out for 2 hours at room temperature using triplicate wells at each concentration. At the end of the incubation, cells are washed with 50 mM Tris HCl (pH 8.0), solubilized with NaOH and bound radioactivity is measured using a scintillation counter.

Specific binding is determined by subtraction of the cpm bound in the presence of 50-100X excess of cold ligand. Binding data assays are analyzed using GraphPad Prism 4.0 and IC50 is generated by non-linear regression from dose-response curves. Ki values are calculated using the Cheng Prusoff equation using the Kd values from saturation isotherms as follows: Ki = IC50/(1+ [Ligand]/Kd).

The Ki value is used as an indicator of the binding affinity of the compound and may be compared to the binding affinity of other opioid agonists. It also is used as a marker for potency and permits evaluation of the likelihood of a given compound to provide effective analgesia.

### Example 5

### cAMP Measurements in Whole Cells

Inhibition of forskolin-stimulated cAMP production is used as a measure of *in vitro* bioactivity of opioids. CHO

cells that heterologously express any one of the recombinant human mu, delta or kappa opioid receptor are plated in 24 well plates at 0.2-0.3x$10^{-6}$ cells/well and washed with PBS + 1 mM IBMX (isobutyl methyl xanthine). Cells in triplicate wells are incubated with increasing concentrations of opioid conjugate followed 10 mins later by the addition of 10 μM forskolin. Following incubation with forskolin for 10 minutes, cells are lysed and cAMP in cells is measured using a commercially available competitive immunoassay kit (Catchpoint®- Molecular Devices). The fluorescence signal is calibrated against a standard curve of cAMP and data are expressed as moles of cAMP/$10^6$ cells. IC50 values are calculated for each opioid conjugate by analysis of the dose-response curve using non-linear regression (Graph Pad Prism), where "dose" is the concentration of the opioid conjugate used.

The cAMP assay is used to provide a measure of the ability of an opioid compound to induce a functional response upon receptor binding, and provides a further indication of the analgesic potential of the compound. It also enables comparison with other opioids for relative potency.

**Example 6**

**Rat Model of Analgesia**

The hotplate withdraw assay is used as a measure of *in vivo* bioactivity of opioids. This experiment uses a standard hotplate withdrawal assay in which latency of withdrawal from a heat stimulus is measured following administration of a test compound. Compounds are administered to the animal and 30 minutes later, a thermal stimulus is provided to the hindpaw. Latency for hindpaw withdrawal in the presence of morphine is used as the measure of full analgesia, while latency in the presence of saline is used as a negative control for no analgesia. The agonist effect of the test compound is evaluated by measuring time to withdrawal compared with a negative control (saline).

**Example 7**

**Monkey Model of Addictive Potential**

Addictive potential of opioid compounds and opioid conjugates of the invention may be assessed through the use of squirrel monkey models as known in the art. Bergman and Paronis, Mol Interventions, 6:273-83 (2006).

**Example 8**

***In vivo* Brain Penetration of PEG-Nalbuphine Conjugates**

The ability of the PEG-nalbuphine conjugates to cross the blood brain barrier (BBB) and enter the CNS was measured using the brain:plasma ratio in rats. Briefly, rats were injected intravenously with 25 mg/kg of nalbuphine, PEG-nalbuphine conjugate or atenolol. An hour following injection, the animals were sacrificed and plasma and the brain were collected and frozen immediately. Following tissue and plasma extractions, concentrations of the compounds in brain and plasma were measured using LC-MS/MS. The brain:plasma ratio was calculated as the ratio of measured concentrations in the brain and plasma. Atenolol, which does not cross the BBB, was used as a measure of vascular contamination of the brain tissue.

FIG. 1 shows the ratio of brain:plasma concentrations of PEG-nalbuphine conjugates. The brain:plasma ratio of nalbuphine was 2.86:1, indicating a nearly 3 fold greater concentration of nalbuphine in the brain compared to the plasma compartment. PEG conjugation significantly reduced the entry of nalbuphine into the CNS as evidenced by a lower brain:plasma ratio of the PEG-nalbuphine conjugates. Conjugation with 3 PEG units reduced the brain:plasma ratio to 0.23:1, indicating that the concentration of 6-O-mPEG$_3$-Nalbuphine in the brain was approximately 4 fold less than that in the plasma. 6-O-mPEG$_6$-Nalbuphine and 6-O-mPEG$_9$-Nalbuphine (6 PEG units and 9 PEG units, respectively) were significantly excluded from the CNS, since their brain:plasma ratios were not significantly different from the vascular marker, atenolol.

**Table 1. Brain:Plasma Ratios**

| Molecule | Brain: Plasma Ratios |
|---|---|
| Nalbuphine | 2.86 |
| 6-O-mPEG$_3$-Nalbuphine | 0.23 |
| 6-O-mPEG$_6$-Nalbuphine | 0.11 |
| 6-O-mPEG$_9$-Nalbuphine | 0.10 |
| Atenolol | 0.11 |

## EXAMPLE 9

### Preparation of $mPEG_n$-OMs (mPEGn-O-Mesylate)

In a 40-mL glass vial was mixed HO-$CH_2CH_2OCH_2CH_2$-OH (1.2 ml, 10 mmol) and DIEA (N,N-diisopropylethylamine, 5.2 ml, 30 mmol, 3 eq), the resulting homogeneous colorless mixture was cooled to 0 °C and MsCl (1.55 ml, 20 mmol, 2 eq) was added via syringe slowly, over 4 minutes, with vigorous stirring. A biphasic mixture resulted upon addition: yellow solid on the bottom and clear supernatant. The ice bath was removed and the reaction was allowed to warm to room temperature overnight. At this point it was dissolved in water, extracted with $CHCl_3$(3x50 mL), washed with 0.1M HCl/brine mixture 2x50 mL, followed by brine 50 mL. The organic layer was dried over $MgSO_4$, filtered to give a yellow solution and evaporated to give brown oil (2.14 g). $^1$H NMR confirms product identity 3.3 (1H NMR $\delta$ 3.1 (s, 3H), 3.3 (s, 3H), 3.5-3.55 (m, 2H), 3.6-3.65 (m, 2H), 3.7-3.75 (m, 2H), 4.3-4.35 (m, 2H) ppm).

All other $PEG_n$-OMs's (n = 3, 4, 5, 6, 7 and 9) were made in similar fashion and afforded final compounds in each case that were isolated as brown oils. Mass spectral and proton NMR data (not shown) confirmed the formation of the desired OMs PEGylated products.

## EXAMPLE 10

### Preparation of $mPEG_n$-O-Morphine Conjugates

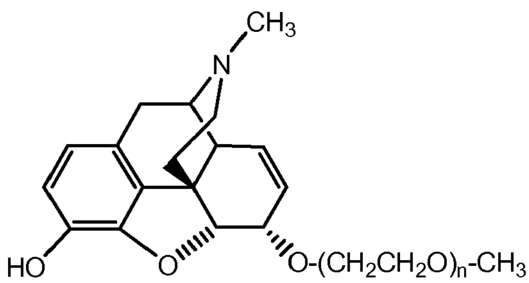

The following describes the preparation of free base using commercially available morphine sulfate hydrate (generally procedure).

Morphine sulfate USP from Spectrum (510 mg) was dissolved in water (70 ml). The solution was then basified to pH 10 using aqueous $K_2CO_3$ to give a white suspension. To the white suspension DCM (dichloromethane, 50 ml) was added, but failed to dissolve the solid. The mixture was made acidic with 1M HCl to result in clear biphasic solution. The organic phase was split off and the aqueous phase was carefully brought to pH 9.30 (monitored by a pH meter) using the same solution of $K_2CO_3$ as above. A white suspension resulted again. The heterogeneous mixture was extracted with DCM (5x25 ml) and an insoluble white solid contaminated both the organic and aqueous layers. The organic layer was dried with $MgSO_4$, filtered and rotary evaporated to yield 160 mg of morphine free base (56% recovery). No additional product was recovered from the filter cake using MeOH, but another 100 mg was recovered from the aqueous phase by 2x50ml extraction with EtOAc to give a combined yield of 260 mg (68%).

MEM Protection of Morphine free base

The general approach for protecting the free base of morphine with the protecting group $\beta$-methoxyethoxymethyl ester ("MEM") is schematically shown below.

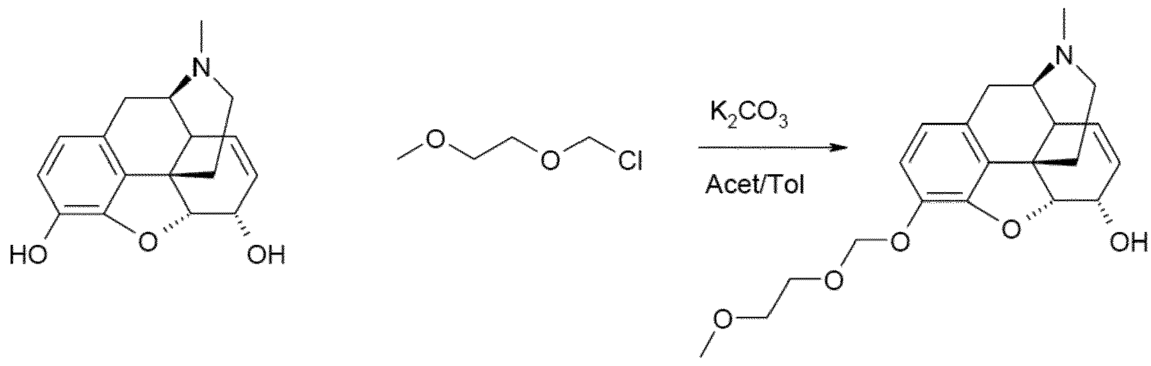


Free base morphine (160 mg, 0.56 mmol) was dissolved in 20 ml of Acetone/Toluene (2/1 mixture). To the resulting solution was added $K_2CO_3$ (209 mg, 1.51 mmol, 2.7 eq) followed by MEMC1 (96 µl, 0.84 mmol, 1.5 eq) and the resulting heterogeneous mixture was stirred overnight at room temperature. After five hours at room temperature, the reaction was deemed comlete by LC-MS. Morphine free base retention time under standard six minute gradient run conditions (std 6 min, Onyx Monolyth C18 column, 50x4.6 mm; 0 to 100% Acetonitrile 0.1% TFA in Water 0.1% TFA, 1.5 ml/min; detection: UV254, ELSD, MS; retention times are quoted for UV254 detector, ELSD has about 0.09 min delay and MS has about 0.04 min delay relative to UV) was 1.09 min; retention time for product 1.54 min (std 6 min), major impurity 1.79 min. The reaction mixture was evaporated to dryness, dissolved in water, extracted with EtOAc (3x, combined organic layer washed with brine, dried over $MgSO_4$, filtered and rotary evaporated) to give 160 mg (77%) of the desired product as a colorless oil. Product purity was estimated to be about 80% by UV254.

Direct MEM protection of morphine sulfate (general procedure)

The general approach for protecting morphine sulfate with the protecting group β-methoxyethoxymethyl ester ("MEM") is schematically shown below. Although not explicitly shown in the scheme below, morphine is actually morphine sulfate hydrate, morphine.0.5 $H_2SO_4$.2.5 $H_2O$.

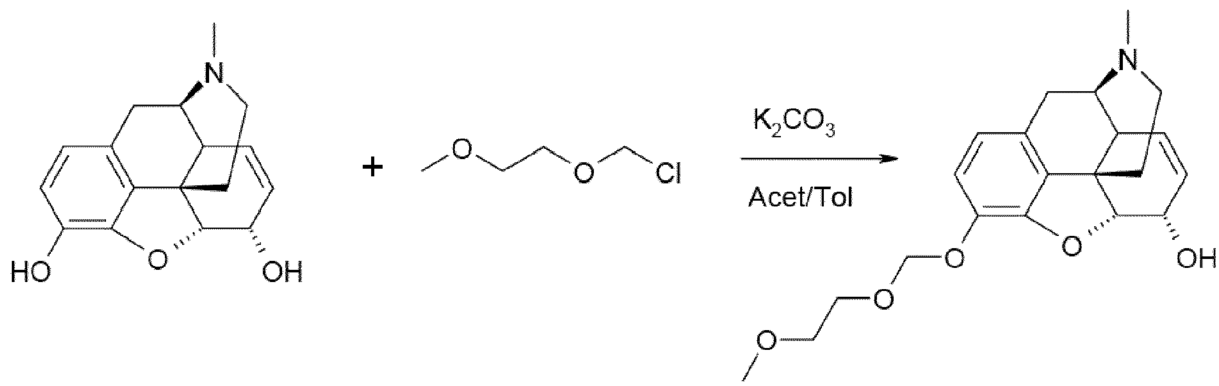


To a suspension of 103 mg of morphine sulfate hydrate (0.26 mmol) in 10 ml of 2:1 acetone:toluene solvent mixture was added 135 mg (1 mmol, 3.7 eq) of $K_2CO_3$ and the suspension stirred at room temperature for 25 minutes. To the resulting suspension was added 60 µl (0.52 mmol) of MEMC1 and the mixture allowed to react at room temperature. It was sampled after one hour (38% nominal conversion, additional peaks at 1.69 min and 2.28 min), three hours (40% nominal conversion, additional peak at 1.72 min (M+1 =493.2)), four and one-half hours (56% nominal conversion, additional peak at 1.73 min), and twenty-three hours (>99% nominal conversion, additional peak at 1.79 min - about 23% of the product peak by height in $UV_{254}$); thereafter, the reaction was quenched with MeOH, evaporated, extracted with EtOAc to give 160 mg of clear oil.

The same reaction was repeated starting with 2 g (5.3 mmol) of morphine sulfate hydrate, 2.2 g (16 mmol, 3 eq) of $K_2CO_3$, 1.2 ml (10.5 mmol, 2 eq) of MEMC1 in 100 ml of solvent mixture. Sampling occurred after two hours (61% nominal conversion, extra peak at 1.72 min (M+1 = 492.8)), after one day (80% nominal conversion, extra peak at 1.73 min), after three days (85% nominal conversion, only small impurities, 12 min gradient run), and after six days (91% conversion); thereafter, the reaction was quenched, evaporated, extracted with EtOAc, purified on combi-flash using a 40 g column, DCM:MeOH 0 to 30% mobile phase. Thre peaks (instead of two) were identified, wherein the middle peak

was collected, 1.15 g (58% yield) of light yellow oil, $UV_{254}$ purity about 87%.

Conjugation of MEM-protected morphine to provide a MEM-protected morphine conjugate

The general approach for conjugating MEM-protected morphine with a water-soluble oligomer to provide a MEM-protected morphine PEG-oligomer conjugate is schematically shown below.

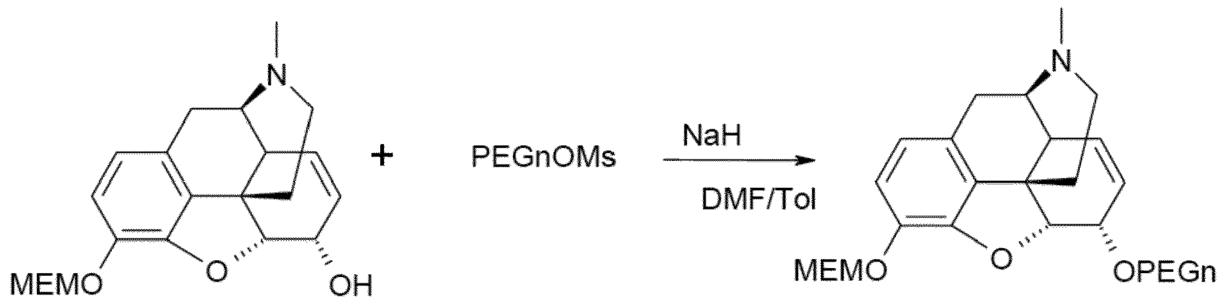


To a solution of toluene/DMF (2:1 mixture, 10 volumes total) was charged MEM-morphine free base followed by NaH (4-6 eq) and then $PEG_nOMs$ (1.2-1.4 eq.), previously prepared. The reaction mixture was heated to 55-75 °C and was stirred until reaction completion was confirmed by LC-MS analysis (12-40 hours depending on PEG chain length). The reaction mixture was quenched with methanol (5 volumes) and the reaction mixture was evaporated to dryness *in vacuo.* The residue was redissolved in methanol (3 volumes) and was chromatographed using a Combiflash system (0-40% MeOH/DCM). The fractions containing large amounts of product were collected, combined and evaporated to dryness. This material was then purified by RP-HPLC to give the products as yellow to orange oils.

Deprotection of MEM-protected morphine conjugate to provide a morphine conjugate

The general approach for deprotecting a MEM-protected morphine conjugate to provide a morphine conjugate is schematically shown below.

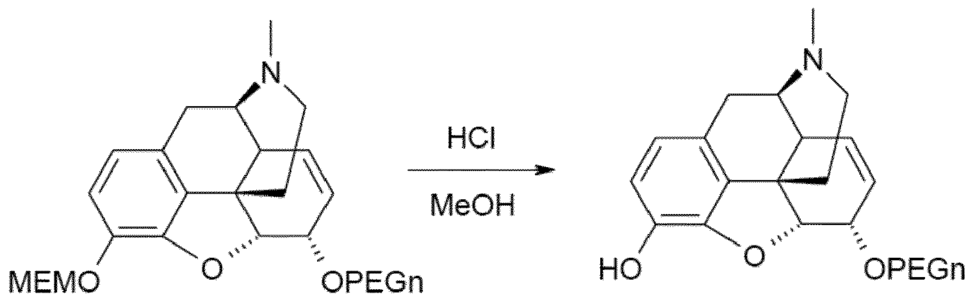


**Compound A**

To a solution of MEM-protected morphine conjugate TFA salt suspended in DCM (8 volumes) was charged 6 volumes of 2M HCl in diethyl ether. The reaction mixture was allowed to stir at room temperature for two hours and was then evaporated to dryness under reduced pressure. The oily residue was dissolved in MeOH (8 volumes), filtered through glass wool and then evaporated under reduced pressure to give a thick orange to yellow oil in quantitative yield. Compounds made by this method include: $\alpha$-6-$mPEG_3$-O-morphine (Compound A, n=3) 217 mg of HCl salt 97% pure (95% by UV254; 98% by ELSD); $\alpha$-6-$mPEG_4$-O-morphine (Compound A, n=4) 275 mg of HCl salt 98% pure (97% by UV254; 98% by ELSD); $\alpha$-6-$mPEG_5$-O-morphine (Compound A, n=5) 177 mg of HCl salt 95% pure (93% by UV254; 98% by ELSD); $\alpha$-6-$mPEG_6$-O-morphine (Compound A, n=6) 310 mg of HCl salt 98% pure (98% by UV254; 99% by ELSD); $\alpha$-6-$mPEG_7$-O-morphine (Compound A, n=7)541 mg of HCl salt 96% pure (93% by UV254; 99% by ELSD); and $\alpha$-6-mPEG-$O_9$-morphine (Compound A, n=9) 466 mg of HCl salt 98% pure (97% by UV254; 99% by ELSD). Additionally, morphine conjugates having a single PEG monomer attached, $\alpha$-6-$mPEG_1$-O-morphine (Compound A, n=1), 124 mg of HCl salt, 97% pure (95% pure by $UV_{254}$; 98% by ELSD); as well as $\alpha$-6-$mPEG_2$-O-morphine (Compound A, n=2), 485 mg of HCl salt, 97% pure (95% pure by $UV_{254}$; 98% by ELSD) were similarly prepared.

## EXAMPLE 11

### Preparation of $mPEG_n$-O-Codeine Conjugates

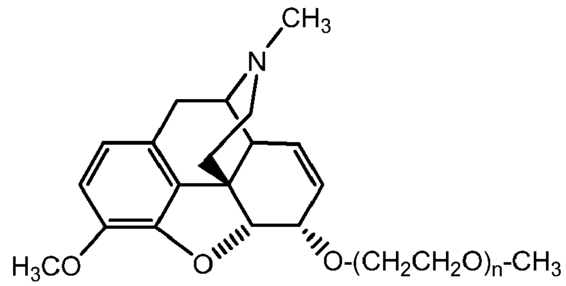

The general approach for conjugating codeine with an activated sulfonate ester of a water-soluble oligomer (using $mPEG_3OMs$ as a representative oligomer) to provide a codeine conjugate is schematically shown below.

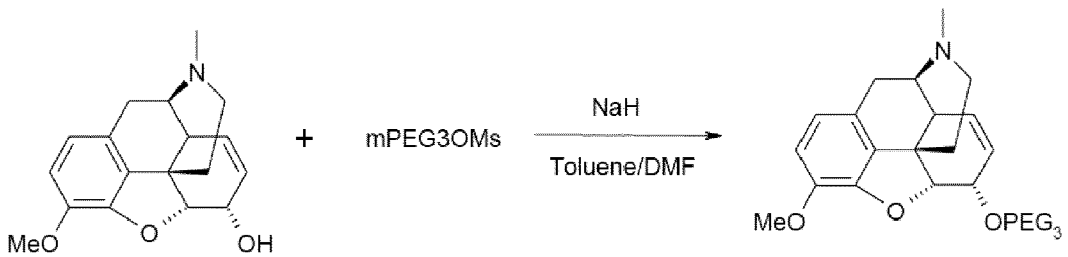


Codeine (30 mg, 0.1 mmol) was dissolved in toluene/DMF (75:1) solvent mixture followed by addition of HO-$CH_2CH_2OCH_2CH_2OCH_2CH_2OMs$ (44 ml, 2eq) and NaH (60% suspension in mineral oil, 24 mg, 6 eq). The resulting homogeneous yellow solution was heated to 45 °C. After one hour, the reaction showed 11% conversion (extra peak at 2.71 min, 12 min run), after eighteen hours, the reaction showed 7% conversion (extra peak at 3.30 min, 12 min run), and after 24 hours, the reaction showed 24% conversion (multitude of extra peaks, two tallest ones are 1.11 min and 2.79 min). At this point, an additional 16 mg of NaH was added and heating continued for six hours, after which, an additional 16 mg of NaH was added followed by continued heating over sixty-six hours. Thereafter, no starting material remained, and analysis revealed many extra peaks, the two tallest ones corresponding to 2.79 min and 3 min (product peak is the second tallest among at least 7 peaks).

This synthesis was repeated using 10x scale wherein 30 ml of solvent mixture was used. After eighteen hours, analysis revealed 71% nominal conversion with additional peaks in the UV (one tall peak at 3.17 min and many small ones; wherein the desired peak corresponded to 3.43 min in UV). Thereafter, 80 mg (2 mmol) of NaH was added followed by continued heating. After three hours, analysis revealed 85% nominal conversion (several extra peaks, main 3.17 min). Reaction mixture was diluted with water, extracted with EtOAc (3x, combined organic layer washed with brine, dried over $MgSO_4$, filtered and rotary evaporated) to give yellow oil (no sm in LC-MS, 90% pure by ELSD, 50% pure by UV - major impurity at 3.2 min). The crude product was dissolved in DCM, applied to a small cartridge filled with 230-400 mesh $SiO_2$, dried, eluted on a Combi-flash via a 4g pre-packed column cartridge with solvent A = DCM and solvent B = MeOH, gradient 0 to 30% of B. Analysis revealed two peaks of poor symmetry: a small leading peak and a larger peak with a tail. LC-MS was used to analyze fractions, wherein none were identified as containing pure product. Combined fractions that contained any product (tt#22-30) yielded, following solvent evaporation, 150 mg (34% yield) of impure product (LC-MS purity at 3.35 min by UV254, wherein about 25% represented the main impurities 3.11 min, 3.92 min, 4.32 min, 5.61 min of a 12 min run). A second purification by HPLC (solvent A = water, 0.1% TFA; solvent B = acetonitrile, 0.1% TFA) employing a gradient corresponding to 15-60% B, 70 min, 10 ml/min) resulted in poor separation from adjacent peaks. Only two fractions were clean enough and gave 21 mg of TFA salt (>95% pure, 4.7% yield). Three additional fractions both before and after the desired product-containing fractions (for a total of six additional fractions were combined to give 70 mg of about 50% pure product as TFA salts.

Using this same approach, other conjugates differing by the number of ethylene oxide units (n= 4, 5, 6, 7, and 9) were made using these NaH conditions outlined above.

Converstion of Codeine-Oligomer Conjugate TFA Salts to Codeine-Oligomer HCl salts.

The general approach for converting codeine-oligomer TFA salts to codeine-oligomer HCl salts is schematically shown below.

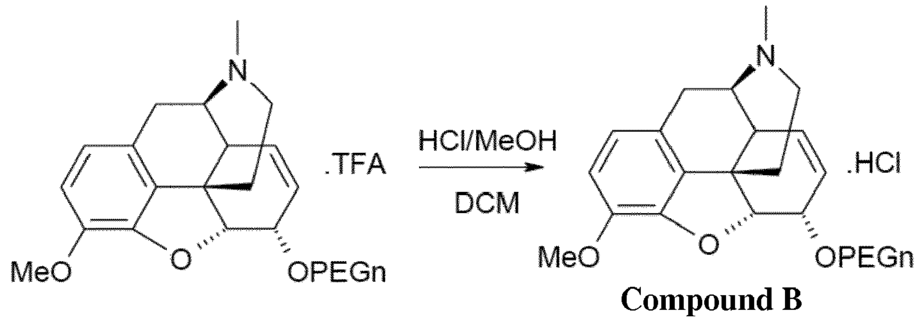


**Compound B**

To a solution of codeine-oligomer conjugate TFA salt suspended in DCM (8 volumes) was charged 6 volumes of 2M HCl in diethyl ether. The reaction mixture was allowed to stir at room temperature for two hours and was then evaporated to dryness under reduced pressure. The oily residue was dissolved in MeOH (8 volumes), filtered through glass wool and then evaporated under reduced pressure to give a thick orange to yellow oil in quantitative yield. Following this general procedure, the following compounds were synthesized: α-6-$mPEG_3$-O-codeine (Compound B, n=3) 235 mg of HCl salt, 98% pure; α-6-$mPEG_4$-O-codeine (Compound B, n=4) 524 mg of HCl salt, 98% pure; α-6-$mPEG_5$-O-codeine (Compound B, n=5) 185 mg of HCl salt, 98% pure + 119 mg of HCl salt 97% pure, α-6-$mPEG_6$-O-codeine (Compound B, n=6) 214 mg of HCl salt, 97% pure; α-6-$mPEG_7$-O-codeine (Compound B, n=7) 182 mg of HCl salt, 98% pure; α-6-$mPEG_9$-O-codeine (Compound B, n=9) 221 mg of HCl salt, 97% pure; α-6-$mPEG_1$-O-codeine (Compound B, n=1) 63 mg of HCl salt, 90% pure; and α-6-$mPEG_2$-O-codeine (Compound B, n=2) 178 mg of HCl salt, 90% pure.

## EXAMPLE 12

### Preparation of $mPEG_n$-O-Hydroxycodone Conjugates

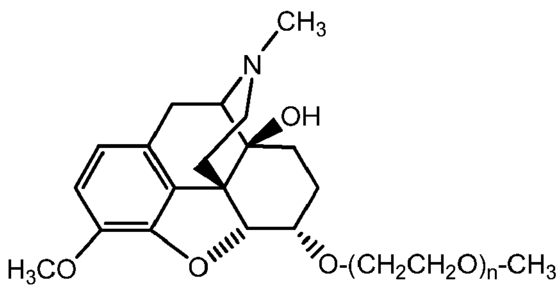

The general approach for conjugating hydroxycodone with an activated sulfonate ester of a water-soluble oligomer (using "$mPEG_nOMs$" as a representative oligomer) to provide a hydroxycodone conjugate is schematically shown below.

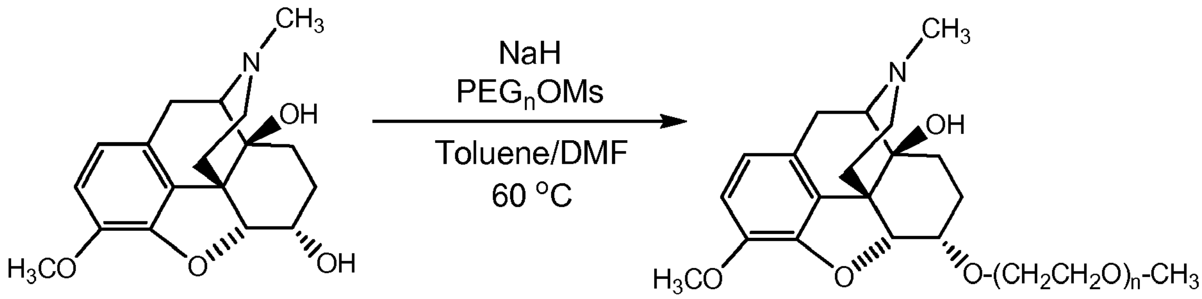


**Compound C**

Reduction of Oxycodone to α-6-hydroxycodone:

To a solution of oxycodone free base in dry THF under nitrogen cooled at -20 °C, was added a 1.0 M THF solution of potassium tri-sec-butylborohydride over 15 minutes. The solution was stirred at -20 °C under nitrogen for 1.5 hours and then water (10 mL) was added slowly. The reaction mixture was stirred another 10 minutes at -20 °C and then allowed to warm to room temperature. All solvents were removed under reduced pressure and $CH_2Cl_2$ was added to the remaining residue. The $CH_2Cl_2$ phase was extracted with a 0.1 N HCl/NaCl water solution and the combined 0.1 N HCl solution extracts were washed with $CH_2Cl_2$, then $Na_2CO_3$ was added to adjust the pH = 8. The solution was extracted with $CH_2Cl_2$. The $CH_2Cl_2$ extracts were dried over anhydrous $Na_2SO_4$. After removing the solvent under reduced pressure, the desired α-6-HO-3-hydroxycodone was obtained.

Conjugation of $mPEG_nOMs$ to α-6-hydroxycodone: To a solution of Toluene/DMF (2:1 mixture, 10 volumes total) was charged hydroxycodone (prepared as set forth in the preceding paragraph) followed by NaH (4 eq) and then $mPEG_nOMs$ (1.3 e.). The reaction mixture was heated to 60-80 °C and was stirred until reaction completion was confirmed by LC-MS analysis (12-40 hours depending on PEG chain length). The reaction mixture was quenched with methanol (5 volumes) and the reaction mixture was evaporated to dryness in vacuo. The residue was re-dissolved in methanol (3 volumes) and was chromatographed using Combiflash (0-40% MeOH/DCM). The fractions containing large amounts of product were collected, combined and evaporated to dryness. This material was then purified by RP-HPLC to provide the final products as yellow to orange oils.

Conversion of hydroxycodone conjugate TFA salts to hydroxycodone conjugate HCl salts

To a solution of hydroxycodone conjugate TFA salt suspended in DCM (8 volumes) was charged 6 volumes of 2M HCl in diethyl ether. The reaction mixture was allowed to stir at room temperature for two hours and was then evaporated to dryness under reduced pressure. The oily residue was dissolved in MeOH (8 volumes), filtered through glass wool and then evaporated under reduced pressure to give a thick orange to yellow oil in quantitative yield. Following this general procedure, the following compounds were synthesized: α-6-$mPEG_3$-O-oxycodone (aka α-6-$mPEG_3$-O-hydroxycodone) (Compound C, n=3) 242 mg of HCl salt, 96% pure; α-6-$mPEG_4$-O-oxycodone (aka α-6-$mPEG_4$-O-hydroxycodone) (Compound C, n=4) 776 mg of HCl salt, 94% pure; α-6-$mPEG_5$-O-oxycodone (aka α-6-$mPEG_5$-O-hydroxycodone) (Compound C, n=5) 172 mg of HCl salt, 93% pure; α-6-$mPEG_6$-O-oxycodone (aka α-6-$mPEG_6$-O-hydroxycodone) (Compound C, n=6) 557 mg of HCl salt, 98% pure; α-6-$mPEG_7$-O-oxycodone (aka α-6-$mPEG_7$-O-hydroxycodone) (Compound C, n=7) 695 mg of HCl salt, 94% pure; and α-6-$mPEG_9$-O-oxycodone (aka α-6-$mPEG_9$-O-hydroxycodone) (Compound C, n=9) 435 mg of HCl salt 95% pure. The following compounds, α-6-$mPEG_1$-O-oxycodone (aka α-6-$mPEG_1$-O-hydroxycodone) (Compound C, n=1) 431 mg of HCl salt 99% pure; and α-6-$mPEG_2$-O-oxycodone (aka α-6-$mPEG_2$-O-hydroxycodone) (Compound C, n=2) 454 mg HCl salt, 98% pure, were similarly prepared.

## EXAMPLE 13

### In-Vivo Analgesic Assay: Phenylquinone Writhing

An analgesic assay was used to determine whether exemplary PEG-oligomer-opioid agonist conjugates belonging to the following conjugate series: $mPEG_{2-7,9}$-O-morphine, $mPEG_{3-7,9}$-O-codeine, and $mPEG_{1-4,6,7,9}$-O-hydroxycodone, are effective in reducing and/or preventing visceral pain in mice.

The assay utilized CD-1 male mice (5-8 mice per group), each mouse being approximately 0.020-0.030 kg on the study day. Mice were treated according to standard protocols. Mice were given a single "pretreatment" dose of a compound lacking covalent attachment of a water-soluble, non-peptidic oligomer (i.e., non-PEG oligomer-modified parent

molecule), a corresponding version comprising the compound covalently attached to a water-soluble, non-peptidic oligomer (i.e., the conjugate), or control solution (IV, SC, IP or orally) thirty minutes prior to the administration of the phenylquinone (PQ) solution. Each animal was given an IP injection of an irritant (phenylquinone, PQ) that induces "writhing" which may include: contractions of the abdomen, twisting and turning of the trunk, arching of the back, and the extension of the hindlimbs. Each animal was given an IP injection of PQ (1 mg/kg PQ, 0.1 mL/10 g bodyweight). After the injection, the animals were returned to their observation enclosure and their behavior was observed. Contractions were counted between 35 and 45 minutes after the 'pretreatment". The animals were used once. Each tested article was dosed at a range between 0.1 and 100 mg/kg (n=5-10 animals/dose).

The results are shown in FIG. 2 (mPEG$_{2-7,9}$-O-morphine and control), FIG. 3 (mPEG$_{1-4,6,7,9}$-O-hydroxycodone and control), and FIG. 4 (mPEG$_{3-7,9}$-O-codeine and control). ED50 values are provided in Tables 2 and 3 below.

**Table 2.** $ED_{50}$ values for mPEG$_n$-O-Morphine Series

| | MORPHINE | PEG 2 | PEG 3 | PEG 4 | PEG 5 | PEG 6 | PEG 7 | PEG 9 |
|---|---|---|---|---|---|---|---|---|
| **$ED_{50}$ (mg/kg)** | 0.3693 | 2.512 | 13.58 | 3.281 | 13.4 | n/a | n/a | n/a |

**Table 3.** $ED_{50}$ values for mPEG$_n$-O-HydroxyCodone Series

| | OXYCODONE | PEG 1 | PEG 2 | PEG 3 | PEG 4 | PEG 6 | PEG 7 | PEG 9 |
|---|---|---|---|---|---|---|---|---|
| **$ED_{50}$ (mg/kg)** | 0.6186 | 6.064 | n/a | n/a | 17.31 | n/a | n/a | n/a |

## EXAMPLE 14

### In-Vivo Analgesic Assay: Hot Plate Latency Assay

A hot plate latency analgesic assay was used to determine whether exemplary PEG-oligomer-opioid agonist conjugates belonging to the following conjugate series: mPEG$_{1-5}$-O-morphine, mPEG$_{1-5}$-O-hydroxycodone, and mPEG$_{2-5,9}$-O-codeine, are effective in reducing and/or preventing visceral pain in mice.

The assay utilized CD-1 male mice (10 mice per group), each mouse being approximately 0.028-0.031 kg on the study day. Mice were treated according to standard protocols. Mice were given a single "pretreatment" dose of a compound lacking covalent attachment of a water-soluble, non-peptidic oligomer (unmodified parent molecule), a corresponding version comprising the compound covalently attached to a water-soluble, non-peptidic oligomer (i.e., the conjugate), or control solution (SC) thirty minutes prior to the hot plate test. The hot plate temperature was set at 55 ± 1°C, calibrated with a surface thermometer before commencement of the experiment. Thirty minutes after "pretreatment", each mouse was placed on the hot plate, and latency to lick a hindpaw was recorded to the nearest 0.1 second. If no lick occurred within 30 seconds, the mouse was removed. Immediately after hot plate testing, a temperature probe was inserted 17 mm into the rectum, and body temperature was read to the nearest 0.1°C when the meter stabilized (approximately 10 seconds). The animals were used once. Each tested article was dosed at a range between 0.3 and 30 mg/kg (n=5-10 animals/dose).

Results are shown in FIG. 5 (hydroxycodone series), FIG. 6 (morphine series) and FIG. 7 (codeine). Plots illustrate latency (time to lick hindpaw, in seconds) versus dose of compound administered in mg/kg.

## EXAMPLE 15

### Pharmacokinetics of PEG$_{oligo}$-Opioid Compounds Following Intravenous (IV) and Oral (PO) Dosing in Male Sprague-Dawley Rats - Study Design

One hundred seventy five (175) adult male Sprague-Dawley rats with indwelling jugular vein and carotid artery catheters (JVC/CAC) (Charles River Labs, Hollister, CA) were utilized for the study. There were 3 rats/group. All animals were food fasted overnight. Prior to dosing the rats were weighed, the tails and cage cards were labeled for identification and the doses were calculated. Anesthesia was induced and maintained with 3.0-5.0% isoflurane. The JVC and CAC were externalized, flushed with HEP/saline (10 IU/mL HEP/ mL saline), plugged, and labeled to identify the jugular vein and carotid artery. The predose sample was collected from the JVC. When all of the animals had recovered from anesthesia and the predose samples were processed, the animals for intravenous group were were dosed, intravenously (IV) via the JVC using a 1 mL syringe containing the appropriate test article, the dead volume of the catheter was flushed with 0.9% saline to ensure the animals received the correct dose and oral group animals were treated orally via gavage.

Following a single IV dose, blood samples were collected at 0 (pre-dose collected as described above), 2, 10, 30, 60, 90, 120, and 240 minutes and following oral dose, blood samples were collected 0 (pre-dose collected as described above), 15, 30, 60, 120, 240 and 480 minutes via the carotid artery catheter and processed as stated in the protocol. Following the last collection point, the animals were euthanized.

Bioanalytical analysis of the plasma samples was conducted using LC-MS/MS methods.

Pharmacokinetic Analyses: PK analysis was performed using WinNonlin (Version 5.2, Mountain View, CA-94014). Concentrations in plasma that were below LLOQ were replaced with zeros prior to generating Tables and PK analysis. The following PK parameters were estimated using plasma concentration-time profile of each animal:

| | |
|---|---|
| $C_0$ | Extrapolated concentration to time "zero" |
| $C_{max}$ | Maximum (peak) concentration |
| $AUC_{all}$ | Area under the concentration-time from zero to time of last concentration value |
| $T_{1/2(Z)}$ | Terminal elimination half-life |
| $AUC_{inf}$ | Area under the concentration-time from zero to time infinity |
| $T_{max}$ | Time to reach maximum or peak concentration following administration |
| CL | Total body clearance |
| $V_z$ | Volume of distribution based on terminal phase |
| $V_{ss}$ | Volume of distribution at steady state |
| $MRT_{last}$ | Mean residence time to last observable concentration |
| F | Bioavailability |

Oral bioavailability was estimated using mean dose-normalized AUCall data for the compounds where one of IV or PO groups with only reported data for <n=3/group.

## EXAMPLE 16

### IV and PO Pharmacokinetics of $mPEG_n$-O-Hydroxycodone Conjugates

A pharmacokinetic study was conducted in Sprague-Dawley rats as described in Example 15 above. Compounds administered were $mPEG_n$-O-hydroxycodone conjugates where n=1, 2, 3, 4, 5, 6, 7, and 9, as well as the parent compound, oxycodone. The objective was to determine the pharmacokinetics of the parent compound and its various oligomer conjugates administered both intravenously and orally.

A summary of plasma PK parameters following IV (1 mg/kg) and PO (5 mg/kg) delivery for oxycodone, $mPEG_0$-oxycodone, $mPEG_1$-O-hydroxycodone, $mPEG_2$-O-hydroxycodone, $mPEG_3$-O-hydroxycodone, $mPEG_4$-O-hydroxycodone, $mPEG_5$-O-hydroxycodone, $mPEG_6$-O-hydroxycodone, $mPEG_7$-O-hydroxycodone, and $mPEG_9$-O-hydroxycodone, are shown in the following tables, Tables 4 and 5.

Based on the observed data (Table 4) for IV administration, $mPEG_9$-O-hydroxycodone appeared to achieve higher plasma concentration with a mean $t_{1/2}$ value 3 times that of the corresponding mean $t_{1/2}$ value observed after parent oxycodone was given.

FIG. 8 shows the mean plasma concentration-time profiles for IV-administered mPEGn-O-hydroxycodone compounds as described above, as well as for oxycodone per se, when administered at a concentration of 1.0 mg/kg.

Based on the observed data (Table 5) for oral administration, $mPEG_5$-O-hydroxycodone, $mPEG_6$-O-hydroxycodone, and $mPEG_7$-O-hydroxycodone appeared to achieve higher mean exposure (approximately 3- to 8-fold) in plasma as compared to parent molecule, oxycodone.

FIG. 9 shows the mean plasma concentration-time profiles for the $mPEG_n$-O-hydroxycodone compounds described above, as well as for oxycodone, when administered orally to rats at a concentration of 5.0 mg/kg.

**Table 4.** Comparative PK Parameters of mPEG$_n$-O-hydroxycodone conjugates given intravenously to rats (Mean ± SD)

| PEG-length | $C_{max}$ (ng/mL) | $T_{1/2}$(z) min | $AUC_{all}$ (min.ng /mL) | $AUC_{inf}$ (min.ng /mL) | $MRT_{last}$ min | CL (mL/min/kg) | $V_{ss}$ (L/kg) |
|---|---|---|---|---|---|---|---|
| 0 | 495±56.0 | 47.0±3.99 | 12800±1090 | 13000±1070 | 37.0±1.28 | 77.1±6.26 | 3.17±0.293 |
| 1 | 425±41.3 | 47.2±6.37 | 9890±1320 | 10100±1440 | 38.7±4.54 | 100±13.4 | 4.31±0.222 |
| 2 | 513±48.8 | 44.6±1.80 | 12000±1610 | 12200±1650 | 37.0±2.60 | 83.3±10.8 | 3.36±0.298 |
| 3 | 746±2.08 | 48.5±7.83 | 13800±1050 | 14000±1010 | 32.5±1.92 | 71.7±4.99 | 2.62±0.206 |
| 4 | 537±31.0 | 43.6±3.27 | 11500±783 | 11600±827 | 35.6±2.88 | 86.5±6.36 | 3.34±0.113 |
| 5 | 622±39.7 | 62.1±3.85 | 16900±1800 | 17700±1990 | 46.2±1.86 | 57.0±6.07 | 3.30±0.184 |
| 6 | 445±83.6 | 62.2±5.17 | 12600±2370 | 13100±2390 | 47.7±1.41 | 77.9±14.4 | 4.68±0.938 |
| 7 | 489±26.5 | 87.0±3.25 | 14300±583 | 15800±728 | 54.3±0.372 | 63.3±2.99 | 5.31±0.139 |
| 9 | 955±149 | 143±14.3 | 16600±2190 | 21000±4230 | 52.7±4.04 | 48.9±9.41 | 6.35±0.349 |

**Table 5.** Comparative PK Parameters of mPEG$_n$-O-hydroxycodone conjugates given orally to Sprague Dawley rats (Mean ± SD)

| PEG-length | $C_{max}$ (ng/mL) | $T_{1/2(z)}$ min | $AUC_{all}$ (min.ng /mL) | $AUC_{inf}$ (min.ng /mL) | $T_{max}$* min | $MRT_{last}$ min | F% |
|---|---|---|---|---|---|---|---|
| 0 | 25.5±1.86 | NC | 4520±1660 | NC | 15.0 | 179±17.4 | 7.1 |
| 1 | 14.3±6.43 | 57.7* | 1050±205 | 1150* | 15.0 | 66.8±23.8 | 2.1 |
| 2 | 99.4±47.3 | 48.5±12.0 | 5910±2690 | 5830±2600 | 15.0 | 55.4±14.7 | 9.4 |
| 3 | 44.5±29.4 | 65.6* | 3620±1910 | 4210* | 15.0 | 84.7±17.0 | 5.3 |
| 4 | 55.8±4.69 | 70.3* | 6340±1810 | 5280* | 15.0 | 96.6±33.6 | 11.0 |
| 5 | 178±14.7 | 75.8±1.08 | 32800±2020 | 33300±2090 | 15.0 | 124±4.84 | 37.6 |
| 6 | 171±76.6 | 85.4±7.83 | 35100±10100 | 36200±10200 | 120 | 154±6.46 | 55.3 |
| 7 | 114±38.0 | 115±29.2 | 20400±3670 | 22200±2900 | 120 | 178±6.09 | 28.1 |
| 9 | 27.6±19.6 | 106(n=1) | 7620±4510 | 13500 (n=1) | 120 | 203±43.8 | 9.2 |

*: n=2, NC: Not calculated. Tmax is reported as median value.

To summarize the results, intravenous administration of PEGylated hydroxycodone with varying oligomeric PEG-lengths (PEG1 to PEG9) resulted in variable plasma concentrations and exposures as compared to oxycodone. PEGs with chain lengths 3, 5, 7 and 9 showed higher mean exposure (AUC) while PEG6 showed comparable mean exposure (AUC) and PEGs with chain lengths 1, 2 or 4 showed slightly lower mean exposure (AUC). The compounds having a PEG length greater than 5 showed trends of lower clearance, higher volume of distribution at steady state, increase in elimination half life values, with increasing PEG length.

Oral administration of PEGylated hydroxycodone with varying oligomeric PEG-lengths (PEG1 to PEG9) resulted in improvement in plasma exposure with the exception of hydroxycodone covalently attached to PEG1 and to PEG3. Oral bioavailability was highest for hydroxycodone covalently attached to mPEG6, 55.3%) followed by mPEG5-hydroxycodone and mPEG7-hydroxycodone with 37.6% and 28.1%, respectively. The elimination half-life values showed a trend of increasing with increase in PEG-length.

## EXAMPLE 17

### IV and PO Pharmacokinetics of mPEG$_n$-O-Morphine Conjugates

A pharmacokinetic study was conducted in Sprague-Dawley rats as described in Example 15 above. Compounds administered were mPEG$_n$-O-morphine conjugates where n=1, 2, 3, 4, 5, 6, 7, and 9, as well as the parent compound, morphine. The objective was to determine the pharmacokinetics of the parent compound and its various oligomer conjugates administered both intravenously and orally.

A summary of plasma PK parameters following IV(1 mg/kg) and PO (5 mg/kg) routes for morphine, mPEG$_1$-O-morphine, mPEG$_2$-O-morphine, mPEG$_3$-O-morphine, mPEG$_4$-O-morphine, mPEG$_5$-O-morphine, mPEG$_6$-O-morphine, mPEG$_7$-O-morphine, mPEG$_9$-O-morphine, are shown in Table 6 and Table 7, respectively.

For the intravenous group: FIG. 10 shows the mean plasma concentration-time profiles for the above mPEG$_n$-O-morphine conjugates after 1.0 mg/kg intravenous administration to rats. There appeared to be one outlier datum in each animal that are inconsistent with plasma profiles of mPEG$_2$-O-morphine, and were excluded from the PK analysis.

Based on the observed data (Table 6), mPEG$_9$-O-morphine appeared to achieve higher plasma concentration with a mean $t_{1/2}$ value 4 times that of the corresponding $t_{1/2}$ value observed after parent morphine was given.

**Table 6.** Comparative PK Parameters of mPEG$_n$-O-morphine Conjugates given intravenously to rats

| PEG-Length | $C_{max}$ (ng/mL) | $T_{1/2(z)}$ min | $AUC_{all}$ (min.ng /mL) | $AUC_{inf}$ (min.ng /mL) | $MRT_{last}$ min | CL (mL/min/kg) | $V_{ss}$ (L/kg) |
|---|---|---|---|---|---|---|---|
| 0 | 132 ± 5.86 | 51.1 ± 20.8 | 2730±276 | 2760 ± 218 | 28.5 ± 6.79 | 364 ± 27.5 | 14.9 ± 4.0 |

(continued)

| PEG-Length | $C_{max}$ (ng/mL) | $T_{1/2(z)}$ min | $AUC_{all}$ (min.ng /mL) | $AUC_{inf}$ (min.ng /mL) | $MRT_{last}$ min | CL (mL/min/kg) | $V_{ss}$ (L/kg) |
|---|---|---|---|---|---|---|---|
| 1 | 483 ± 37.1 | 40.0 ± 2.58 | 11400±1230 | 11500 ± 1260 | 29.8 ± 5.05 | 87.8 ± 9.40 | 2.75 ± 0.236 |
| 2 | 378 ± 48.8 | 38.1±8.03 | 7510±106 | 7410±404 | 26.4±5.90 | 135±7.60 | 4.2 ± 0.270 |
| 3 | 483 ± 81.0 | 45.0 ± 2.73 | 12700±1950 | 12900 ± 1990 | 39.3 ± 1.69 | 78.5 ± 11.8 | 3.43 ± 0.616 |
| 4 | 622 ± 72.5 | 52.9 ± 6.50 | 14600±1140 | 15000 ± 1270 | 40.1 ± 0.962 | 67.1 ± 5.58 | 3.17 ± 0.168 |
| 5 | 514 ± 38.6 | 68.4 ±0.826 | 13200±998 | 14000 ± 1050 | 49.7 ± 1.20 | 71.6 ± 5.17 | 4.74 ± 0.347 |
| 6 | 805 ± 30.6 | 93.7 ± 17.1 | 19000±1430 | 21600 ± 2060 | 56.2 ± 3.84 | 46.6 ± 4.67 | 4.39 ± 0.630 |
| 7 | 1110 ± 123 | 111 ± 32.9 | 18100±956 | 21200 ± 1990 | 49.6 ± 5.20 | 47.4 ± 4.21 | 4.76 ± 0.997 |
| 9 | 1840 ± 123 | 204 ± 28.3 | 23300±1460 | 29000 ± 3240 | 34.2 ± 2.72 | 34.7 ± 3.64 | 4.52 ± 0.473 |

For the oral group, FIG. 11 shows the mean plasma concentration-time profiles for the above described $mPEG_n$-O-morphine conjugates after the oral administration (5.0 mg/kg) to rats.

Based on the observed data (Table 7), $mPEG_4$-O-morphine appeared to achieve highest plasma concentrations among the conjugates tested as compared to parent molecule, morphine.

**Table 7.** Comparative PK Parameters of $mPEG_n$-O-morphine conjugates given orally to Sprague Dawley rats (Mean ± SD)

| PEG-Length | $C_{max}$ (ng/mL) | $T_{1/2(z)}$ min | $AUC_{all}$ (min.ng /mL) | $AUC_{inf}$ (min.ng /mL) | $T_{max}$ min | $MRT_{last}$ min | F% |
|---|---|---|---|---|---|---|---|
| 0 | 29.8 ± 7.78 | 144 ± 32.1 | 5510 ± 667 | 7230 ± 897 | 15.0 | 194 ± 22.0 | 40.4¥ |
| 2 | 3.84* | 104* | 448* | 778* | 15.0* | 60.7* | 0.15 |
| 3 | 30.3 ± 4.42 | 377* | 4250± 2140 | 8370* | 15.0 | 151 ± 69.4 | 9.0 |
| 4 | 87.1 ± 53.6 | 191 ± 104 | 15600 ± 7690 | 18200±10300 | 30.0 | 149 ± 26.7 | 22.1 |
| 5 | 35.6 ± 19.8 | 247* | 9190 ± 5650 | 17400* | 120 | 205 ± 26.2 | 13.9 |
| 6 | 42.8 ± 31.2 | 121* | 8290 ± 4970 | 10800* | 120 | 177 ± 29.4 | 8.7 |
| 7 | 9.38 ± 0.883 | 236* | 2210 ± 221 | 2720* | 60.0 | 187 ± 32.0 | 2.4 |
| 9 | 7.15 ± 3.34 | 363* | 1360 ± 311 | 2270* | 15.0 | 166 ± 26.0 | 1.2 |
| No PK parameters were not reported for $mPEG_1$-morphine because all the concentrations were <LLOQ. *n=2. | | | | | | | |

In summary, for the IV data, administration of oligomeric PEGylated morphine with varying PEG-lengths (PEG1 to PEG9) resulted in higher plasma concentrations and exposure (AUC) as compared to morphine per se. There was

a clear trend of increase in mean AUC with increase in PEG-length of 5 onwards, with 10-fold higher mean AUC for the PEG9-morphine compound as compared to non-conjugated morphine. The mean half-life and mean residence time also increased with increase in PEG-length. The lower mean clearance values were consistent with observed higher mean AUC values.

Mean volume of distribution estimated for steady state, immediately decreased by 5-fold with the introduction of single PEG, and reached a constant value at PEG-length 5. Overall, PEGylation appeared to increase the elimination $t_{1/2}$ and lower the tissue distribution of morphine.

Based upon the oral data, administration of PEGylated morphine conjugates with varying PEG-lengths (PEG1 to PEG9) resulted in a reduction in oral bioavailability compared to morphine. The reduction in bioavailability appeared to be related to the absorption component rather than metabolism component for these PEG-conjugates. Among the PEG-conjugates, the conjugate with PEG-length 4 showed maximum F-value (22.1%) while conjugates with shorter or longer PEG-length showed a clear trend of loss in absorption.

In this study, morphine F% value was 3-fold higher than literature value of 15% at 7.5 mg/kg (J. Pharmacokinet. Biopharm. 1978, 6:505-19). The reasons for this higher exposure are not known.

## EXAMPLE 18

### IV and PO Pharmacokinetics of $mPEG_n$-O-Codeine Conjugates

A pharmacokinetic study was conducted in Sprague-Dawley rats as described in Example 15 above. Compounds administered were $mPEG_n$-O-codeine conjugates where n=1, 2, 3, 4, 5, 6, 7, and 9, as well as the parent compound, codeine (n=0). The objective was to determine the pharmacokinetics of the parent compound, i.e., codeine, and its various oligomer conjugates administered both intravenously and orally.

A summary of plasma PK parameters following IV(1 mg/kg) and PO (5 mg/kg) routes for codeine, $mPEG_1$-O-codeine, $mPEG_2$-O-codeine, $mPEG_3$-O-codeine, $mPEG_4$-O-codeine, $mPEG_5$-O-codeine, $mPEG_6$-O-codeine, $mPEG_7$-O-codeine, $mPEG_9$-O-codeine, are shown in Table 8 and Table 9, respectively.

For the IV group: FIG. 12 shows the mean plasma-concentration-time profiles for parent molecule, codeine, as well as for the $mPEG_n$-O-codeine conjugates described above, after intravenous administration.

Based on the observed data (Table 8), $mPEG_6$-O-codeine appeared to achieve higher plasma concentrations among the tested conjugates with a mean $t_{1/2}$ value approximately 2.5 times that of the corresponding $t_{1/2}$ value observed following administration of the parent molecule, codeine.

**Table 8.** Comparative PK Parameters of Codeine and its Oligomeric PEG Conjugates Admnistered Intravenously to Rats

| PEG-Length | $C_{max}$ (ng/mL) | $T_{1/2(z)}$ min | $AUC_{all}$ (min.ng /mL) | $AUC_{inf}$ (min.ng /mL) | $MRT_{last}$ min | CL (mL/min/kg) | $V_{ss}$ (L/kg) |
|---|---|---|---|---|---|---|---|
| 0 | 469 ± 20.4 | 42.1±3.15 | 11000 ± 1600 | 11400±2070 | 40.2 ± 9.08 | 89.7±15.3 | 4.14±0.700 |
| 1 | 723 ± 31.2 | 42.1 ± 4.84 | 15500±2020 | 15700 ± 2130 | 32.2 ± 4.59 | 64.6 ± 8.75 | 2.22 ±0.899 |
| 2 | 685 ± 41.0 | 35.3 ± 2.78 | 14500±1590 | 14600 ± 1590 | 31.5 ± 2.96 | 69.0 ±7.57 | 2.25 ± 0.166 |
| 3 | 732 ± 27.1 | 39.4 ± 1.49 | 17300±1520 | 17400 ± 1550 | 33.8 ± 2.40 | 57.7 ± 4.89 | 2.07 ± 0.127 |
| 4 | 746 ± 70.0 | 57.1 ± 43.8 | 15200±2160 | 15400 ± 2240 | 27.5 ± 4.55 | 65.9 ± 10.4 | 2.30 ± 0.720 |
| 5 | 533 ± 38.9 | 42.7 ± 3.56 | 11500±878 | 11700 ± 913 | 31.8 ± 1.53 | 86.2 ± 7.04 | 2.95 ± 0.157 |
| 6 | 1780 ± 149 | 58.0 ± 4.79 | 45600±2020 | 47100±2000 | 41.7 ± 3.08 | 21.3 ± 0.876 | 1.08 ± 0.143 |
| 7 | 443 ± 43.3 | 74.5 ± 5.76 | 12700±481 | 13700 ± 320 | 50.7 ± 2.07 | 73.1 ± 1.73 | 5.20 ± 0.596 |

(continued)

| PEG-Length | $C_{max}$ (ng/mL) | $T_{1/2(z)}$ min | $AUC_{all}$ (min.ng /mL) | $AUC_{inf}$ (min.ng /mL) | $MRT_{last}$ min | CL (mL/min/kg) | $V_{ss}$ (L/kg) |
|---|---|---|---|---|---|---|---|
| 9 | 730 ± 68.0 | 109 ± 1.80 | 17800±2310 | 20800 ± 2840 | 57.2 ± 2.46 | 48.6 ± 6.74 | 5.18 ± 0.538 |
| Tmax is reported as median value. *: n=2. | | | | | | | |

For the oral group, FIG. 13 shows the mean plasma concentration-time profiles for parent molecule, codeine, versus $mPEG_n$-codeine conjugates after oral administration to rats (5.0 mg/kg).

Based on the observed data (Table 9), the PEG-6 compound, $mPEG_6$-codeine, appeared to achieve highest plasma concentrations (52 times higher mean AUCall) among the tested conjugates as parent molecule, codeine.

**Table 9.** Comparative PK Parameters of Codeine and Various $mPEG_n$-Codeine Conjugates given Orally to Sprague Dawley Rats (Mean ± SD)

| PEG-Length | $C_{max}$ (ng/mL) | $T_{1/2(z)}$ min | $AUC_{all}$ (min.ng /mL) | $AUC_{inf}$ (min.ng /mL) | $T_{max}$ min | $MRT_{last}$ min | F% |
|---|---|---|---|---|---|---|---|
| 0 | 6.24 ± 2.51 | 80.8# | 328 ± 216 | 431# | 15.0 | 33.2 ± 12.9 | 0.60 |
| 2 | 3.47 ± 0.606 | 97.6 ± 28.4 | 351±195 | 419 ± 226 | 15.0 | 62.0 ± 27.4 | 0.57 |
| 3 | 25.0 ± 6.59 | 125 ± 64.6 | 1920±245 | 2080 ± 498 | 15.0 | 71.0 ± 9.16 | 2.39 |
| 4 | 31.1 ± 13.1 | 118 ± 60.0 | 2530±682 | 2670 ± 870 | 15.0 | 83.8 ± 22.5 | 3.47 |
| 5 | 48.7 ± 10.8 | 125 ± 63.7 | 5510±963 | 5890 ±1470 | 15.0 | 108 ± 35.4 | 10.1 |
| 6 | 617 ± 56.4 | 126 ± 54.1 | 70500±12300 | 74500±10000 | 15.0 | 119 ± 11.1 | 31.6 |
| 7 | 76.6 ± 12.8 | 97.6* | 17100±4220 | 16000* | 120 | 171 ± 21.7 | 26.9 |
| 9 | 31.5 ± 8.43 | 143* | 7320±3330 | 6840* | 15.0 | 179 ± 21.6 | 8.22 |
| No PK parameters were not reported for NKT-10479 because the concentrations were LLOQ. #: n=1, *: n=2. $T_{max}$ is reported as median value. | | | | | | | |

In summary, for the IV data, PEGylation of codeine with varying oligomeric PEG-lengths (PEG1 to PEG9) improved exposure (mean AUC) only slightly and moderate improvement (approximately 4-fold) was observed for the PEG-6 conjugate. Both clearance and volume of distribution decreased for this PEG-conjugate by 4-fold. Conjugates with PEG-lengths 7 and 9 showed longer mean $t_{1/2}$ values, however, mean clearance and mean volume of distribution (Vss) were decreased for both for both the PEG7- and PEG9-codeine conjugates.

For the oral data, oral bioavailability for codeine is very low (F=0.52%). Oral bioavailability appeared to increase with increase in PEG-length from 2 onwards, reaching maximum with 32% bioavailability for the codeine conjugate with PEG-length 6, decreasing thereafter. In general, mean $t_{1/2}$ and mean residence values increased with PEG-length. There was no difference in time to reach peak concentrations (Tmax = 15 min) amongst all the compounds tested, suggesting that absorption was rapid and the absorption rate was not altered.

## EXAMPLE 19

### In-Vitro Binding of $mPEG_n$-O-Opioid Conjugates to Opioid Receptors

The binding affinities of the various PEG-opioid conjugates ($mPEG_n$-O-morphine, $mPEG_n$-O-codeine, and $mPEG_n$-O-hydroxycodone) were measured *in vitro* in membrane preparations prepared from CHO cells that heterologously express the cloned human mu, kappa or delta opioid receptors in a manner similar to that described in Example 4. Radioligand displacement was measured using scintillation proximity assays (SPA).

Briefly, serial dilutions of the test compounds were placed in a 96-well plate to which were added SPA beads, membrane and radioligand. The assay conditions for each opioid receptor subtype are described in Table 10 below. The plates were incubated for 8 hours-overnight at room temperature, spun at 1000 rpm to pellet the SPA beads, and radioactivity was measured using the TopCount® microplate Scintillation counter. Specific binding at each concentration of test compound was calculated by subtracting the non-specific binding measured in the presence of excess cold ligand. $IC_{50}$ values were obtained by non-linear regression of specific binding versus concentration curves and Ki values were calculated using Kd values that were experimentally pre-determined for each lot of membrane preparations.

**Table 10.** Assay conditions for opioid receptor binding assays.

| EXPERIMENTAL VARIABLE | MU OPIOID RECEPTOR | KAPPA OPIOID RECEPTOR | DELTA OPIOID RECEPTOR |
|---|---|---|---|
| SPA beads | PVT-WGA PEI Type A (GE Healthcare, Cat. # RPNQ0003) | PVT-WGA (GE Healthcare, Cat. #RPNQ0001) | PVT-WGA PEI Type B (GE Healthcare, Cat. #RPNQ0004) |
| Radioligand; Concentration | DAMGO, [Tyrosyl-3,5-3H (N)]- (Perkin Elmer, Cat. # NET-902); 6 nM | U-69,593, [Phenyl-3,4-3H]- (Perkin Elmer, Cat. #NET-952); 10 nM | Naltrindole, [5',7'-3H] -(Perkin Elmer, Cat. #NET-1065); 3 nM |
| Non-specific binding control | CTAP | *nor*-Binaltorphimine (*nor*-BNI) | SNC80 |
| Buffer | 50 mM Tris-HCl, pH 7.5<br>5 mM MgCl2 ;<br>1 mM EDTA | 50 mM Tris-HCl, pH 7.5<br>5 mM MgC12 | 50 mM Tris-HCl, pH 7.5<br>5 mM MgC12 |
| Receptor and source | Recombinant human mu opioid receptor expressed in CHO-K1 host cell membranes (Perkin Elmer, Cat. #ES-542-M) | Recombinant human kappa opioid receptor expressed in Chem-1 host cell membranes (Millipore, Cat. #HTS095M) | Recombinant human delta opioid receptor expressed in Chem-1 host cell membranes (Millipore, Cat. #HTS100M). |

The binding affinities of the oligomeric PEG conjugates of morphine, codeine and hydroxycodone are shown in Table 11. Overall, all of the conjugates displayed measurable binding to the mu-opioid receptor, consistent with the known pharmacology of the parent molecules. For a given PEG size, the rank order of mu-opioid binding affinity was PEG-morphine > PEG-hydroxycodone > PEG-codeine. Increasing PEG size resulted in a progressive decrease in the binding affinity of all PEG conjugates to the mu opioid receptor compared to unconjugated parent molecule. However, the PEG-morphine conjugates still retained a high binding affinity that was within 15X that of parent morphine. The mu-opioid binding affinities of PEG-hydroxycodones were 20-50 fold lower than those of the PEG-morphine conjugates. Codeine and its PEG conjugates bound with very low affinity to the mu opioid receptor. PEG-morphine conjugates also bound to the kappa and delta opioid receptors; the rank order of selectivity was mu>kappa>delta. Binding affinities of codeine and hydroxycodone conjugates to the kappa and delta opioid receptors were significantly lower than that at the mu-opioid receptor.

**Table 11.** Binding affinities of the PEG-opioid conjugates to opioid receptors.

| COMPOUND | KI (NM) | | |
|---|---|---|---|
| | Mu opioid receptor | Kappa opioid receptor | Delta opioid receptor |
| Morphine | 8.44 | 118.38 | 4,297 |
| $\alpha$-6-mPEG$_1$-O-Morphine | 15.72 | 444.54 | 2,723 |
| $\alpha$-6-mPEG$_2$-O-Morphine | 21.97 | 404.33 | 2,601 |
| $\alpha$-6-mPEG$_3$-O-Morphine | 50.66 | 575.98 | 6,176 |
| $\alpha$-6-mPEG$_4$-O-Morphine | 23.11 | 438.88 | 3,358 |
| $\alpha$-6-mPEG$_5$-O-Morphine | 39.40 | 557.54 | 2,763 |
| $\alpha$-6-mPEG$_6$-O-Morphine | 72.98 | 773.56 | 2,595 |

(continued)

| COMPOUND | KI (NM) | | |
|---|---|---|---|
| | Mu opioid receptor | Kappa opioid receptor | Delta opioid receptor |
| α-6-mPEG$_7$-O-Morphine | 56.86 | 669.56 | 2,587 |
| α-6-mPEG$_9$-O-Morphine | 111.05 | 1253.71 | 5,783 |
| | | | |
| Oxycodone | 133.48 | N/A | N/A |
| α-6-mPEG$_1$-O-Hydroxycodone | 653.90 | N/A | N/A |
| α-6-mPEG$_2$-O-Hydroxycodone | 631.76 | N/A | N/A |
| α-6-mPEG$_3$-O-Hydroxycodone | 775.19 | N/A | N/A |
| α-6-mPEG$_4$-O-Hydroxycodone | 892.70 | N/A | N/A |
| α-6-mPEG$_5$-O-Hydroxycodone | 1862.14 | N/A | N/A |
| α-6-mPEG$_6$-O-Hydroxycodone | 1898.30 | N/A | N/A |
| α-6-mPEG$_7$-O-Hydroxycodone | 1607.19 | N/A | N/A |
| α-6-mPEG$_9$-O-Hydroxycodone | 3616.60 | N/A | N/A |
| | | | |
| Codeine | 1,953 | 28,067 | N/A |
| α-6-mPEG$_1$-O-Codeine | 1821.51 | 54669.89 | N/A |
| α-6-mPEG$_2$-O-Codeine | 1383.07 | 22603.05 | N/A |
| α-6-mPEG$_3$-O-Codeine | 4260.21 | 36539.78 | N/A |
| α-6-mPEG$_4$-O-Codeine | 2891.36 | 96978.61 | N/A |
| α-6-mPEG$_5$-O-Codeine | 2427.13 | 59138.22 | N/A |
| α-6-mPEG$_6$-O-Codeine | 14202.77 | >160,000 | N/A |
| α-6-mPEG$_7$-O-Codeine | 9963.93 | 108317.50 | N/A |
| α-6-mPEG$_9$-O-Codeine | 9975.84 | 72246.23 | N/A |

N/A indicates that Ki values could not be calculated since a 50% inhibition of binding was not achieved at the highest concentration of compound tested.

## EXAMPLE 20

### In-Vitro Efficacy of mPEG$_n$-O-Opioid Conjugates to Inhibit cAMP Formation

The efficacy of the various PEG-opioid conjugates was measured by their ability to inhibit cAMP formation following receptor activation in a manner similar to that described in Example 5. Studies were conducted in CHO cells heterologously expressing the cloned human mu, kappa or delta opioid receptors. cAMP was measured using a cAMP HiRange homogenous time-resolved fluorescence assay (HTRF Assay), that is based on a competitive immunoassay principle (Cisbio, Cat.#62AM6PEC).

Briefly, suspensions of cells expressing either the mu, kappa or delta opioid receptors were prepared in buffer containing 0.5 mM isobutyl-methyl xanthine (IBMX). Cells were incubated with varying concentrations of PEG-opioid conjugates and 3 μM forskolin for 30 minutes at room temperature. cAMP was detected following a two-step assay protocol per the manufacturer's instructions and time resolved fluorescence was measured with the following settings: 330 nm excitation; 620 nm and 665 nm emission; 380 nm dichroic mirror. The 665nm/620nm ratio is expressed as Delta F% and test compound-related data is expressed as a percentage of average maximum response in wells without forskolin. $EC_{50}$ values were calculated for each compound from a sigmoidal dose-response plot of concentrations versus maximum response. To determine if the compounds behaved as full or partial agonists in the system, the maximal

response at the highest tested concentrations of compounds were compared to that produced by a known full agonist.

The $EC_{50}$ values for inhibition of cAMP formation in whole cells are shown in Table 12. Oligomeric PEG conjugates of morphine, codeine and hydroxycodone were full agonists at the mu opioid receptor, albeit with varying efficacies. Morphine and its conjugates were the most potent of the three series of opioids tested, while the PEG hydroxycodone and PEG codeine conjugates displayed significantly lower efficacies. A progressive decrease in the efficacy of the PEG-morphine conjugates was observed with increasing PEG size, however the conjugates retained mu-agonist potency to within 40X of parent. In contrast to the effect at the mu opioid receptor, morphine and PEG-morphine conjugates behaved as weak partial agonists at the kappa opioid receptor, producing 47-87% of the maximal possible response. $EC_{50}$ values could not be calculated for the codeine and hydroxycodone conjugates at the kappa and delta opioid receptors since complete dose-response curves could not be generated with the range of concentrations tested (upto 500 $\mu$M).

Overall, the results of the receptor binding and functional activity indicate that the PEG-opioids are mu agonists *in vitro.*

**Table 12.** *In vitro* efficacies of PEG-opioid conjugates

| COMPOUND | Mu OPIOID RECEPTOR | | KAPPA OPIOID RECEPTOR | | DELTA OPIOID RECEPTOR |
|---|---|---|---|---|---|
| | $EC_{50}$, nM | % max effect | $EC_{50}$, nM | % max effect | |
| Morphine | 28.5 | 102 | 624 | 69 | N/A |
| $\alpha$-6-mPEG$_1$-O-Morphine | 85.0 | 91 | 1,189 | 81 | N/A |
| $\alpha$-6-mPEG$_2$-O-Morphine | 93.3 | 91 | 641 | 87 | N/A |
| $\alpha$-6-mPEG$_3$-O-Morphine | 270 | 100 | 4,198 | 82 | N/A |
| $\alpha$-6-mPEG$_4$-O-Morphine | 128 | 100 | 3,092 | 77 | N/A |
| $\alpha$-6-mPEG$_5$-O-Morphine | 157 | 95 | 2,295 | 71 | N/A |
| $\alpha$-6-mPEG$_6$-O-Morphine | 415 | 98 | 3,933 | 62 | N/A |
| $\alpha$-6-mPEG$_7$-O-Morphine | 508 | 90 | 4,237 | 57 | N/A |
| $\alpha$-6-mPEG$_9$-O-Morphine | 1,061 | 87 | 4,417 | 47 | N/A |
| | | | | | |
| Oxycodone | 478 | 95 | N/A | N/A | N/A |
| Hydroxycodone | 3,162 | | N/A | N/A | |
| $\alpha$-6-mPEG$_1$-O-Hydroxycodone | 3,841 | 102 | N/A | N/A | N/A |
| $\alpha$-6-mPEG$_2$-O-Hydroxycodone | 5,005 | 101 | N/A | N/A | N/A |
| $\alpha$-6-mPEG$_3$-O-Hydroxycodone | 2,827 | 108 | N/A | N/A | N/A |
| $\alpha$-6-mPEG$_4$-O-Hydroxycodone | 3,715 | 109 | N/A | N/A | N/A |
| $\alpha$-6-mPEG$_5$-O-Hydroxycodone | 5,037 | 108 | N/A | N/A | N/A |
| $\alpha$-6-mPEG$_6$-O-Hydroxycodone | 12,519 | 102 | N/A | N/A | N/A |
| $\alpha$-6-mPEG$_7$-O-Hydroxycodone | 7,448 | 101 | N/A | N/A | N/A |
| $\alpha$-6-mPEG$_9$-O-Hydroxycodone | 17,948 | 95 | N/A | N/A | N/A |

(continued)

| COMPOUND | Mu OPIOID RECEPTOR | | KAPPA OPIOID RECEPTOR | | DELTA OPIOID RECEPTOR |
|---|---|---|---|---|---|
| | $EC_{50}$, nM | % max effect | $EC_{50}$, nM | % max effect | |
| | | | | | |
| Codeine | 10,418 | 81 | N/A | 3 | N/A |
| α-6-mPEG$_1$-O-Codeine | 8,574 | 80 | N/A | 51 | N/A |
| α-6-mPEG$_2$-O-Codeine | 5,145 | 75 | 40,103 | 59 | N/A |
| α-6-mPEG$_3$-O-Codeine | 19,740 | 91 | N/A | 49 | N/A |
| α-6-mPEG$_4$-O-Codeine | 22,083 | 99 | N/A | 61 | N/A |
| α-6-mPEG$_5$-O-Codeine | 23,235 | 95 | N/A | 60 | N/A |
| α-6-mPEG$_6$-O-Codeine | 97,381 | 80 | N/A | 21 | N/A |
| α-6-mPEG$_7$-O-Codeine | 44,729 | 75 | N/A | 48 | N/A |
| α-6-mPEG$_9$-O-Codeine | 48,242 | 80 | N/A | 61 | N/A |

## EXAMPLE 21

### Brain:Plasma Ratios of mPEG$_n$-O-Opioid Conjugates

The ability of oligomeric mPEG-O-morphine, mPEG-O-codeine and mPEG-O-hydroxycodone conjugates to cross the blood brain barrier (BBB) and enter the CNS (central nervous system) was assessed by measuring the brain:plasma ratio in rats subsequent to IV administration.

Briefly, groups of 3 rats were injected intravenously (i.v) with 5 mg/kg each of morphine, mPEG$_n$-O-morphine conjugate, codeine and m-PEG$_n$-O-codeine conjugates. PEG-2,3 and 4-oxycodone conjugates were administered at 10mg/kg i.v. and oxycodone and the other PEG sizes of oxycodone conjugates were administed at 1 mg/kg (i.v). The doses of the oxycodone conjugates had to be adjusted to allow for the detection of sufficient concentrations in brain tissue. Atenolol, which does not cross the BBB, was used as a measure of vascular contamination of the brain tissue and was administered at a concentration of 10 mg/kg to a separate group of rats. An hour following injection, the animals were sacrificed and plasma and the brain were collected and frozen immediately. Following tissue and plasma extractions, concentrations of the compounds in brain and plasma were measured using LC-MS/MS. The brain:plasma ratio was calculated as the ratio of measured concentrations in the brain and plasma. The results are shown in FIGS. 16A-C.

FIGs 14A, 16B, and 16C show the brain:plasma ratios of various oligomeric mPEG$_n$-O-morphine, mPEG$_n$-O-codeine, and PEG$_n$-O-hydroxycodone conjugates, respectively. The brain:plasma ratio of atenolol is shown in each figure to provide a basis for comparison. PEG-conjugation results in a decrease in the brain:plasma ratio of all conjugates compared to their respective unconjugated parent molecule, which in the case of hydroxycodone is oxycodone. Only PEG-1-morphine displayed a greater brain:plasma ratio than its parent, morphine.

## EXAMPLE 22

### Time-Course of Brain and Plasma Concentrations of Various Exemplary mPEG$_n$-O-Opioid Conjugates

Experiments were conducted to determine the concentrations of various oligomeric PEG-opioid conjugates in brain and plasma over time following IV administration.

The experimental methodology and concentrations used were the same as those used for the single time point experiments described in Example 21, however, the brains and plasma were harvested at various differing time points.

All PEG-hydroxycodone conjugates were administered at 10 mg/kg iv, while the oxycodone parent was administered at 1 mg/kg iv. The data for the brain and plasma concentrations versus time for the various PEG-opioid conjugates administered is shown in FIGS. 15A-H (morphine series), FIGS. 16A-H (codeine series), and FIGS. 17A-H (oxycodone/hydroxycodone series).

The data demonstrate that the maximal increase in brain concentrations for all parent molecules and oligomeric

PEG-conjugates occurs at the earliest time point, i.e., 10 minutes following iv injection. PEG conjugation results in a significant reduction in the brain concentrations and with the larger PEG conjugates (≥ PEG-4), the brain concentrations remain relatively low and steady over time.

The present invention can also be described as set out in the following numbered clauses.

1. A compound of the formula:

OP-X-POLY

wherein OP is an opioid compound, X is a linker, and POLY is a small water-soluble oligomer.

2. The compound according to clause 1 wherein OP is selected from the group consisting of fentanyl, hydromorphone, methadone, morphine, codeine, oxycodone, and oxymorphone.

3. The compound according to clause 1 or clause 2 wherein X is a stable linker.

4. The compound according to clause 3 wherein POLY is a small PEG moiety.

5. The compound according to clause 4 wherein the PEG moiety consists of 1-5 ethylene glycol subunits.

6. The compound according to clause 4 wherein the PEG moiety consists of 1-3 ethylene glycol subunits.

7. The compound according to clause 3 having a logP of about 1.0 to about 3.5.

8. The compound according to clause 3 wherein the compound has a logP about 0.5 units more negative than that of OP.

9. The compound according to clause 3 wherein OP-X-POLY binds to an OP target receptor that is the same target receptor to which OP in its unconjugated form binds.

10. The compound according to clause 9 which exhibits a less than about a 10-fold loss of affinity of OP-X-POLY for the OP target receptor relative to the affinity of OP to its target receptor.

11. The compound according to clause 9 wherein the reduction in affinity of OP-X-POLY for the OP target receptor is no greater than 20% relative to the affinity of OP to its target receptor.

12. The compound according to clause 3 wherein OP-X-POLY retains at least 10% bioactivity relative to the bioactivity of unconjugated OP.

13. The compound according to clause 12 wherein OP-X-POLY retains at least 50% bioactivity relative to the bioactivity of unconjugated OP.

14. The compound according to clause 3 wherein OP-X-POLY exhibits less than about a 10-fold decrease in bioactivity relative to the bioactivity of unconjugated OP.

15. The compound according to clause 1 wherein the molecular weight of the compound is less than 1000 Daltons.

16. The compound according to clause 1 wherein the molecular weight of X-POLY is less than 1000 Daltons.

17. The compound according to any one or more of the foregoing clauses, having a blood-brain barrier crossing rate that is less than the rate of blood-brain barrier crossing of OP alone.

18. The compound according to clause 17, having a blood-brain barrier crossing rate that is at least 50% less than the rate of OP alone.

19. The compound according to clause 17, having a blood-brain barrier crossing rate that is at least 75% less than the rate of OP alone.

20. The compound of clause 3, having a blood-brain barrier crossing rate that is at least 10-fold less than the blood-brain barrier crossing rate of OP alone.

21. The compound of clause 1 or 2, wherein X is a physiologically cleavable linker.

22. The compound according to clause 21 wherein POLY is a small PEG moiety consisting of at least 6 polyethylene glycol subunits.

23. The compound according to clause 21 wherein OP-X-POLY exhibits greater than about a 10-fold decrease in bioactivity relative to unconjugated OP.

24. The compound of clause 21 wherein OP-X-POLY retains less than 5% of the bioactivity of unconjugated OP.

25. The compound of clause 24 wherein OP-X-POLY retains less than 2% of the bioactivity of unconjugated OP.

26. The compound of clause 21, having at least about a 10-fold loss of affinity for its target receptor relative to the affinity of unconjugated OP for the same OP target receptor.

27. A composition comprising the compound of any one of clauses 1-26 and a pharmaceutically acceptable excipient or carrier.

28. A method of treating a patient in need of opioid therapy comprising administering an effective amount of a compound of any one of clauses 1-26.

29. A method of reducing the abuse potential of an opioid compound comprising conjugating the compound to a small water-soluble oligomer to form a compound of any one of clauses 1-26.

30. A method of reducing the addictive properties of an opioid agonist comprising conjugating the opioid agonist to a small water-soluble oligomer to form a compound of any one of clauses 1-26.

31. A method of reducing, but not substantially eliminating, the rate of crossing the blood brain barrier of an opioid compound comprising conjugating the compound to a small water-soluble oligomer.

32. A prodrug comprising a mu, kappa, or delta opioid agonist reversibly attached via a covalent bond to a releasable moiety, wherein a given molar amount of the prodrug administered to a patient exhibits a rate of accumulation and a $C_{max}$ of the mu, kappa, or delta opioid agonist in the central nervous system in the mammal that is less than the rate of accumulation and the $C_{max}$ of an equal molar amount of the mu, kappa, or delta opioid agonist had the mu, kappa, or delta opioid agonist not been administered as part of a prodrug.

33. Use of a compound of any one of clauses 1-26 for treating a patient in need of opioid therapy.

34. Use of a compound of any one of clauses 1-26 for the manufacture of a medicament for reducing the abuse potential of the opioid compound, OP.

35. Use of a prodrug comprising a mu, kappa, or delta opioid agonist reversibly attached via a covalent bond to a releasable moiety for achieving a rate of accumulation and a $C_{max}$ of the mu, kappa, or delta opioid agonist in the central nervous system of a mammal to whom a given molar amount of prodrug is administered that is less than the rate of accumulation and the $C_{max}$ of an equal molar amount of the mu, kappa, or delta opioid agonist when administered to the mammal in non-prodrug form.

## Claims

**1.** A compound having a formula:

OP-X-POLY

wherein OP is an opioid compound, X is a linker, POLY is a small water-soluble oligomer, and OP-X-POLY is

selected from:

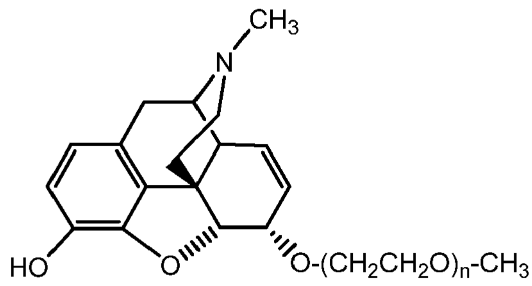

Compound A

and

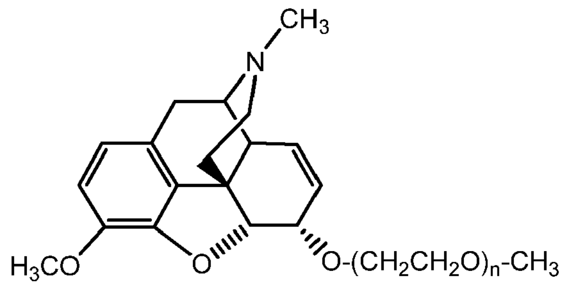

Compound B,

wherein n is an integer from 1 to 9, and salt forms thereof.

**2.** The compound of claim 1, wherein n is 1.

**3.** The compound of claim 1, wherein n is 2.

**4.** The compound of claim 1, wherein n is 3.

**5.** The compound of claim 1, wherein n is 4.

**6.** The compound of claim 1, wherein n is 5.

**7.** The compound of claim 1, wherein n is 6.

**8.** The compound of claim 1, wherein n is 7.

**9.** The compound of claim 1, wherein n is 8.

**10.** The compound of claim 1, wherein n is 9.

**11.** A composition comprising the compound of any one of claims 1-10 and a pharmaceutically acceptable excipient or carrier for use in a method of reducing or preventing pain.

**12.** The composition of claim 11for use in the treatment of a patient in need of opioid therapy.

**13.** A compound of claim 1, wherein OP-X-POLY (i) binds to an OP target receptor that is the same target receptor to which OP in its unconjugated form binds, and (ii) exhibits a less than a 10-fold loss of affinity for the OP target

receptor relative to the affinity of OP to its target receptor.

**14.** A compound according to claim 1 wherein OP-X-POLY retains at least 10% bioactivity relative to the bioactivity of unconjugated OP.

**15.** A compound of claim 1, having a blood-brain barrier crossing rate that is at least 10-fold less than the blood-brain barrier crossing rate of OP alone.

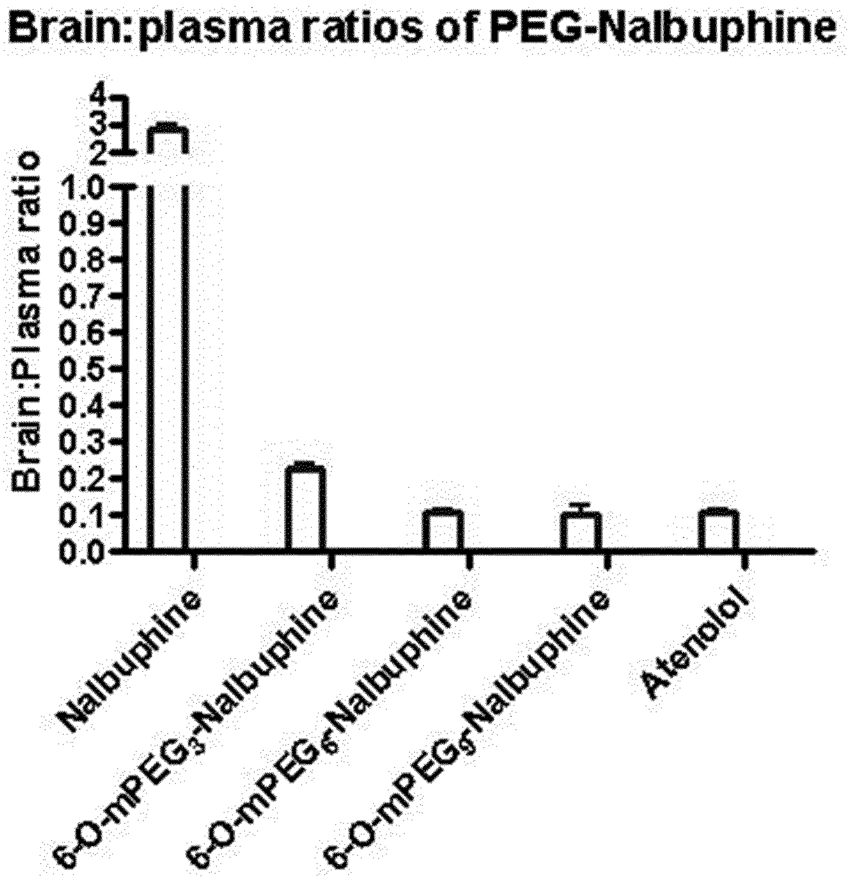


| Molecule | Brain:plasma ratios |
|---|---|
| Nalbuphine | 2.86 |
| 6-O-mPEG$_3$-Nalbuphine | 0.23 |
| 6-O-mPEG$_6$-Nalbuphine | 0.11 |
| 6-O-mPEG$_9$-Nalbuphine | 0.10 |
| Atenolol | 0.11 |

FIG. 1

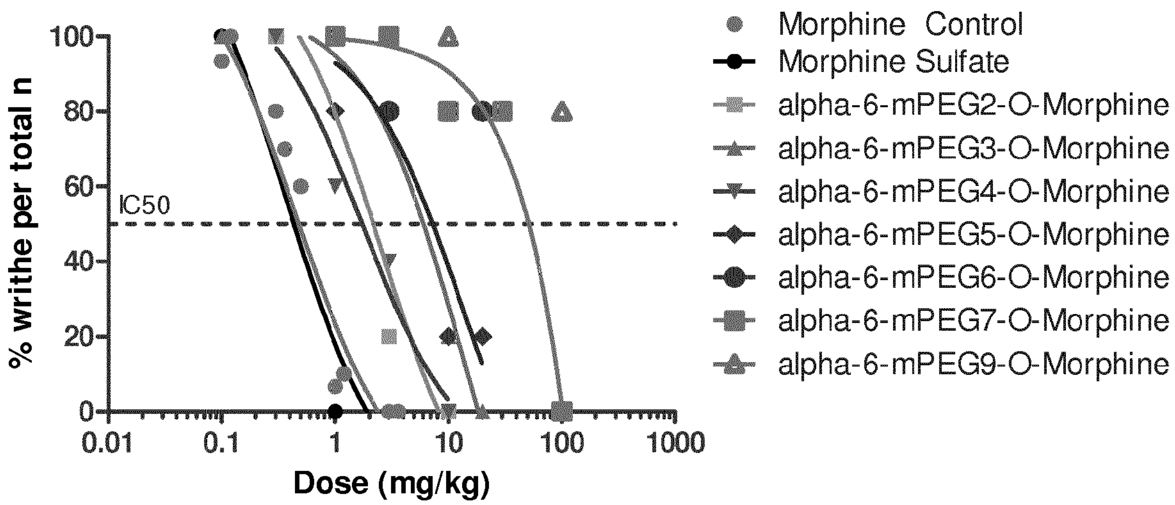


FIG. 2

Hydroxycodone Series Phenylquinone Writhing

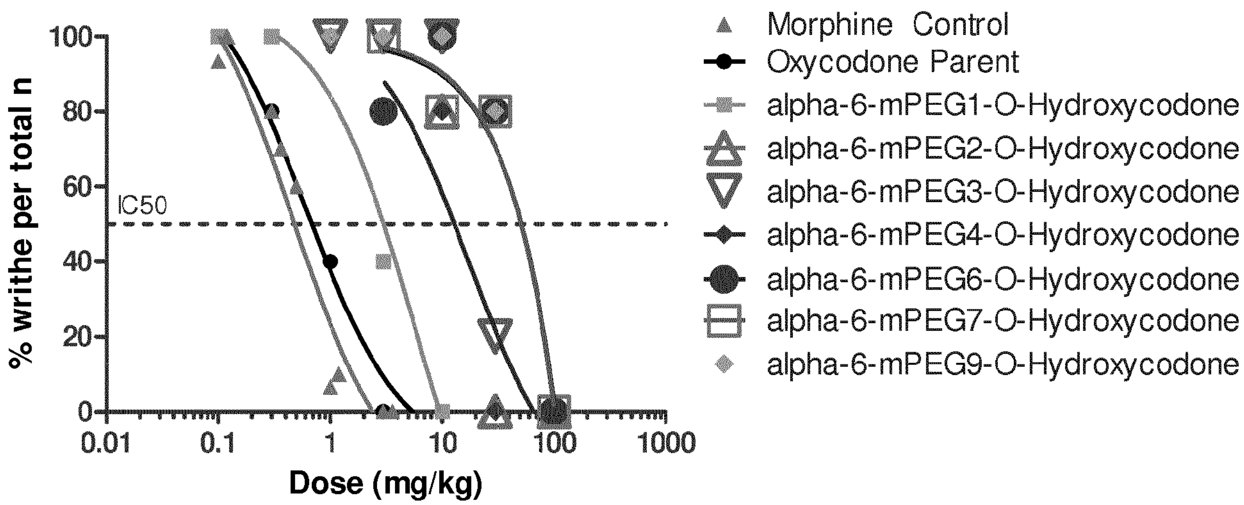


FIG. 3

Codeine Series Phenylquinone Writhing

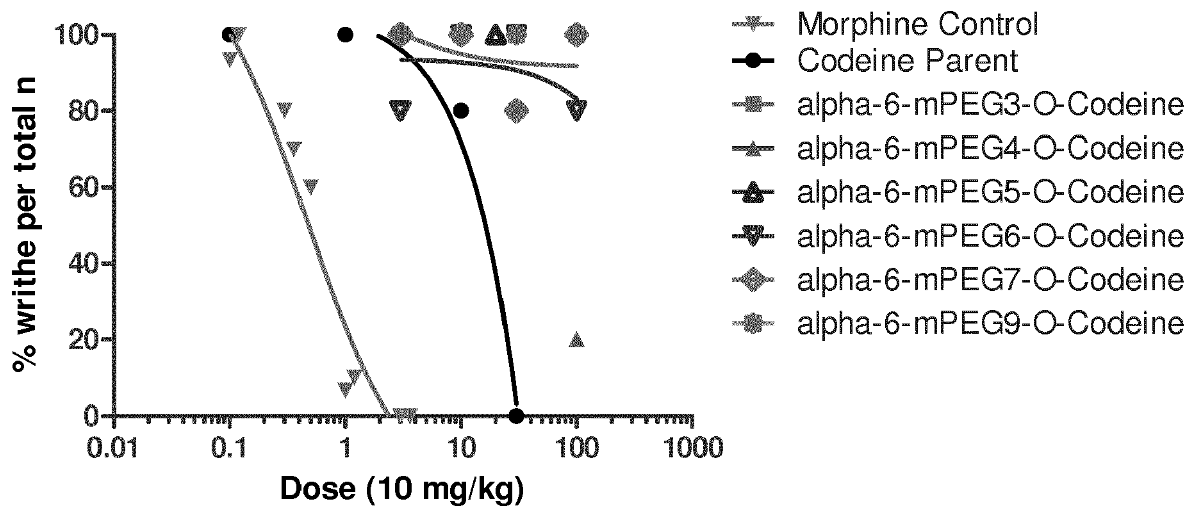


FIG. 4

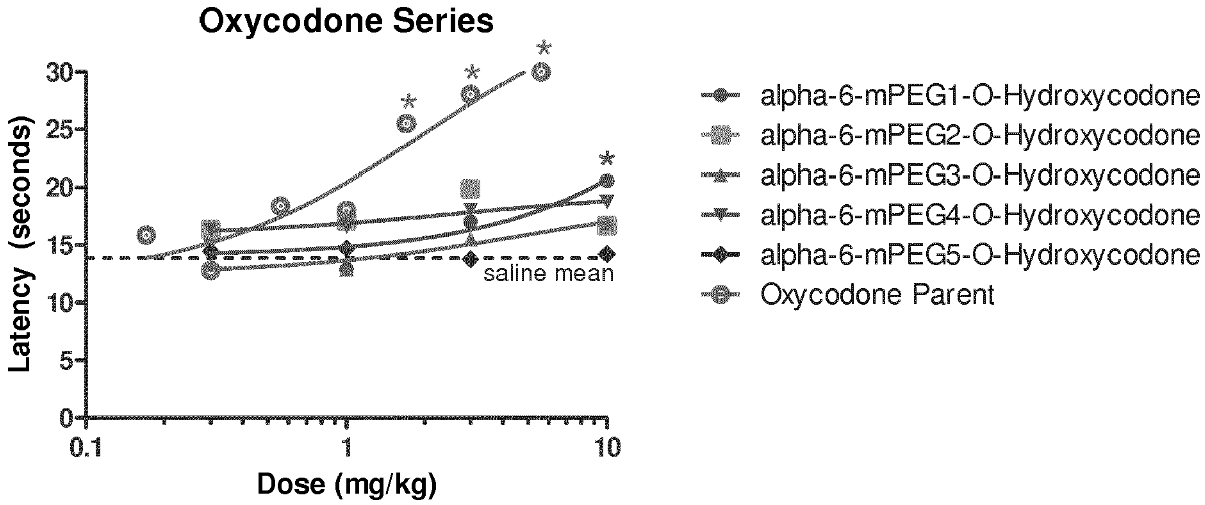


* indicates p < .05 vs. Saline by ANOVA/Dunnett's.

FIG. 5

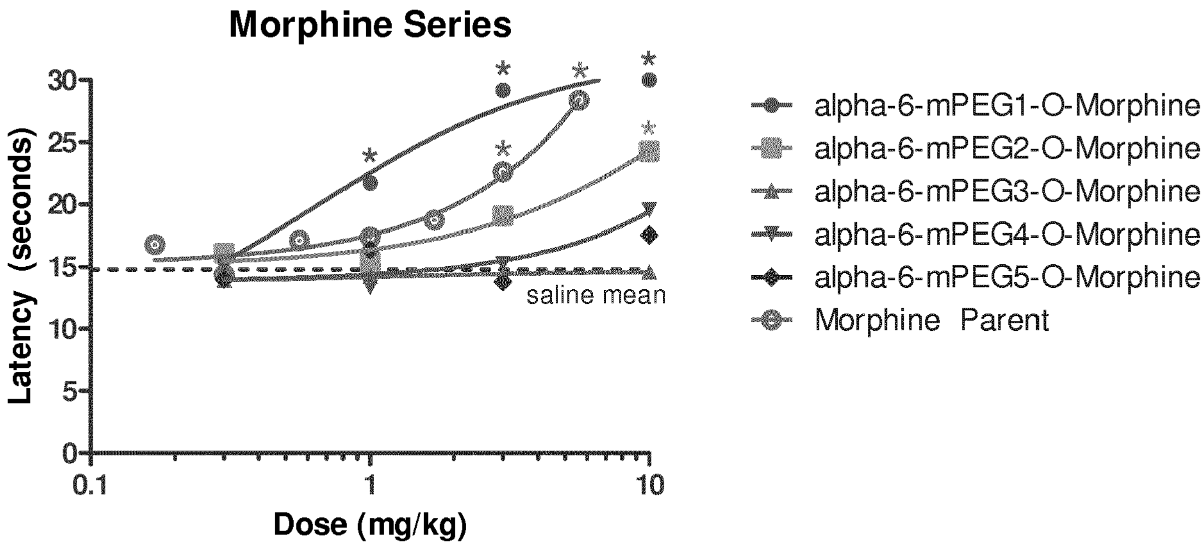


* indicates p < .05 vs. Saline by ANOVA/Dunnett's.

FIG. 6

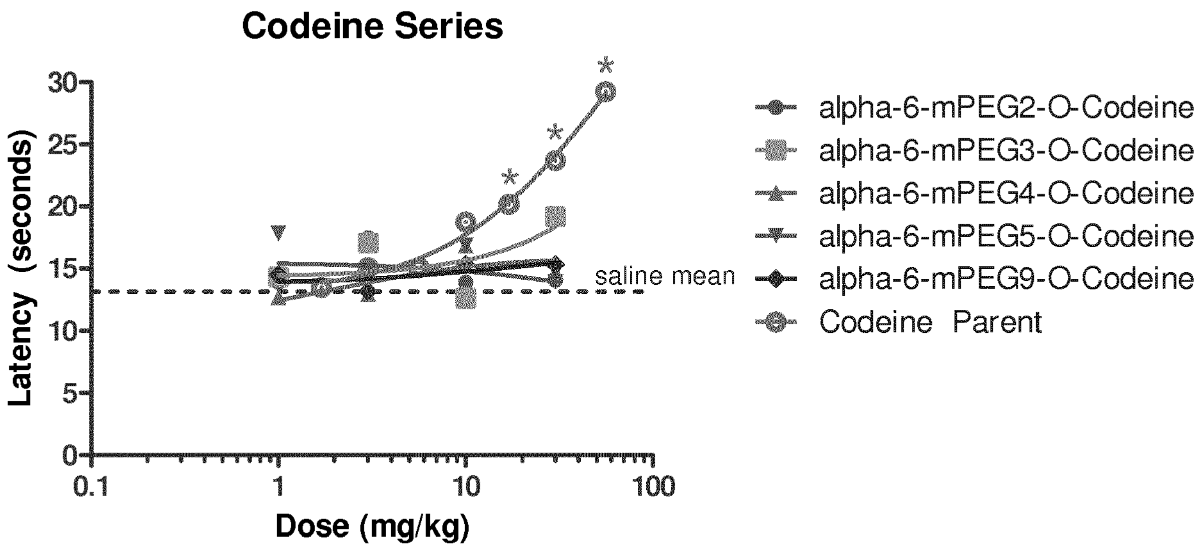


* indicates p < .05 vs. Saline by ANOVA/Dunnett's.

FIG. 7

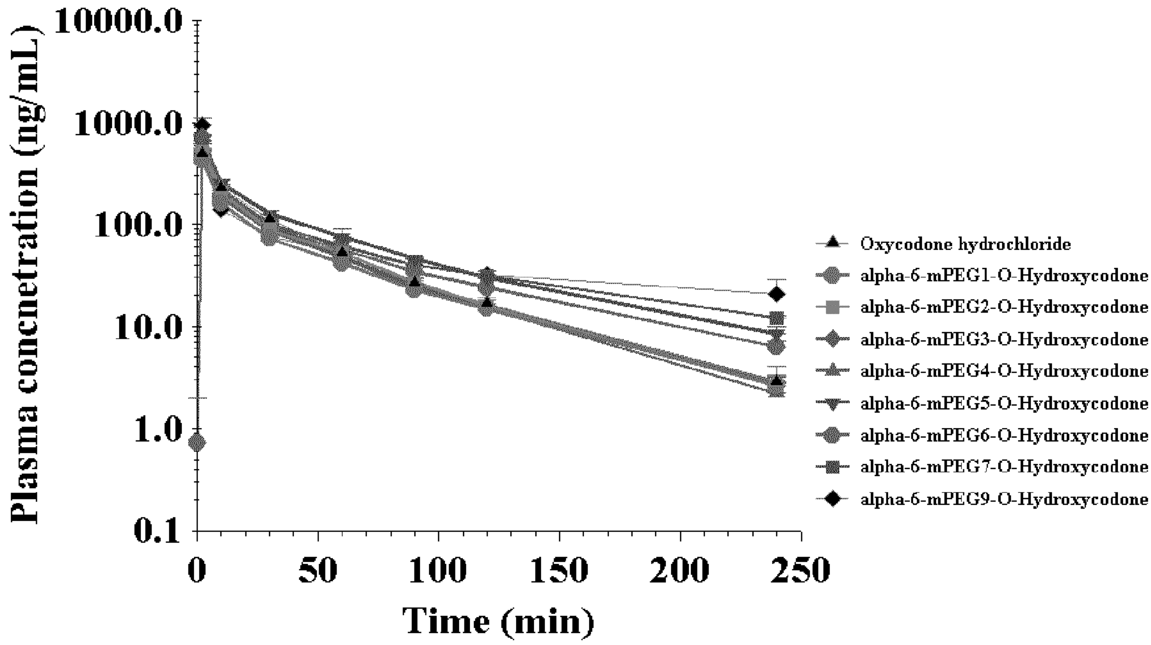
Plasma concnetration (ng/mL)
10000.0
1000.0
100.0
10.0
1.0
0.1
0
50
100
150
200
250
Time (min)
Oxycodone hydrochloride
alpha-6-mPEG1-O-Hydroxycodone
alpha-6-mPEG2-O-Hydroxycodone
alpha-6-mPEG3-O-Hydroxycodone
alpha-6-mPEG4-O-Hydroxycodone
alpha-6-mPEG5-O-Hydroxycodone
alpha-6-mPEG6-O-Hydroxycodone
alpha-6-mPEG7-O-Hydroxycodone
alpha-6-mPEG9-O-Hydroxycodone

FIG. 8

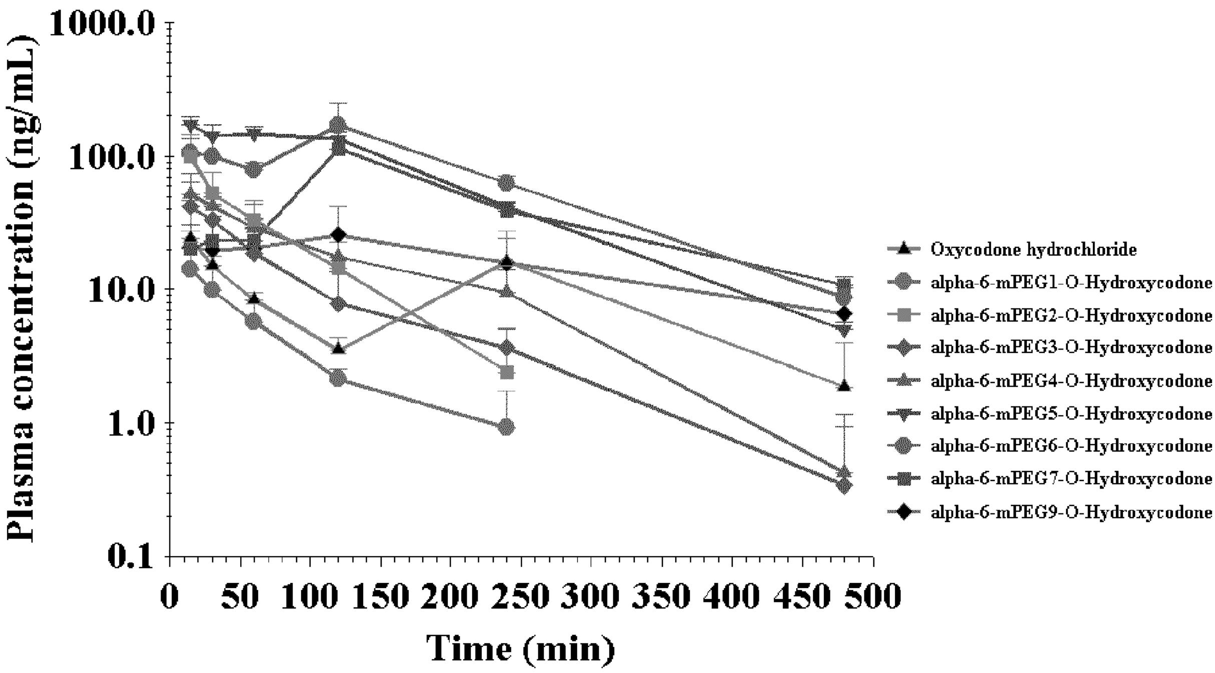

Plasma concentration (ng/mL)
1000.0
100.0
10.0
1.0
0.1
0
50
100
150
200
250
300
350
400
450
500
Time (min)
Oxycodone hydrochloride
alpha-6-mPEG1-O-Hydroxycodone
alpha-6-mPEG2-O-Hydroxycodone
alpha-6-mPEG3-O-Hydroxycodone
alpha-6-mPEG4-O-Hydroxycodone
alpha-6-mPEG5-O-Hydroxycodone
alpha-6-mPEG6-O-Hydroxycodone
alpha-6-mPEG7-O-Hydroxycodone
alpha-6-mPEG9-O-Hydroxycodone


FIG. 9

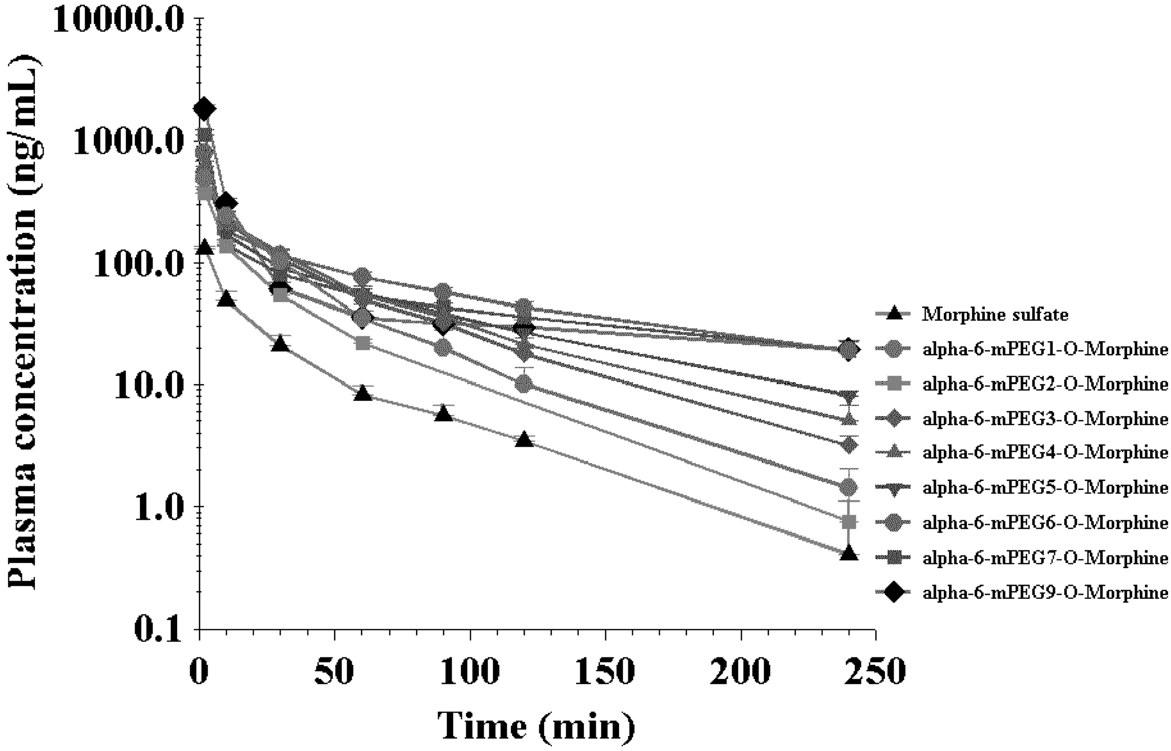
10000.0
1000.0
100.0
10.0
1.0
0.1
Plasma concentration (ng/mL)
0
50
100
150
200
250
Time (min)
Morphine sulfate
alpha-6-mPEG1-O-Morphine
alpha-6-mPEG2-O-Morphine
alpha-6-mPEG3-O-Morphine
alpha-6-mPEG4-O-Morphine
alpha-6-mPEG5-O-Morphine
alpha-6-mPEG6-O-Morphine
alpha-6-mPEG7-O-Morphine
alpha-6-mPEG9-O-Morphine

FIG. 10

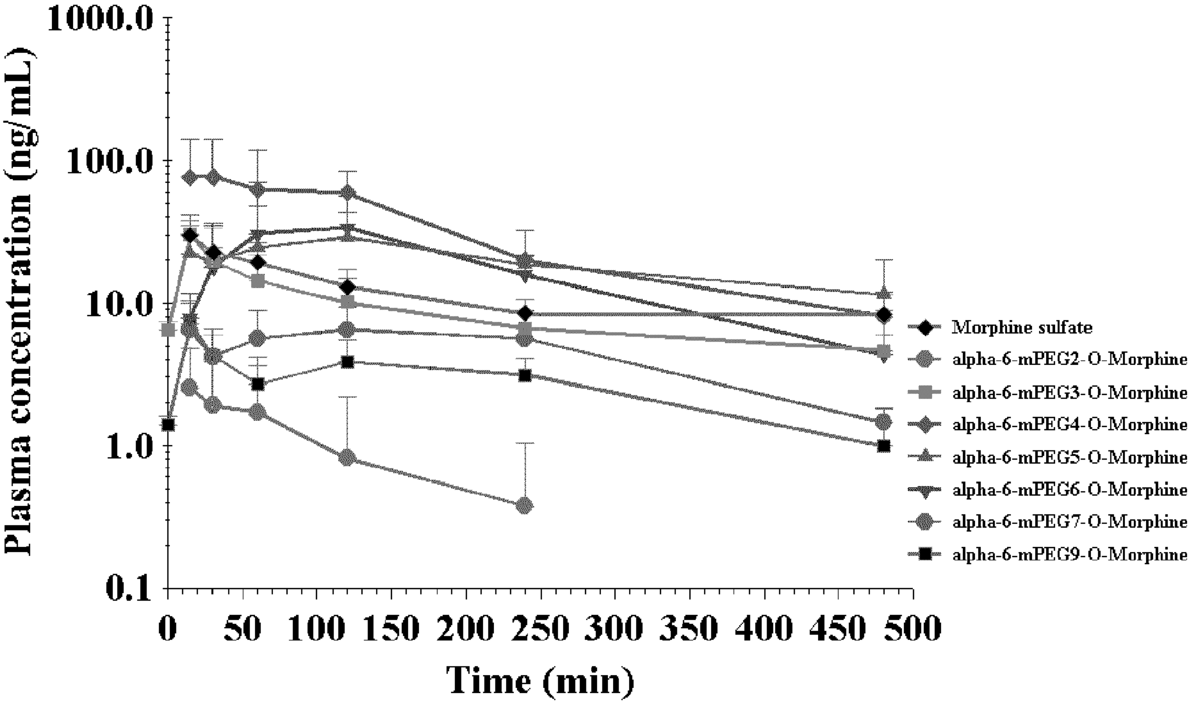
1000.0
100.0
10.0
1.0
0.1
Plasma concentration (ng/mL)
0
50
100
150
200
250
300
350
400
450
500
Time (min)
Morphine sulfate
alpha-6-mPEG2-O-Morphine
alpha-6-mPEG3-O-Morphine
alpha-6-mPEG4-O-Morphine
alpha-6-mPEG5-O-Morphine
alpha-6-mPEG6-O-Morphine
alpha-6-mPEG7-O-Morphine
alpha-6-mPEG9-O-Morphine

FIG. 11

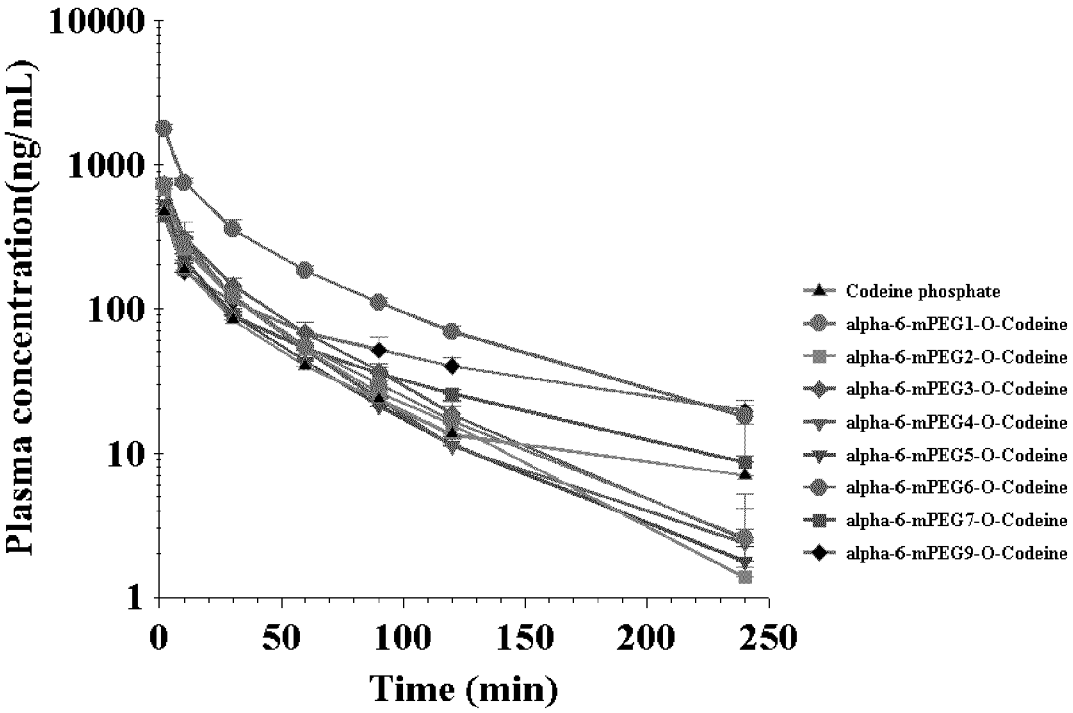

Plasma concentration(ng/mL)
10000
1000
100
10
1
0
50
100
150
200
250
Time (min)
Codeine phosphate
alpha-6-mPEG1-O-Codeine
alpha-6-mPEG2-O-Codeine
alpha-6-mPEG3-O-Codeine
alpha-6-mPEG4-O-Codeine
alpha-6-mPEG5-O-Codeine
alpha-6-mPEG6-O-Codeine
alpha-6-mPEG7-O-Codeine
alpha-6-mPEG9-O-Codeine


FIG. 12

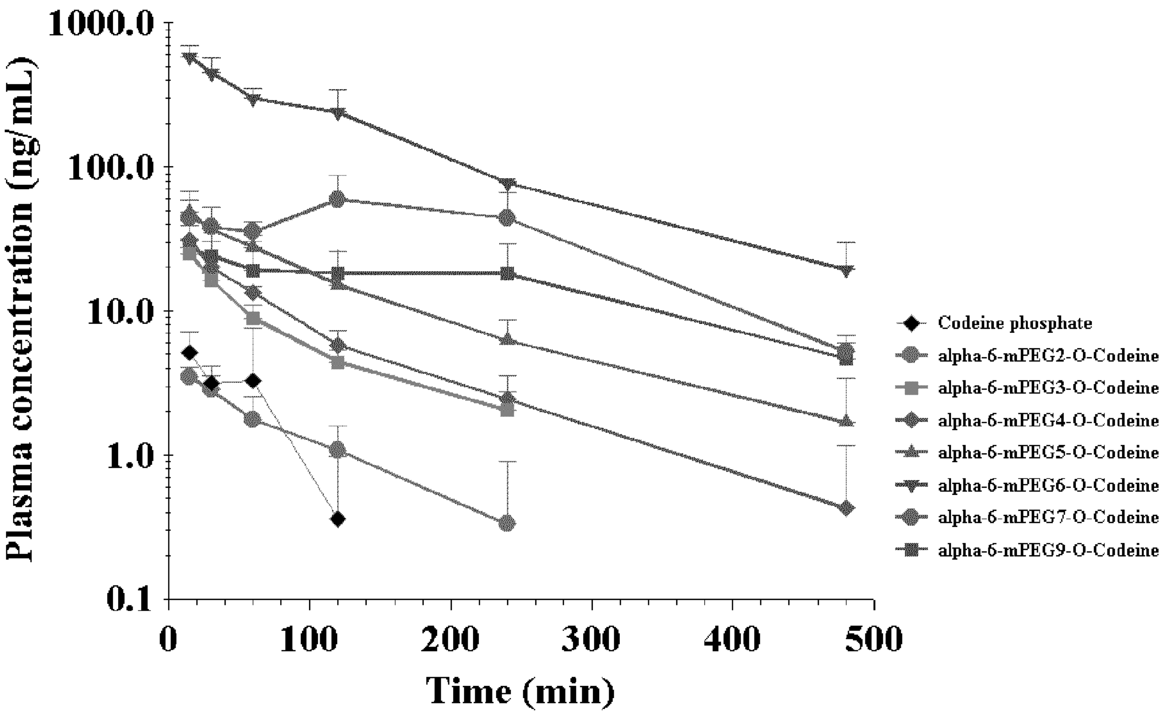
1000.0
100.0
10.0
1.0
0.1
Plasma concentration (ng/mL)
0
100
200
300
400
500
Time (min)
Codeine phosphate
alpha-6-mPEG2-O-Codeine
alpha-6-mPEG3-O-Codeine
alpha-6-mPEG4-O-Codeine
alpha-6-mPEG5-O-Codeine
alpha-6-mPEG6-O-Codeine
alpha-6-mPEG7-O-Codeine
alpha-6-mPEG9-O-Codeine

FIG. 13

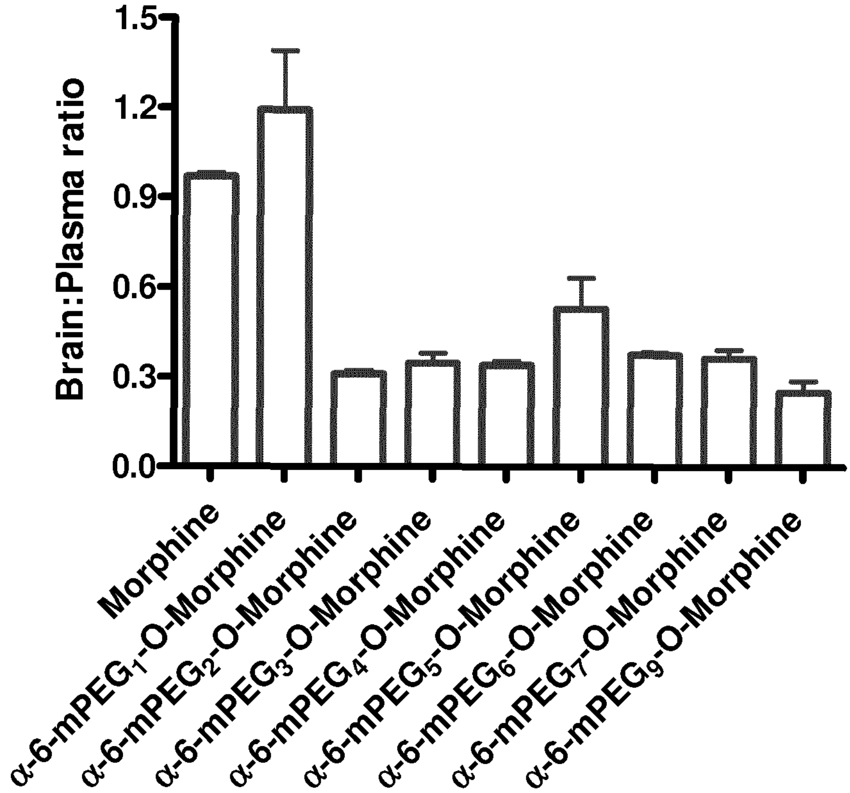


| | A / B | | | C / D | | |
|---|---|---|---|---|---|---|
| | Mean | SD | N | Mean | SD | N |
| Morphine | 0.97 | 0.019 | 3 | | | |
| α-6-$mPEG_1$-O-Morphine | 1.19 | 0.341 | 3 | | | |
| α-6-$mPEG_2$-O-Morphine | 0.31 | 0.019 | 3 | | | |
| α-6-$mPEG_3$-O-Morphine | 0.35 | 0.057 | 3 | | | |
| α-6-$mPEG_4$-O-Morphine | 0.34 | 0.023 | 3 | | | |
| α-6-$mPEG_5$-O-Morphine | 0.53 | 0.179 | 3 | | | |
| α-6-$mPEG_6$-O-Morphine | 0.37 | 0.016 | 3 | | | |
| α-6-$mPEG_7$-O-Morphine | 0.36 | 0.050 | 3 | | | |
| α-6-$mPEG_9$-O-Morphine | 0.25 | 0.067 | 3 | | | |
| Atenolol | | | | 0.060 | 0.008 | 3 |

FIG. 14A

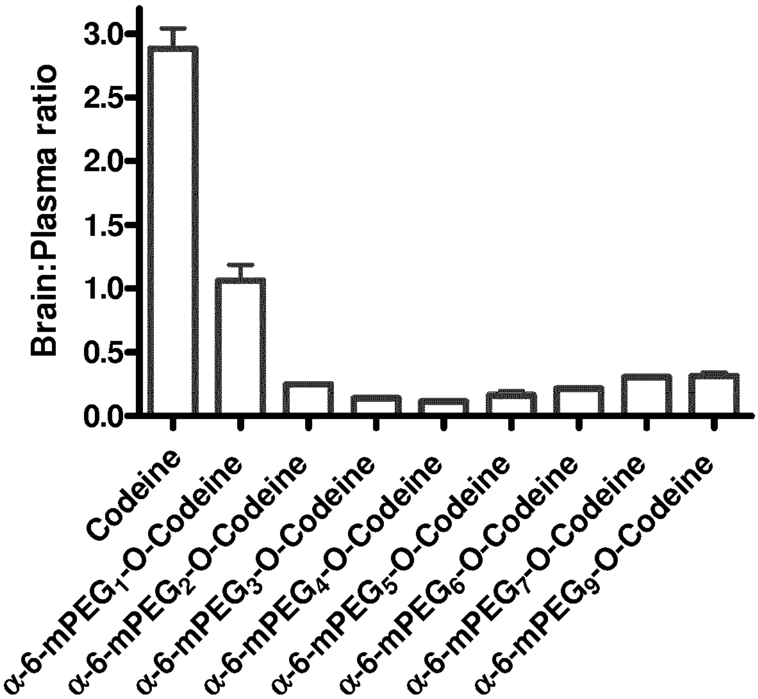


Molecule Brain:plasma ratios

| | A / B | | | C / D | | |
|---|---|---|---|---|---|---|
| | Mean | SD | N | Mean | SD | N |
| Codeine | 2.88 | 0.276 | 3 | | | |
| α-6-mPEG$_1$-O-Codeine | 1.06 | 0.214 | 3 | | | |
| α-6-mPEG$_2$-O-Codeine | 0.24 | 0.014 | 3 | | | |
| α-6-mPEG$_3$-O-Codeine | 0.14 | 0.011 | 3 | | | |
| α-6-mPEG$_4$-O-Codeine | 0.12 | 0.009 | 3 | | | |
| α-6-mPEG$_5$-O-Codeine | 0.16 | 0.049 | 3 | | | |
| α-6-mPEG$_6$-O-Codeine | 0.21 | 0.006 | 3 | | | |
| α-6-mPEG$_7$-O-Codeine | 0.30 | 0.017 | 3 | | | |
| α-6-mPEG$_9$-O-Codeine | 0.31 | 0.047 | 3 | | | |
| Atenolol | | | | 0.060 | 0.013 | 3 |

FIG. 14B

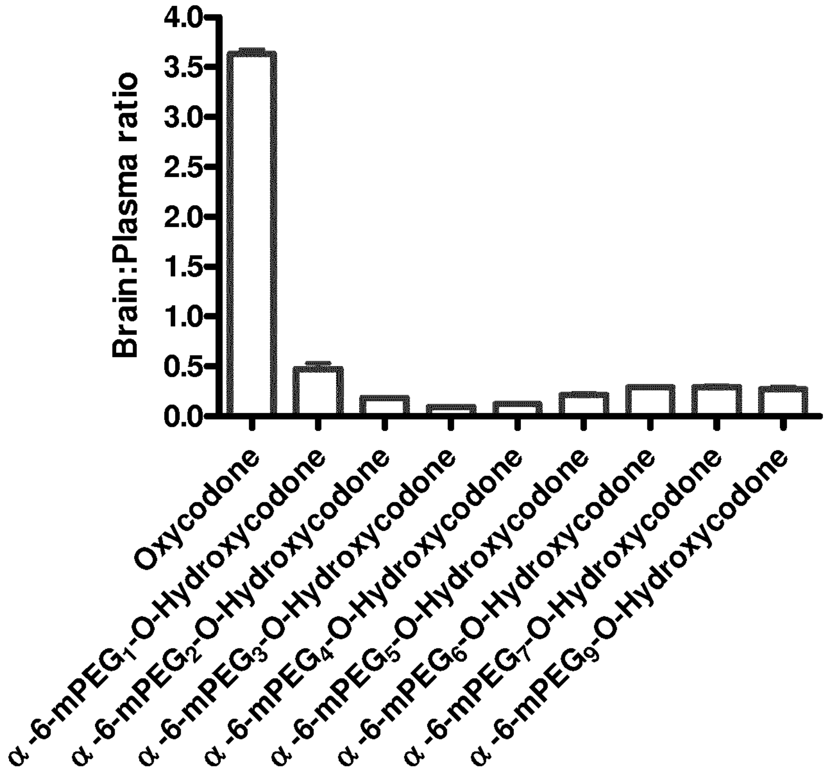


Molecule Brain:plasma ratios

| | A / B | | | C / D | | |
|---|---|---|---|---|---|---|
| | Mean | SD | N | Mean | SD | N |
| Oxycodone | 3.63 | 0.073 | 3 | | | |
| α -6-mPEG$_1$-O-Hydroxycodone | 0.47 | 0.108 | 3 | | | |
| α -6-mPEG$_2$-O-Hydroxycodone | 0.18 | 0.008 | 3 | | | |
| α -6-mPEG$_3$-O-Hydroxycodone | 0.10 | 0.006 | 3 | | | |
| α -6-mPEG$_4$-O-Hydroxycodone | 0.12 | 0.028 | 3 | | | |
| α -6-mPEG$_5$-O-Hydroxycodone | 0.21 | 0.029 | 3 | | | |
| α -6-mPEG$_6$-O-Hydroxycodone | 0.29 | 0.026 | 3 | | | |
| α -6-mPEG$_7$-O-Hydroxycodone | 0.29 | 0.020 | 3 | | | |
| α -6-mPEG$_9$-O-Hydroxycodone | 0.27 | 0.039 | 3 | | | |
| Atenolol | | | | 0.059 | 0.015 | 3 |

FIG. 14C

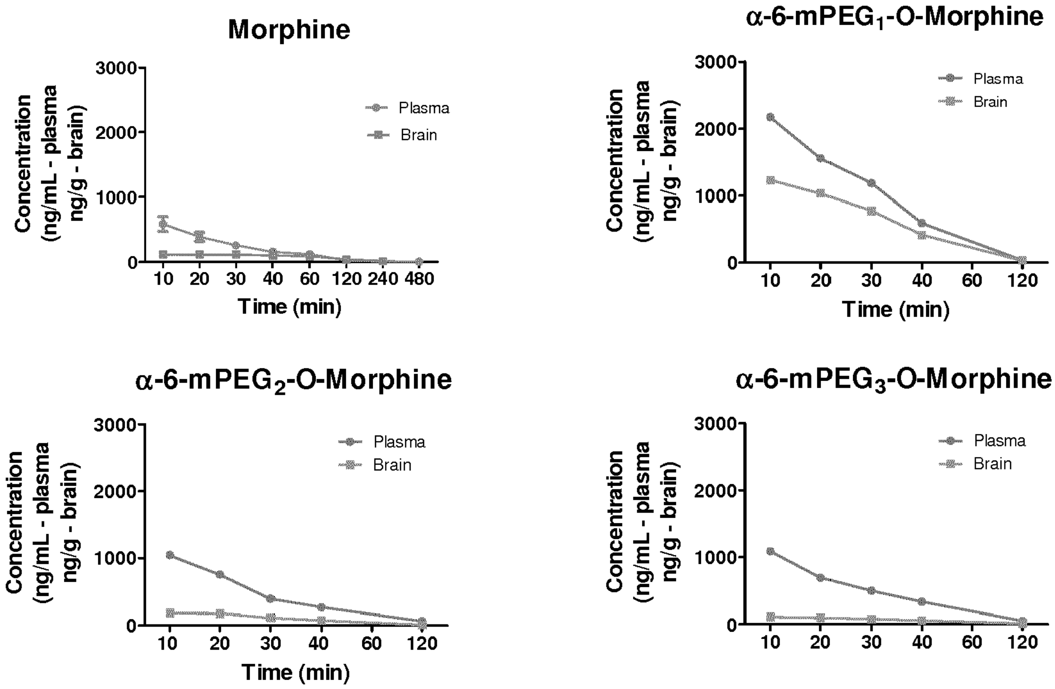


**FIG. 15A** (Top)
**FIG. 15C** (Bottom, PEG2)

**FIG. 15B** (Top, PEG1)
**Fig. 15D** (Bottom, PEG3)

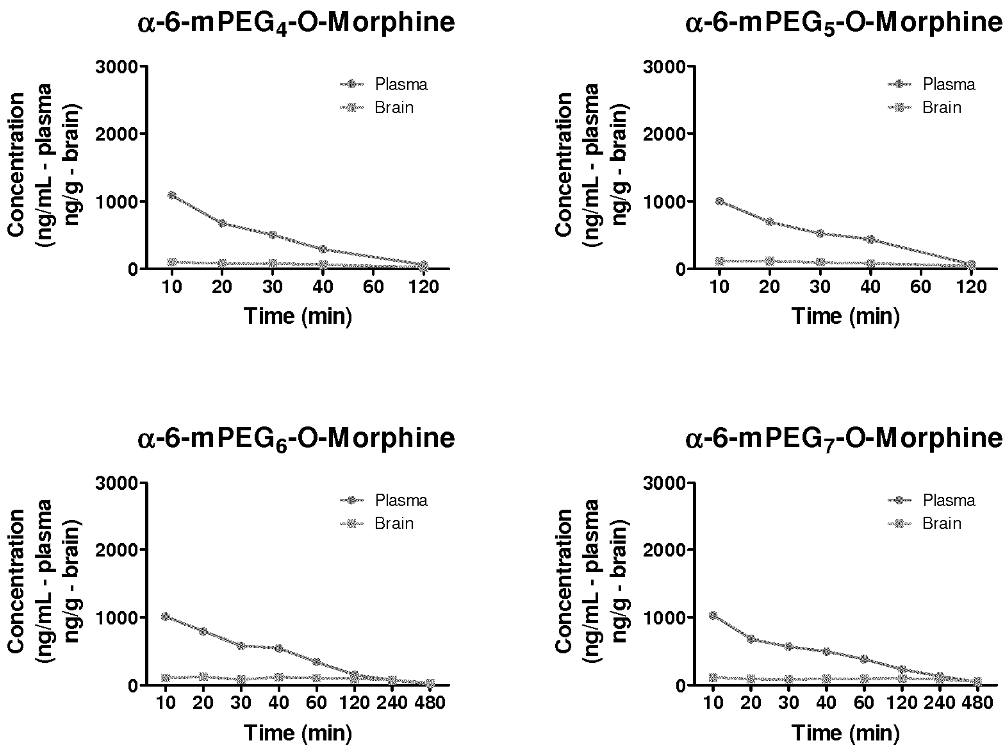


**FIG. 15E** (Top, PEG4)
**FIG. 15G** (Bottom, PEG6)

**FIG. 15F** (Top, PEG5)
**FIG. 15H** (Bottom, PEG7)

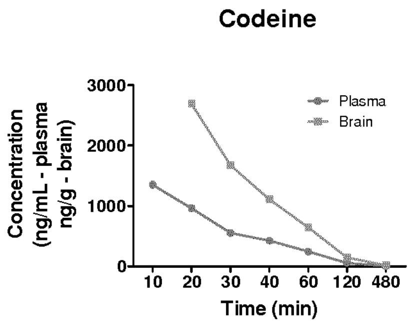


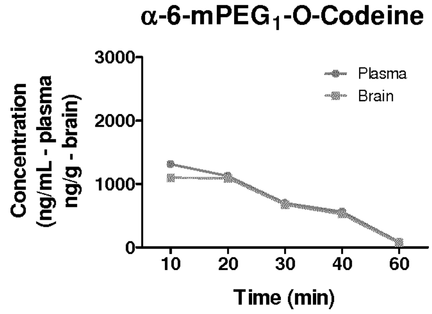


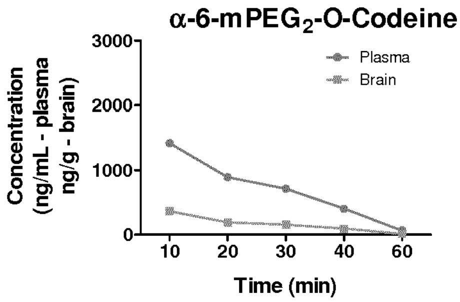


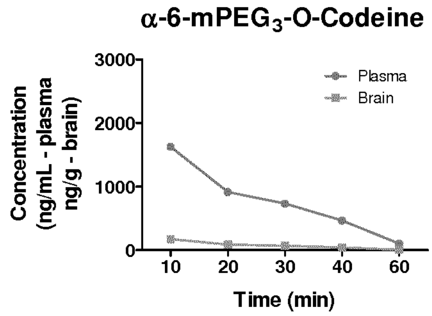


**FIG. 16A** (Top)
**FIG. 16C** (Bottom, PEG2)

**FIG. 16B** (Top, PEG1)
**FIG. 16D** (Bottom, PEG3)

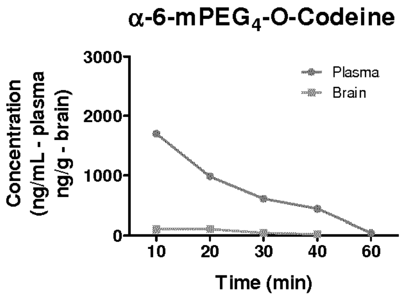


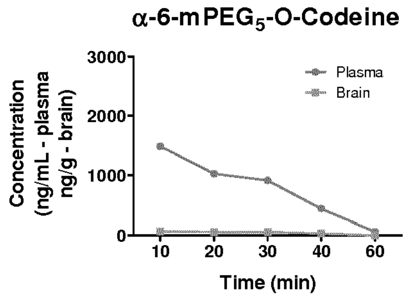


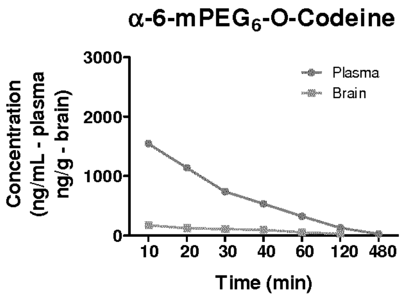


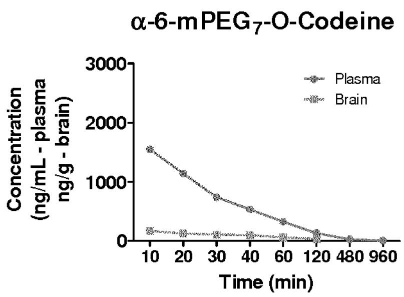


**FIG. 16E** (PEG4)
**FIG. 16G** (Bottom, PEG6)

**FIG. 16F** (Top, PEG5)
**FIG. 16H** (Bottom, PEG7)

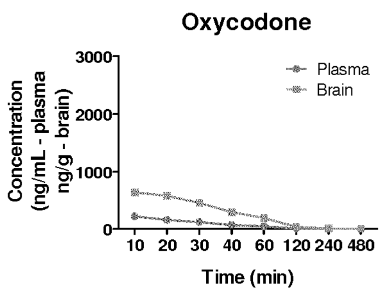


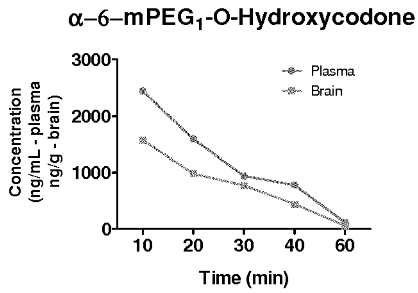


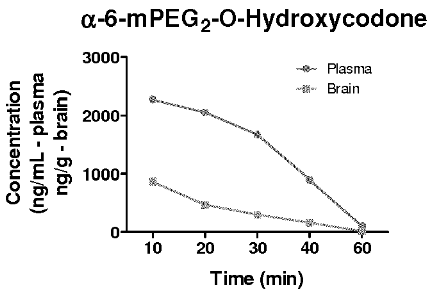


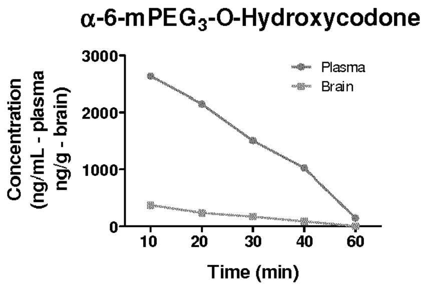


**FIG. 17A** (Top)
**FIG. 17C** (Bottom, PEG2)

**FIG. 17B** (Top, PEG1)
**FIG. 17D** (Bottom, PEG3)

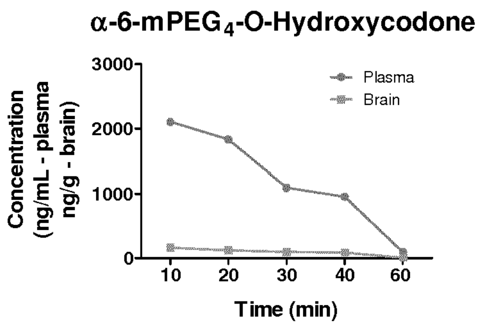


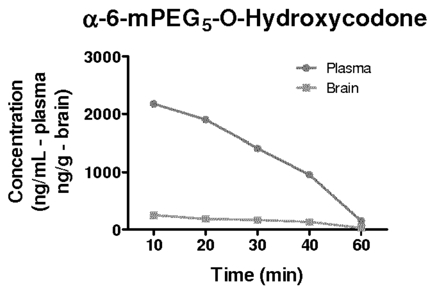


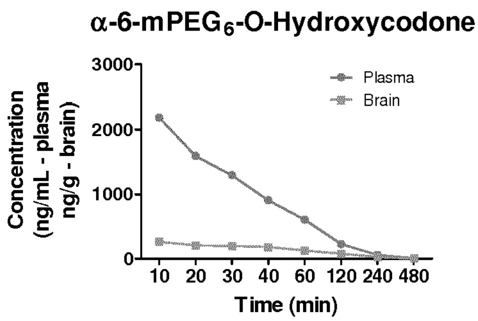


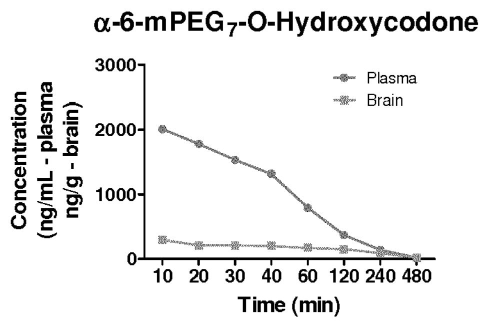


**FIG. 17E** (PEG4)
**FIG. 17G** (Bottom, PEG6)

**FIG. 17F** (Top, PEG5)
**FIG. 17H** (Bottom, PEG7)

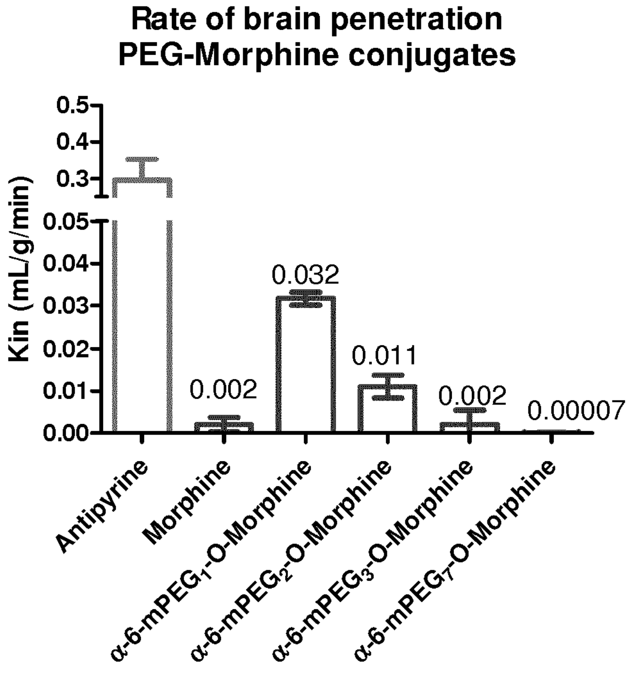


FIG. 18A

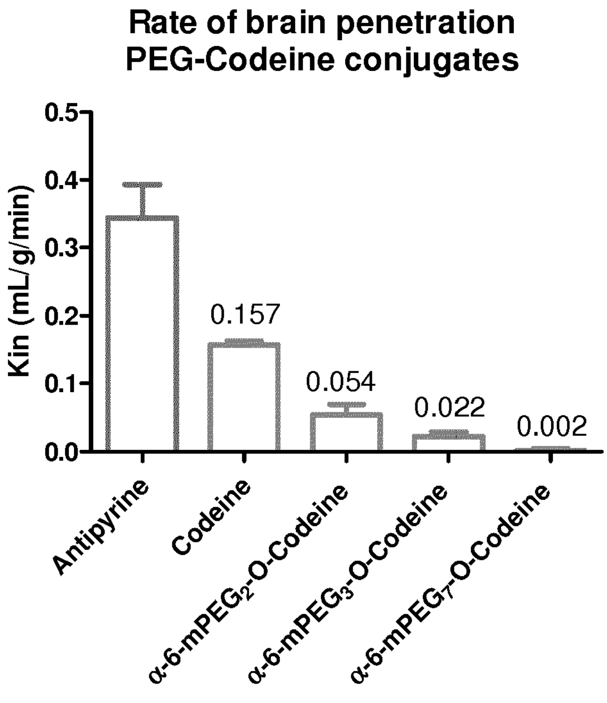


FIG. 18B

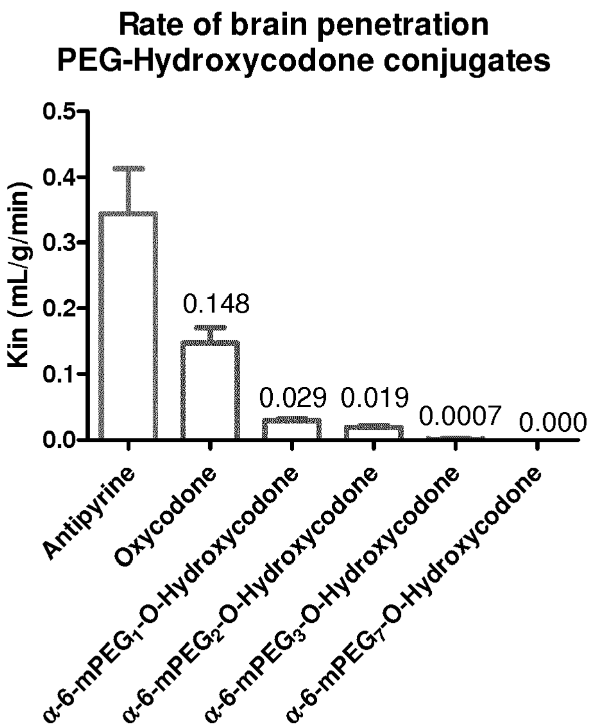
Rate of brain penetration
PEG-Hydroxycodone conjugates
Kin (mL/g/min)
0.5
0.4
0.3
0.2
0.1
0.0
0.148
0.029
0.019
0.0007
0.000
Antipyrine
Oxycodone
α-6-mPEG1-O-Hydroxycodone
α-6-mPEG2-O-Hydroxycodone
α-6-mPEG3-O-Hydroxycodone
α-6-mPEG7-O-Hydroxycodone

FIG. 18C

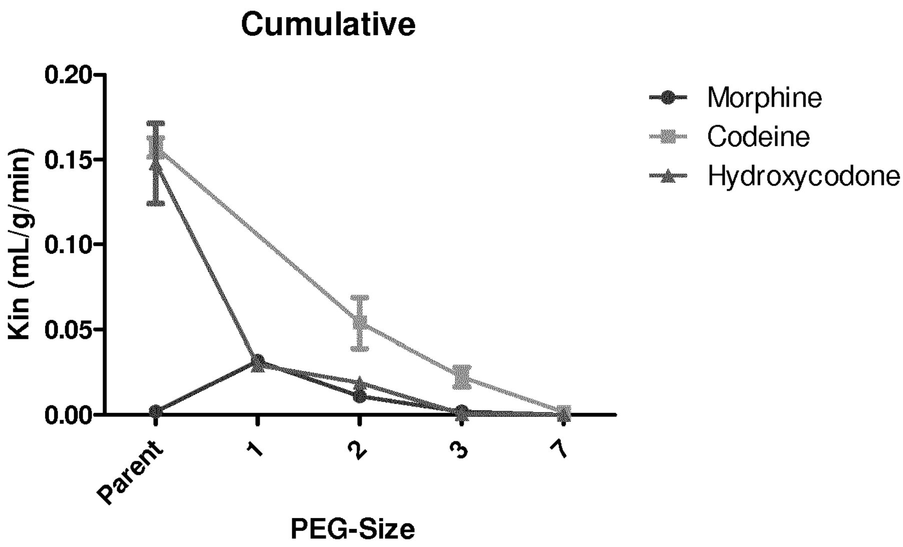


| Fold change in Kin versus parent | | | | | | |
|---|---|---|---|---|---|---|
| | Morphine | | Codeine | | Hydroxycodone | |
| | Mean | SEM | Mean | SEM | Mean | SEM |
| Parent | 1.000 | 0.707 | 1.000 | 0.029 | 1.000 | 0.130 |
| 1 | 15.833 | 7.929 | | | 0.199 | 0.022 |
| 2 | 5.500 | 2.854 | 0.343 | 0.055 | 0.129 | 0.018 |
| 3 | 1.000 | 1.118 | 0.142 | 0.021 | 0.005 | 0.005 |
| 7 | 0.033 | 0.037 | 0.011 | 0.011 | 0.000 | |

FIG. 19

Europäisches Patentamt
European Patent Office
Office européen des brevets

# EUROPEAN SEARCH REPORT

Application Number
EP 17 19 8484

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | WO 2008/112288 A2 (NEKTAR THERAPEUTICS AL CORP [US]; RIGGS-SAUTHIER JENNIFER [US]; DENG B) 18 September 2008 (2008-09-18)<br>* paragraphs [0007]-[0013], [0059]-[0061], [0064], [0074]-[0076], [0101]-[0109], [0111], [0118], [0130], [0141] *<br>* examples 1-7 *<br>* claims 1-33 *<br>----- | 1-15 | INV.<br>A61K47/60<br>A61P25/04<br>A61P25/36 |
| X | US 2006/182692 A1 (FISHBURN C S [US] ET AL) 17 August 2006 (2006-08-17)<br>* examples 5-10 *<br>* paragraphs [0111], [0125], [0134] *<br>----- | 1-15 | |

TECHNICAL FIELDS SEARCHED (IPC)

A61K
A61P

The present search report has been drawn up for all claims

1

| Place of search | Date of completion of the search | Examiner |
|---|---|---|
| The Hague | 11 May 2018 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 19 8484

This annex lists the patent family members relating to the patent documents cited in the above-mentioned European search report. The members are as contained in the European Patent Office EDP file on The European Patent Office is in no way liable for these particulars which are merely given for the purpose of information.

11-05-2018

| Patent document cited in search report | Publication date | Patent family member(s) | Publication date |
|---|---|---|---|
| WO 2008112288 A2 | 18-09-2008 | AU 2008226822 A1 | 18-09-2008 |
| | | CA 2679479 A1 | 18-09-2008 |
| | | CN 101646464 A | 10-02-2010 |
| | | CY 1117368 T1 | 26-04-2017 |
| | | DK 2134371 T3 | 27-04-2015 |
| | | EP 2134371 A2 | 23-12-2009 |
| | | EP 2620163 A1 | 31-07-2013 |
| | | EP 2623123 A1 | 07-08-2013 |
| | | EP 3222293 A1 | 27-09-2017 |
| | | ES 2534741 T3 | 28-04-2015 |
| | | HR P20150315 T1 | 24-04-2015 |
| | | IL 200845 A | 31-08-2015 |
| | | JP 5420427 B2 | 19-02-2014 |
| | | JP 5877403 B2 | 08-03-2016 |
| | | JP 2010521466 A | 24-06-2010 |
| | | JP 2013151563 A | 08-08-2013 |
| | | KR 20090118952 A | 18-11-2009 |
| | | PT 2134371 E | 16-04-2015 |
| | | SI 2134371 T1 | 30-04-2015 |
| | | WO 2008112288 A2 | 18-09-2008 |
| US 2006182692 A1 | 17-08-2006 | US 2006182692 A1 | 17-08-2006 |
| | | US 2010210676 A1 | 19-08-2010 |
| | | US 2012088745 A1 | 12-04-2012 |
| | | US 2014303403 A1 | 09-10-2014 |
| | | US 2017095565 A1 | 06-04-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61192247 A
- US 55839509 A
- US 2628962 A
- US 2654756 A
- US 2649454 A
- US 2806033 A
- WO 02098949 A
- US 20050136031 A
- US 5672662 A


### Non-patent literature cited in the description


- **BALSTER ; SCHUSTER.** *J Exp Anal Behav,* 1973, vol. 20, 119-129
- **PANLILIO ; SCHINDLER.** *Psychopharmacology,* 2000, vol. 150, 61-66
- **WINGER et al.** *J Pharmacol Exp Ther,* 2002, vol. 301, 690-697
- **KO et al.** *J Pharmacol Exp Ther,* vol. 301, 698-704
- **ABREU et al.** *Psychopharmacologia,* 2001, vol. 154, 76-84
- **T.W. GREENE ; G.M. WUTS.** Protecting Groups in Organic Synthesis. Wiley, 1999
- **CHEN ; BAKER.** *J. Org. Chem.,* 1999, 6870-6873
- **SUMMERFIELD et al.** *J Pharmacol Exp Ther,* 2007, vol. 322, 205-213
- **TSUJI.** *NeuroRx,* 2005, vol. 2, 54-62
- **RUBIN ; STADDON.** *Annu. Rev. Neurosci.,* 1999, vol. 22, 11-28
- **ERTL, P. et al.** *J. Med. Chem.,* 2000, vol. 43, 3714-3717
- **KELDER, J. et al.** *Pharm. Res.,* 1999, vol. 16, 1514-1519
- Remington: The Science & Practice of Pharmacy. Williams & Williams, 1995
- Physician's Desk Reference. Medical Economics, 1998
- **KIBBE, A.H.** Handbook of Pharmaceutical Excipients. American Pharmaceutical Association, 2000
- **BERGMAN ; PARONIS.** *Mol Interventions,* 2006, vol. 6, 273-83
- *J. Pharmacokinet. Biopharm.,* 1978, vol. 6, 505-19